(19) Europäisches Patentamt
European Patent Office
Office européen des brevets

(11) Publication number: **0 161 905**
**B1**

(12) **EUROPEAN PATENT SPECIFICATION**

(45) Date of publication of patent specification: **01.08.90**

(21) Application number: **85303256.3**

(22) Date of filing: **08.05.85**

(51) Int. Cl.[5]: **C 07 D 239/46,**
C 07 D 409/12,
C 07 D 405/12, A 01 N 47/36

(54) **Herbicidal halopyrimidines.**

(30) Priority: **11.06.84 US 619276**
**12.04.85 US 721602**
**09.05.84 US 608556**

(43) Date of publication of application:
**21.11.85 Bulletin 85/47**

(45) Publication of the grant of the patent:
**01.08.90 Bulletin 90/31**

(84) Designated Contracting States:
**BE CH DE FR GB IT LI LU NL SE**

(56) References cited:
**EP-A-0 044 808**
**EP-A-0 079 683**
**EP-A-0 099 339**

**The file contains technical information submitted after the application was filed and not included in this specification**

(73) Proprietor: **E.I. DU PONT DE NEMOURS AND COMPANY**
**1007 Market Street**
**Wilmington Delaware 19898 (US)**

(72) Inventor: **Brown, Hugh Malcolm**
**322 Rosehill Road**
**West Grove Pennsylvania 19390 (US)**
Inventor: **Pasteris, Robert James**
**305 Plymouth Road**
**Wilmington Delaware 19803 (US)**

(74) Representative: **Hildyard, Edward Martin et al**
**Frank B. Dehn & Co. Imperial House 15-19 Kingsway**
**London WC2B 6UZ (GB)**

**EP 0 161 905 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Background of the Invention

The present invention relates to specific halopyrimidine compounds which are useful as general or selective preemergent or postemergent herbicides or as plant growth regulants.

U.S. Patent 4,394,506, issued July 19, 1983 to Levitt discloses herbicidal N - (heterocyclicaminocarbonyl)arylsulfonamides of the formula

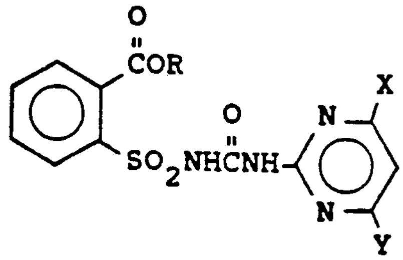

wherein, among other substituents, X may be $OCH_3$ or $OC_2H_5$ and Y may be F. Cl, or Br.

Netherlands Patent 121,788, published September 15, 1966, discloses the preparation of compounds of the following Formula and their use as general or selective herbicides:

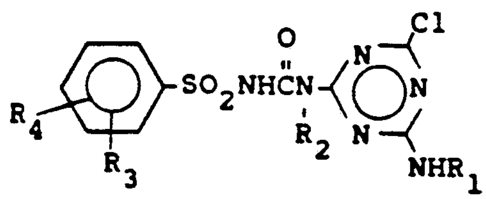

(i)

wherein

$R_1$ and $R_2$ may independently be alkyl of 1—4 carbon atoms; and

$R_3$ and $R_4$ may independently be hydrogen, chlorine or alkyl of 1—4 carbon atoms.

U.S. Patent 3,637,366 discloses compounds having the formula:

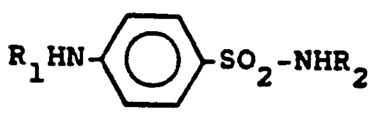

wherein

$R_1$ is hydrogen or lower saturated aliphatic acyl and

$R_2$ is hydrogen, 2-pyrimidinyl, pyridyl, amidino, acetyl or carbamoyl.

The disclosed compounds are said to provide control of crabgrass, cress, endive, clover and *Poa annua.*

French Patent No. 1,468,747 discloses the following *para*-substituted phenylsulfonamides as being useful as antidiabetic agents:

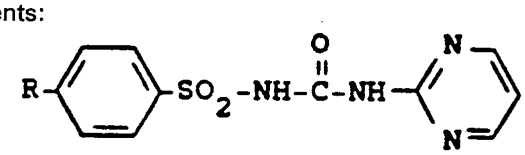

wherein

R = H, halogen, $CF_3$ or alkyl.

Logemann et al., Chem. Ab., *53,* 18052g (1959), disclose a number of sulfonamides, including uracil derivatives and those having the formula:

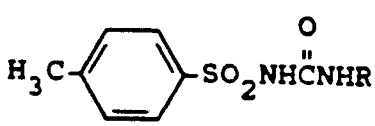

wherein

R is butyl, phenyl or

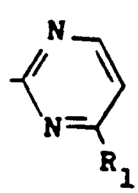

;

and

$R_1$ is hydrogen or methyl.

When tested for hypoglycemic effect in rats (oral doses of 25 mg/100 g), the compounds in which R is butyl and phenyl were most potent. The others were of low potency or inactive.

Wojciechowski, J. Acta. Polon, Pharm. *19*, p. 121—5 (1962) [Chem. Ab., *59*, 1633 e] describes the synthesis of N - [(2,6 - dimethoxypyrimidin - 4 - yl)aminocarbonyl] - 4 - methylbenzenesulfonamide:

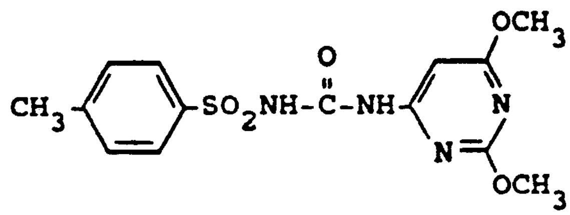

U.S. Patent 4,127,405 teaches compounds which are useful for controlling weeds in wheat having the formula

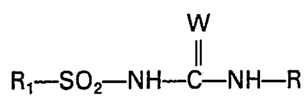

wherein

R is

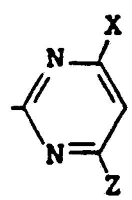

or

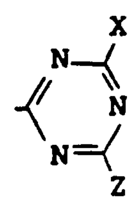

;

$R_1$ is

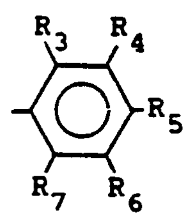

,

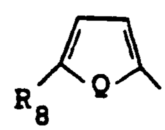

,

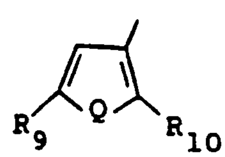

or

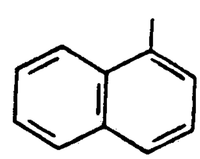

$R_3$ and $R_6$ are independently hydrogen, fluorine, chlorine, bromine, iodine, alkyl of 1—4 carbon atoms, alkoxy of 1—4 carbon atoms, nitro, trifluoromethyl, cyano, $CH_3S(O)_n$— or $CH_3CH_2S(O)_n$—;

$R_4$ is hydogen, fluorine, chlorine, bromine or methyl;

$R_5$ is hydrogen, fluorine, chlorine, bromine, methyl or methoxy;

$R_7$ is hydrogen, fluorine, chlorine, bromine, alkyl of 1—2 carbon atoms or alkoxy of 1—2 carbon atom;

$R_8$ is hydrogen, methyl, chlorine or bromine;

$R_9$ and $R_{10}$ are independently hydrogen, methyl, chlorine or bromine;

W and Q are independently oxygen or sulfur;

n is 0, 1 or 2;

X is hydrogen, chlorine, bromine, methyl, ethyl, alkoxy of 1—3 carbon atoms, trifluoromethyl, $CH_3S$— or $CH_3OCH_2$—; and

Z is methyl or methoxy;

or their agriculturally suitable salts; provided that:

(a) when $R_5$ is other than hydrogen, at least one of $R_3$, $R_4$, $R_6$ and $R_7$ is other than hydrogen and at least two of $R_3$, $R_4$, $R_6$ and $R_7$ must be hydrogen;

(b) when $R_5$ is hydrogen and all of $R_3$, $R_4$, $R_6$ and $R_7$ are other than hydogen, then all of $R_3$, $R_4$, $R_6$ and $R_7$ must be either chlorine or methyl; and

(c) when $R_3$ and $R_7$ are both hydrogen, at least one of $R_4$, $R_5$ or $R_6$ must be hydrogen.

EP—A—79,683 discloses herbicidal benzofuran and benzothiophine sulfonylureas, wherein the pyrimidin heterocycle may be substituted with, among other values, F or Cl.

The presence of undesired vegetation causes substantial damage to useful crops, especially agricultural products that satisfy man's basic food and fiber needs, such as cotton, rice, corn, wheat, and the like. The current population explosion and concomitant world food and fiber shortage demand improvements in the efficiency of producing these crops. Prevention or minimizing the loss of a portion of such valuable crops by killing, or inhibiting the growth of undesired vegetation is one way of improving this efficiency. A wide variety of materials useful for killing, or inhibiting (controlling) the growth of undesired vegetation is available; such materials are commonly referred to as herbicides. The need still exists however, for more effective, selective herbicides.

Summary of the Invention

This invention relates to novel compounds of Formulae I and Ia, agriculturally suitable compositions containing them, and their method-of-use as general or selective preemergent or postemergent herbicides or as plant growth regulants.

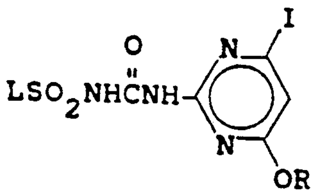

I

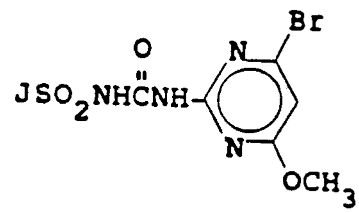

Ia

wherein

R is $CH_3$ or $CH_2CH_3$;

L is 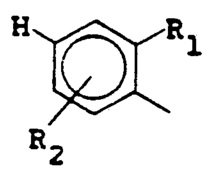,

L-1

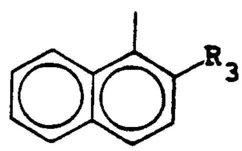,

L-2

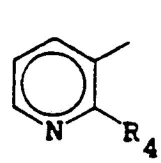,

L-3

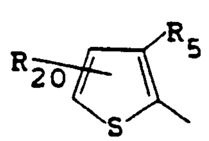,

L-4

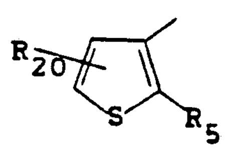,

L-5

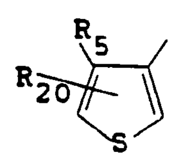,

L-6

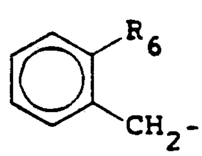,

L-7

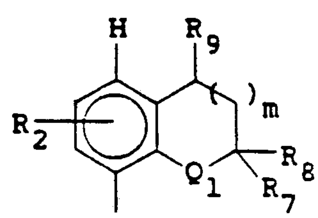,

L-8

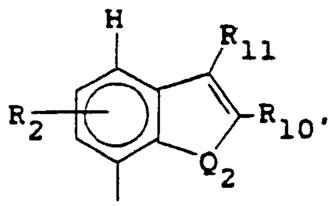,

L-9

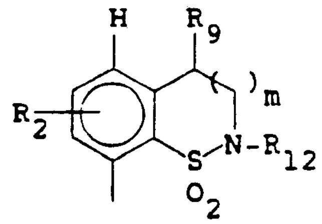,

L-10

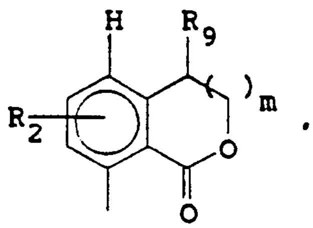,

L-11

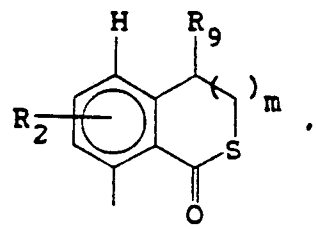,

L-12

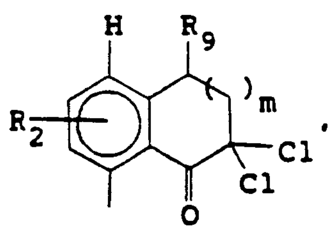,

L-13

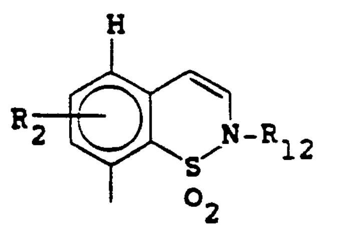,

L-14

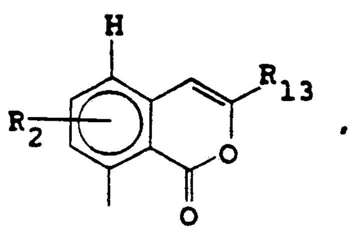,

L-15

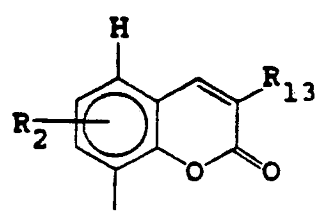

L-16

or 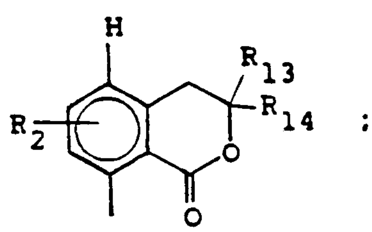 ;

L-17

J is 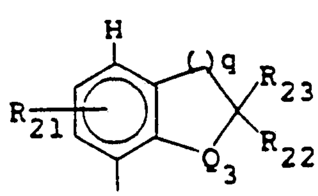 or 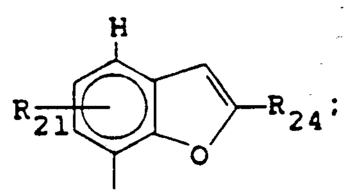 ;

J-1

J-2

$R_1$ is $C_1$—$C_4$ alkyl, $C_1$—$C_4$ alkoxy, $OCH_2CH_2OCH_3$, F, Cl, Br, $NO_2$, $CF_3$, $CO_2R_{15}$, $SO_2NR_{16}R_{17}$, $C(O)R_{25}$, $CR_{25}(OR_{26})$, $SO_2N(OCH_3)CH_3$, $OSO_2R_{18}$, $S(O)_nR_{19}$, $WCF_3$, $WCHF_2$, $C_3$—$C_4$ alkenyloxy, $C_3$—$C_4$ alkynyloxy, $C_1$—$C_2$ alkyl substituted with $OCH_3$ or $OCH_2CH_3$, $C_6H_5$,

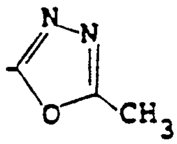, 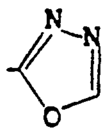, 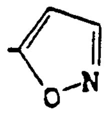, 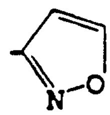, 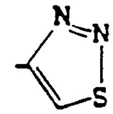,

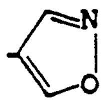, 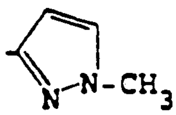, 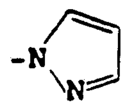, 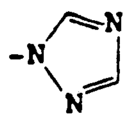, 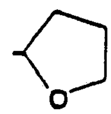,

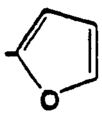, 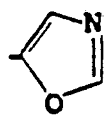, 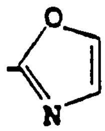, 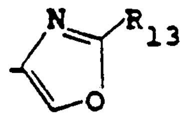, 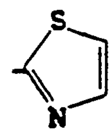,

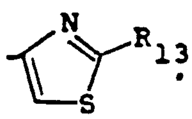, 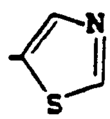, 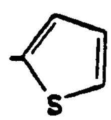, 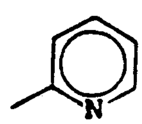 or 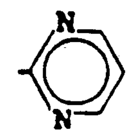;

$R_2$ is H, F, Cl, Br, $C_1$—$C_4$ alkyl, $C_1$—$C_4$ alkoxy, $C_3$—$C_4$ alkenyloxy, $C_3$—$C_4$ alkynyloxy, $C_1$—$C_4$ haloalkoxy, $C_1$—$C_4$ haloalkyl, $S(O)_pC_1$—$C_4$ alkyl, $S(O)_pC_3$—$C_4$ alkenyl, $S(O)_pC_3$—$C_4$ alkynyl or $C_1$—$C_2$ alkyl substituted with $OCH_3$ or $SCH_3$;

$R_3$ is H, $CH_3$, $OCH_3$, F, Cl, Br, $SO_2N(CH_3)_2$, $OSO_2CH_3$ or $S(O)_nCH_3$;

$R_4$ is $CH_3$, $CH_2CH_3$, $OCH_3$, $OCH_2CH_3$, F, Cl, Br, $SO_2NR_{16}R_{17}$, $SO_2N(OCH_3)CH_3$, $S(O)_nR_{19}$, $C_3$—$C_4$ alkenyloxy or $C_3$—$C_4$ alkynyloxy;

$R_5$ is $C_1$—$C_3$ alkyl, F, Cl, Br, $NO_2$, $CO_2R_{15}$, $SO_2NR_{16}R_{17}$, $SO_2N(OCH_3)CH_3$ or $S(O)_nR_{19}$;

$R_6$ is Cl, $NO_2$, $CO_2CH_3$, $CO_2CH_2CH_3$, $SO_2N(CH_3)_2$, $OSO_2CH_3$, $SO_2CH_3$, $SO_2CH_2CH_3$, $OCH_3$ or $OCH_2CH_3$;

$R_7$ is H, $CH_3$ or $CH_2CH_3$;

$R_8$ is H, $CH_3$ or $CH_2CH_3$;

$R_9$ is H or $CH_3$;

$R_{10}$ is H or $CH_3$;

$R_{11}$ is H or $CH_3$;

$R_{12}$ is H, $C_1$—$C_4$ alkyl, $C_3$—$C_4$ alkenyl, $C_3$—$C_4$ alkynyl, benzyl, $C_1$—$C_4$ alkylcarbonyl, $C_1$—$C_4$ alkoxycarbonyl, $CH_2CN$, $CH_2C(O)CH_3$, $CH_2CO_2(C_1$—$C_2$ alkyl), $C_1$—$C_4$ alkyl substituted with 0—3 F, 0—1 Cl, OH, $OCH_3$ or $OC_2H_5$ or $C_3$—$C_4$ alkenyl substituted with 1—3 F or 1—3 Cl;

$R_{13}$ is H or $CH_3$;

$R_{14}$ is $CH_3$ or $C_2H_5$;

$R_{15}$ is $C_1$—$C_4$ alkyl, $CH_2CH_2OCH_3$, $CH_2CH_2Cl$ or $CH_2CH = CH_2$;

$R_{16}$ is H, $C_1$—$C_3$ alkyl;

$R_{17}$ is $C_1$—$C_3$ alkyl;

$R_{18}$ is $C_1$—$C_3$ alkyl or $N(CH_3)_2$;

$R_{19}$ is $C_1$—$C_3$ alkyl or $CH_2CH = CH_2$;

$R_{20}$ is H, Cl or $CH_3$;

$R_{21}$ is H, Cl, $CH_3$ or $OCH_3$;

$R_{22}$ is H or $C_1$—$C_4$ alkyl;

$R_{23}$ is H or $CH_3$;

$R_{24}$ is H or $CH_3$;

$R_{25}$ is H, $C_1$—$C_4$ alkyl, $C_3$—$C_4$ alkenyl, $C_3$—$C_4$ alkynyl or $C_3$—$C_6$ cycloalkyl;

$R_{26}$ is $C_1$—$C_2$ alkyl;

m is 0 or 1;

n is 0 or 2;

p is 0, 1 or 2;

q is 1 or 2;

$Q_1$ is O, S or $SO_2$;
$Q_2$ is O or S;
$Q_3$ is O, S, SO or SO2; and
W is O, S or $SO_2$;

provided that

1) the total number of carbon atoms of $R_{16}$ and $R_{17}$ is less than or equal to four;
2) when m is 1, then $R_9$ is H; and
3) when $R_{23}$ is $CH_3$, then $Q_3$ is $SO_2$ and $R_{22}$ is $CH_3$;

and their agriculturally suitable salts.

Preferred for reasons of their higher herbicidal activity, greater plant growth regulant activity or more favorable ease of synthesis are:

1) Compounds of *Formula 1* where R is $CH_3$.
2) Compounds of *Preferred 1* where L is L-1, L-2, L-3, L-5, L-8, L-10, L-11, L-16 or L-17;
3) Compounds of *Preferred 2* where L is L-1;
$R_1$ is $OCH_3$, $OC_2H_5$, Cl, $NO_2$, $CF_3$, $CO_2CH_3$, $CO_2C_2H_5$, $SO_2N(CH_3)_2$, $SO_2N(OCH_3)CH_3$, $OSO_2R_{18}$, $C(O)CH_3$, $C(O)C_2H_5$, $S(O)_nR_{19}$, $OCF_2H$, $SCF_2H$, $OCH_2CH=CH_2$, $OCH_2C{\equiv}CH$,

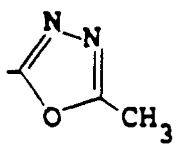 , 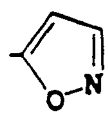 , 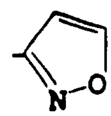 or 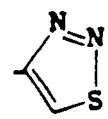 ;

$R_2$ is H, F, Cl, Br, $CH_3$, $C_2H_5$, $S(O)_pC_1$—$C_2$ alkyl, $S(O)_pCH_2CH=CH_2$, $S(O)_pCH_2C{\equiv}CH$, $C_1$—$C_3$ alkoxy, $OCH_2CH=CH_2$, $OCH_2C{\equiv}CH$, $C_1$—$C_2$ haloalkoxy, $C_1$—$C_2$ haloalkyl, $CH_2OCH_3$ or $CH_2SCH_3$;
$R_{18}$ is $C_1$—$C_3$ alkyl;
$R_{19}$ is $CH_3$ or $C_2H_5$; and
n is 2.

4) Compounds of *Preferred 3* where

L is 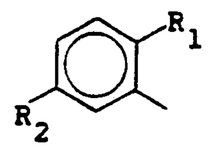 ;

and

$R_2$ is H, F, Cl, Br, $CH_3$, $CH_2CH_3$, $SCH_3$, $SC_2H_5$, $OCH_3$, $OC_2H_5$, $CF_3$, $CH_2OCH_3$ or $CH_2SCH_3$.

5) Compounds of *Preferred 2* where
L is L-2; and
$R_3$ is Cl, $CH_3$, $OCH_3$, $SCH_3$ or Br.

6) Compounds of *Preferred 2* where
L is L-3; and
$R_4$ is Cl, $SO_2CH_3$ or $SO_2N(CH_3)_2$.

7) Compounds of *Preferred 2* where
L is L-5;
$R_5$ is $CO_2CH_3$ or $CO_2C_2H_5$; and
$R_{20}$ is H.

8) Compounds of *Preferred 2* where L is L-8;
m is O;
$R_2$ is H, F, Cl, Br, $CH_3$, $CH_2CH_3$, $SCH_3$, $SC_2H_5$, $OCH_3$, $OC_2H_5$, $CF_3$, $CH_2OCH_3$ or $CH_2SCH_3$; and
$R_9$ is H.

9) Compounds of *Preferred 2* where
L is L-10;
$R_2$ is H, F, Cl, Br, $CH_3$, $CH_2CH_3$, $SCH_3$, $SC_2H_5$, $OCH_3$, $OC_2H_5$, $CF_3$, $CH_2OCH_3$ or $CH_2SCH_3$.
$R_9$ is H; and
$R_{12}$ is H, $C_1$—$C_4$ alkyl, $C_1$—$C_4$ alkylcarbonyl, $CH_2F$ or $C_2$—$C_3$ alkyl substituted with 1 F or 1 Cl.

10) Compounds of *Preferred 2* where
L is L-11;
$R_2$ is H, F, Cl, Br, $CH_3$, $CH_2CH_3$, $SCH_3$, $SC_2H_5$, $OCH_3$, $OC_2H_5$, $CF_3$, $CH_2OCH_3$ or $SC_2SCH_3$; and
$R_9$ is H.

11) Compounds of *Preferred 2* where
L is L-16; and
$R_2$ is H, F, Cl, Br, $CH_3$, $CH_2CH_3$, $SCH_3$, $SC_2H_5$, $OCH_3$, $OC_2H_5$, $CF_3$, $CH_2OCH_3$ or $CH_2SCH_3$.

12) Compounds of *Preferred 2* where
L is L-17; and

$R_2$ is H, F, Cl, Br, $CH_3$, $CH_2CH_3$, $SCH_3$, $SC_2H_5$, $OCH_3$, $OC_2H_5$, $CF_3$, $CH_2OCH_3$ or $CH_2SCH_3$.

13) Compounds of Formula Ia where

J is J-1;

$R_{22}$ is H, $CH_3$ or $C_2H_5$; and

$R_{23}$ is H.

Specifically Preferred for reasons of their highest herbicidal activity, greatest growth regulant activity and/or most favorable ease of synthesis are:

- 2-[[(4-iodo-6-methoxypyrimidin-2-yl)aminocarbonyl)aminosulfonyl]benzoic acid, methyl ester, m.p. 194—196°C(d);
- N'-[(4-iodo-6-methoxypyrimidin-2-yl)aminocarbonyl]-N,N-dimethyl-1,2-benzenedisulfonamide, m.p. 231—232.5°C; and
- 2-[[(4-iodo-6-methoxypyrimidin-2-yl)aminocarbonyl]aminosulfonyl]-4-methylbenzoic acid, methyl ester, m.p. 185—186°C.
- N-[(4-bromo-6-methoxypyrimidin-2-yl)aminocarbonyl]-2,3-dihydrobenzo[b]thiophene-7-sulfonamide, 1,1-dioxide, m.p. 238—240°C.
- N-[(4-bromo-6-methoxypyrimidin-2-ylaminocarbonyl]-2,3-dihydro-2-methylbenzo[b]thiophene-7-sulfonamide, 1,1-dioxide, m.p. 225—227°C.

## Detailed Description of the Invention

### Synthesis

The compounds of Formula I can be prepared by one or more of the following methods described below in Equations 1, 4, 5 and 6. Reaction conditions are given by way of example only.

As shown in Equation 1 below, compounds of Formula I can be prepared by reacting an appropriately substituted sulfonyl isocyanate of Formula II with an aminoheterocycle of Formula III where L and R are as previously defined.

Equation 1

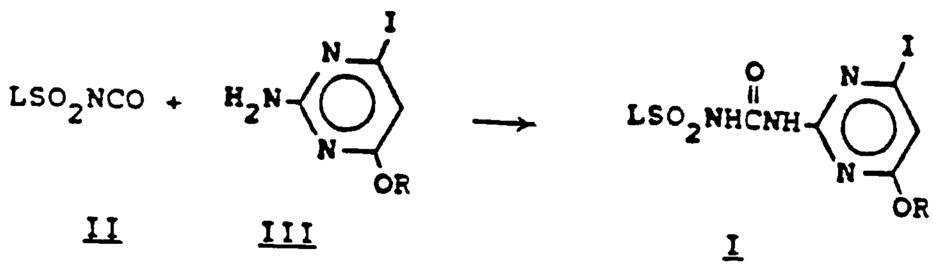

II III I

The reaction is best carried out in inert aprotic organic solvents such as dichloromethane, 1,2-dichloroethane, tetrahydrofuran, or acetonitrile, at a temperature between 20° and 85°C. The order of addition is not critical; however, it is often convenient to add the sulfonyl isocyanate or a solution of it in the reaction solvent to a stirred solution of the amine.

In some cases, the desired product is insoluble in the reaction solvent at ambient temperature and crystallizes from it in pure form. Products soluble in the reaction solvent are isolated by evaporation of the solvent. Compounds of Formula I then may be purified by trituration of the evaporation residue with solvents such as 1-chlorobutane or ethyl ether and filtration, by recrystallization from mixtures of solvents such as 1,2-dichloroethane, 1-chlorobutane and heptane or by chromatography on silica gel.

Sulfonylisocyanates of Formula II are known in the art and are prepared from the corresponding sulfonamides (IV) by one of the following two general methods.

Equation 2

$$\underset{IV}{LSO_2NH_2} \xrightarrow[COCl_2,\ cat]{CH_3(CH_2)_3NCO} \underset{II}{LSO_2NCO}$$

The sulfonamide IV and an alkyl isocyanate (e.g., *n*-butyl isocyanate) in xylene or other solvent boiling above 135°C are mixed in the presence or absence of a catalytic amount of 1,4-diaza[2.2.2]bicyclooctane (DABCO) and heated to 135—140°C. After 5—60 minutes phosgene is slowly added to the heated mixture at such a rate that the temperature remains between 133 and 135°C. When the consumption of phosgene has ceased, the mixture is cooled and filtered to remove insoluble material. Finally, the solvent, alkyl isocyanate, and excess phosgene are evaporated, leaving the sulfonyl isocyanate (II).

If desired, the alkyl isocyanate-sulfonamide adduct can be made and isolated before reaction with the phosgene. In this case the sulfonamide (IV), alkyl isocyanate, and anhydrous base (e.g., $K_2CO_3$) in a polar, aprotic solvent (e.g., acetone, butanone, or acetonitrile) are mixed and heated under reflux for 1 to 6 hours.

The reaction mixture is then diluted with water, and the pH is adjusted to about 3 with acid (e.g., HCl, $H_2SO_4$). The adduct is filtered out and dried, and then reacted with phosgene as described above. This procedure modification is especially useful when sulfonamide (IV) is high melting and has low solubility in the phosgenation solvent.

Sulfonyl isocyanates (II) can also be prepared by the following method.

Equation 3

$$\text{(a)}\quad \underset{\text{(IV)}}{LSO_2NH_2} \xrightarrow{SOCl_2} \underset{\text{(V)}}{LSO_2NSO}$$

$$\text{(b)}\quad \text{(V)} \xrightarrow[\text{pyridine cat.}]{COCl_2,} \underset{\text{(II)}}{LSO_2NCO}$$

The sulfonamide (IV) is heated at reflux in an excess of thionyl chloride. The reaction is continued until the sulfonamide protons are no longer detectable in the proton magnetic resonance spectrum. From 16 hours to 5 days is typically sufficient for complete conversion to the thionylamide (V) (Equation 3a).

The thionyl chloride is evaporated and the residue is treated with an inert solvent (e.g., toluene) containing at least one equivalent (typically 2—3 equivalents) of phosgene. A catalytic amount of pyridine (typically 0.1 equivalent) is added, and the mixture is heated to about 60—140°C. with 80—100° preferred. Conversion to the isocyanate (II) is usually substantially complete within 15 minutes to 3 hours (Equation 3b). The mixture is then cooled and filtered, and the solvent is evaporated, leaving the sulfonyl isocyanate (II).

Many of the compounds of Formula I can be prepared by the procedure shown in Equation 4.

Equation 4

$$\underset{\text{VI}}{LSO_2NHC(=O)OC_6H_5} + \text{III} \longrightarrow \text{I}$$

The reaction shown in Equation 4 is carried out by contacting phenylcarbamates of Formula VI with aminoheterocycles of Formula III in an inert organic solvent such as dioxane or tetrahydrofuran at temperatures of about 20—100°C for a period of about one-half to twenty-four hours. The product can be isolated by evaporation of the reaction solvent and purified by methods previously described.

Phenyl carbamates of Formula VI can be prepared by the methods described, or modifications thereof known to those skilled in the art, in European Patent Application 81810282.4 (Publ. No. 44,808), published January 27, 1982; or South African Patent Application 825042.

Alternatively, many of the compounds of Formula I can be prepared by the method described in Equation 5.

Equation 5

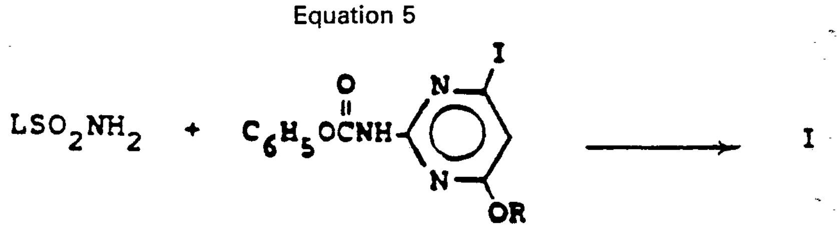

IV VII

The reaction of Equation 5 can be carried out by contacting equimolar amounts of a sulfonamide of Formula IV with a heterocyclic phenylcarbamate of Formula VII in the presence of a base such as 1,8-diaza-bicyclo[5.4.0]undec-7-ene (DBU), by methods analogous to those described in South African Patent Application 830441. The phenyl carbamates of Formula VII can be prepared by methods, or modifications thereof known to those skilled in the art, described in South African Patent Application 825671 and South African Patent Application 825045.

Rarely, a sulfonamide of Formula IV may not be of sufficient stability to be useful as a starting material in any of the above procedures. In these cases, as well as others, the sulfonyl isocyanate of Formula II can be prepared as an unisolated intermediate by treating the corresponding sulfonyl chloride (VIII) with isocyanate anion in the presence of the heterocyclic amine of Formula III as shown in Equation 6.

Equation 6

$$LSO_2Cl \quad + NCO^{\ominus} + III \longrightarrow I$$

VIII

The reaction is best carried out by adding over one to six hours a solution of at least one equivalent of a tetraalkylammonium isocyanate, such as tetra-*n*-butylammonium isocyanate, in a suitable aprotic organic solvent, such as dichloromethane or tetrahydrofuran, to a well-stirred mixture of one equivalent of sulfonyl chloride of Formula VIII and at least one equivalent of heterocyclic amine of Formula III in a similar suitable organic solvent at 20—40°C. The reaction mixture is then diluted with dichloromethane washed with 1*N* sulfuric acid, and dried over sodium sulfate. Rotary evaporation of the solvent leaves the product of Formula I in crude form. This may be purified as has already been described for Equation 1.

Certain sulfonyl chlorides are best prepared by chlorosulfonation of a substituted benzene, naphthalene, or thiophene in tetrachloromethane according to the teachings of H. T. Clarke et al., *Org. Synth. Coll.,* Vol. 1, 2nd Ed. 1941, p. 85. Other sulfonyl chlorides can be made by diazotization of the appropriate amine with sodium nitrite in hydrochloric acid, followed by reaction of the diazonium salt with sulfur dioxide and cuprous or cupric chloride in acetic acid according to the teachings of H. L. Yale and F. Sowinski, *J. Org. Chem., 25,* 1824 (1960) and of H. Meerwein et al., *Chem. Ber., 90,* 841 (1957).

Reference to the following patents is suggested for further details regarding the preparation of the sulfonamides (IV) and sulfonyl isocyanates (II): U.S. Patent 4,169,719; U.S. Patent 4,127,405; U.S. Patent 4,394,506; and E.P.—A—79,683.

Iodopyrimidines of Formula III can be prepared by the procedure shown in Equation 7.

Equation 7

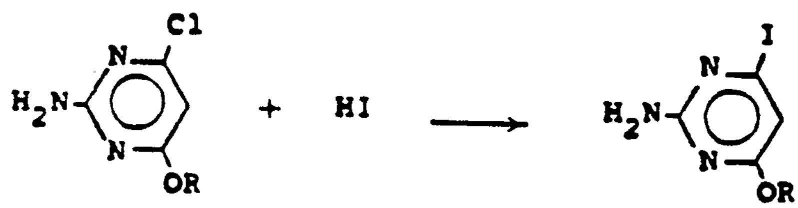

IX III

Treatment of a 2-amino-4-chloro-6-alkoxypyrimidines of Formula IX with 57% aqueous hydroiodic acid according to the procedures found in *J. Chem. Soc. C.* 1204 (1967) gives the iodopyrimidines of Formula III. The chloropyrimidines of Formula IX are known in the art.

Compounds of Formula Ia may be prepared utilizing the procedures of Equations 1—6, the corresponding sulfonylisocyanates of formula $JSO_2NCO$ or the sulfonamides of formula $JSO_2NH_2$ and the corresponding aminoheterocycle 2-amino-4-bromo-6-methoxypyrimidine. For the preparation of 2-amino-4-bromo-6-methoxypyrimidine see T. Hirayama, et al., *Heterocycles, 2,* 461 (1974).

Agriculturally suitable salts of compounds of Formula I are also useful herbicides and can be prepared in a number of ways known to the art. For example, metal salts can be made by contacting compounds of Formula I with a solution of an alkali or alkaline earth metal salt having a sufficiently basic anion (e.g., hydroxide, alkoxide, carbonate or hydroxide). Quaternary amine salts can be made by similar techniques.

Salts of compounds of Formula I can also be prepared by exchange of one cation for another. Cationic exchange can be effected by direct contact of an aqueous solution of a salt of a compound of Formula I (e.g., alkali or quaternary amine salt) with a solution containing the cation to be exchanged. This method is most effective when the desired salt containing the exchanged cation is insoluble in water and can be separated by filtration.

Exchange may also be effected by passing an aqueous solution of a salt of a compound of Formula I (e.g., an alkali metal or quaternary amine salt) through a column packed with a cation exchange resin containing the cation to be exchanged for that of the original salt and the desired product is eluted from the column. This method is particularly useful when the desired salt is water-soluble.

Acid addition salts, useful in this invention, can be obtained by reacting a compound of Formula I with a suitable acid, e.g., *p*-toluenesulfonic acid, trichloroacetic acid or the like.

The synthetic routes described above are equally applicable, *mutatis mutandis,* to the preparation of compounds of Formula Ia and their agriculturally suitable salts.

The following examples further illustrate the synthesis of this invention.

Example 1

2-Amino-4-iodo-6-methoxypyrimidine

18.0 g of 2-amino-4-chloro-6-methoxypyrimidine was added to 65 mL of 57% aqueous hydroiodic acid with ice bath cooling. The resulting thick mixture was kept at ambient temperature for 65 hours, diluted

with water and neutralized with potassium carbonate. The solid was collected and recrystallized from aqueous ethanol to give 5.0 g of white crystals, m.p. 141.5—144°C.

NMR ($CDCl_3$): 3.82 (s, 3H, $OCH_3$);
5.3 (br s, 2H, $NH_2$); and
6.54 (s, 1H, pyr C5—H).

Example 2

2-[[(4-iodo-6-methoxypyrimidin-2-yl)aminocarbonyl]aminosulfonyl]benzoic acid, methyl ester

To a solution of 0.25 g of 2-amino-4-iodo-6-methoxypyrimidine in 5 mL of dry methylene chloride was added 0.30 g of 2-carbomethoxybenzenesulfonyl isocyanate. The resulting solution was allowed to stand for 20 hours at ambient temperature. The solution was cooled in an ice bath and crystallization induced with scratching. The crystals were collected, washed with *n*-butylchloride and dried to afford 0.10 g of the title compound, m.p. 194—196°C (dec).

IR (nujol) 1728 $cm^{-1}$ (ester) and
1705 $cm^{-1}$ (sulfonylurea).

NMR ($CDCl_3$): 3.94 (s, 3H, $CO_2CH_3$);
4.06 (s, 3H, $OCH_3$);
6.95 (s, 1H, pyr. C5—H);
7.4 (br s, 1H, NH);
7.75 (m, 3H, aromatics);
8.4 (m, 1H, aromatic); and
12.15 (br s, 1H, NH).

Example 3

N-[(4-Bromo-6-methoxypyrimidin-2-yl)aminocarbonyl]-2,3-dihydrobenzo[b]thiophene-7-sulfonamide, 1,1-dioxide

A suspension of 5.0 g (20 mmol) of 2,3-dihydrobenzo[b]thiophene-7-sulfonamide-1,1-dioxide in 50 mL of thionyl chloride was refluxed for 40 hours, cooled, diluted with 100 mL of dry toluene and fractionally distilled to remove excess thionyl chloride. The cooled toluene solution was contacted with 2 drops of pyridine and 3 mL of liquified phosgene and the mixture was refluxed for 3 hours, cooled and concentrated *in vacuo* to give 5.8 g of 2,3-dihydrobenzo[b]thiophene-7-sulfonylisocyanate-1,1-dioxide as a brown powder whose IR spectrum exhibited a strong band at 2250 $cm^{-1}$.

A solution of 1.4 g (5 mmol) of the crude isocyanate and 0.82 g (4 mmol) of 2-amino-4-bromo-6-methoxypyrimidine in 20 mL of dry methylene chloride was heated to reflux for 5 minutes and stirred at room temperature for 16 hours. The resulting precipitate was filtered, washed with dry methylene chloride and anhydrous ether and air dried to give 1.6 g of the title compound as a white powder, m.p. 238—240°C.

200 MHz $^1H$ NMR (DMSO-$d_6$): δ
12.2 (br, 1H, NH);
10.9 (b, 1H, NH);
8.1 (m, 1H, arom);
7.9 (m, 2H, arom);
7.0 (s, 1H, CH),
4.0 (s. 3H, $OCH_3$);
3.7 (m, 2H, $CH_2$); and
3.4 (m, 2H, $CH_2$) ppm.

IR (nujol): 3380, 1730, 1710 $cm^{-1}$.

Using the procedures and examples shown above, the compounds in Tables 1—19 can be prepared.

## General Structures for Tables

### General Structure 1

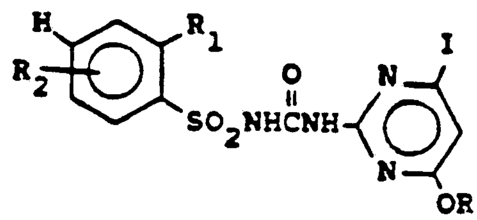

### General Structure 2

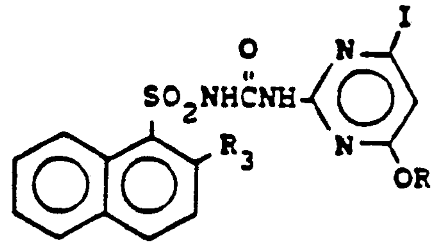

### General Structure 3

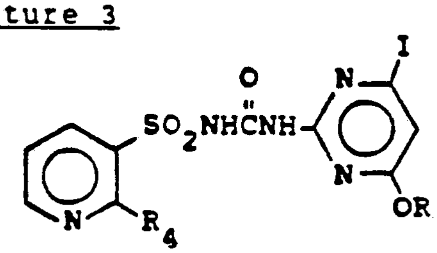

### General Structure 4

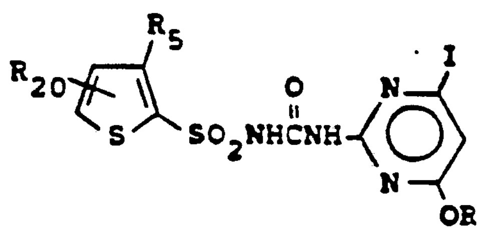

### General Structure 5

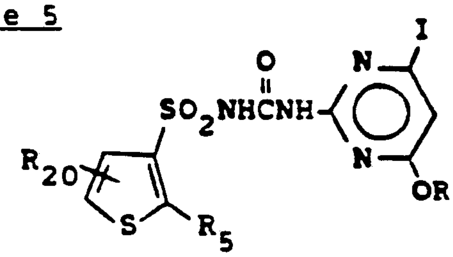

General Structures for Tables (continued)

General Structure 6

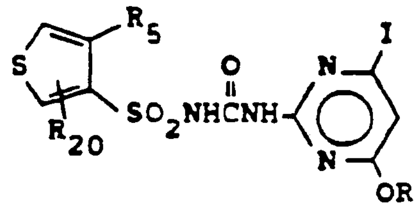

General Structure 7

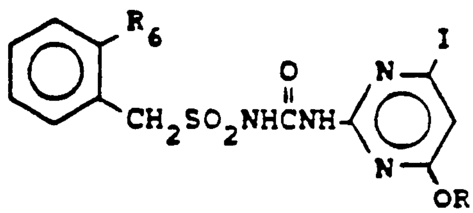

General Structure 8

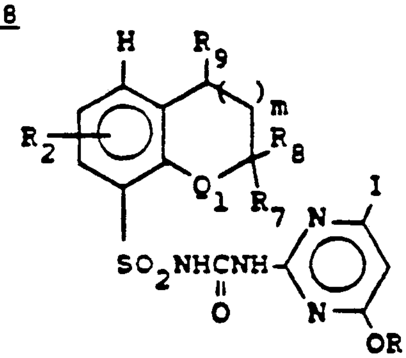

General Structure 9

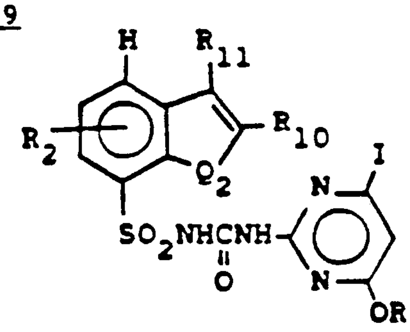

General Structures for Tables (continued)

General Structure 10

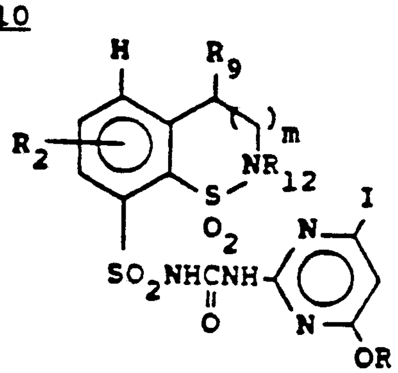

General Structure 11

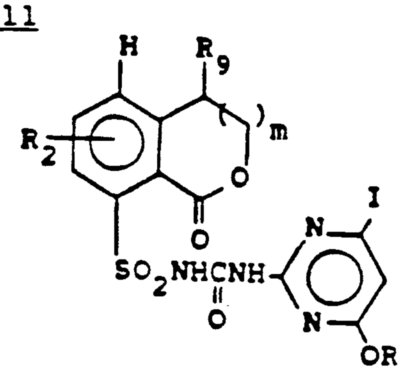

General Structure 12

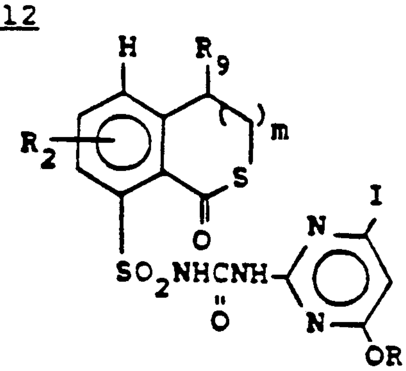

General Structure 13

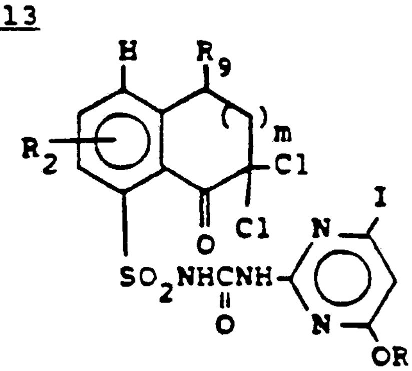

General Structures for Tables (continued)

General Structure 14

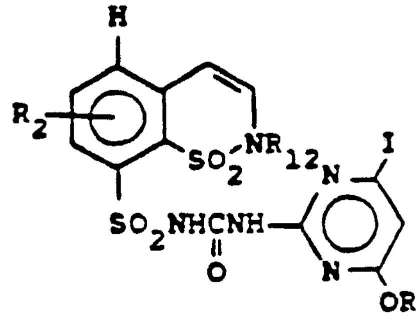

General Structure 15

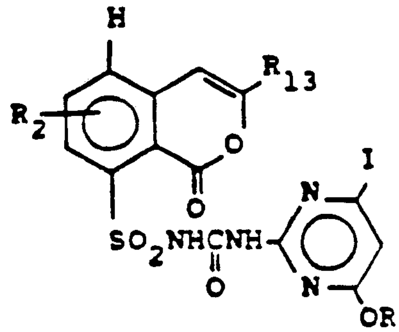

General Structure 16

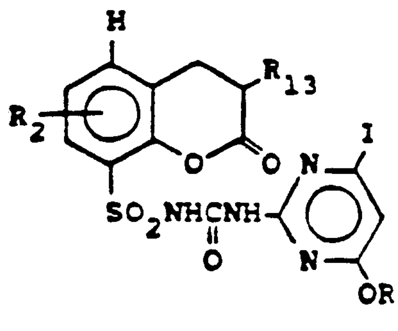

General Structure 17

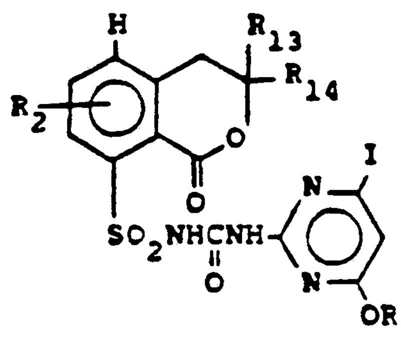

General Structures for Tables (continued)

General Structure 18

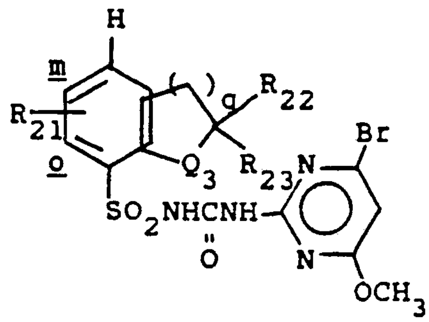

General Structure 19

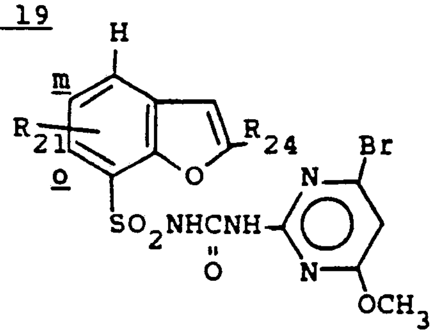

Table 1

General Structure 1

| R | $R_1$ | $R_2$ | m.p. (°C) |
|---|---|---|---|
| $CH_3$ | $CH_3$ | H | |
| $CH_3$ | $CH_2CH_3$ | H | |
| $CH_3$ | $CH_2CH_2CH_3$ | H | |
| $CH_3$ | $CH(CH_3)_2$ | H | |
| $CH_3$ | $CH(CH_3)CH_2CH_3$ | H | |
| $CH_3$ | $OCH_3$ | H | |
| $CH_3$ | $OCH_2CH_3$ | H | |
| $CH_3$ | $OCH_2CH_2CH_3$ | H | |
| $CH_3$ | $OCH(CH_3)_2$ | H | |
| $CH_3$ | $OCH_2CH_2CH_2CH_3$ | H | |
| $CH_3$ | $OCH_2CH_2OCH_3$ | H | |
| $CH_3$ | F | H | |
| $CH_3$ | Cl | H | 197-200 (dec) |
| $CH_3$ | Br | H | |
| $CH_3$ | $NO_2$ | H | 223.5-225 (dec) |
| $CH_3$ | $CF_3$ | H | 217-220 (dec) |
| $CH_3$ | $CO_2CH_3$ | H | 194-196 (dec) |
| $CH_3$ | $CO_2CH_3$ | 5-F | |
| $CH_3$ | $CO_2CH_3$ | 3-Cl | |
| $CH_3$ | $CO_2CH_3$ | 5-Cl | |
| $CH_3$ | $CO_2CH_3$ | 6-Cl | |
| $CH_3$ | $CO_2CH_3$ | 5-Br | |
| $CH_3$ | $CO_2CH_3$ | 5-$CF_3$ | 148-150 |
| $CH_3$ | $CO_2CH_3$ | 5-$CH_3$ | 185-187 |
| $CH_3$ | $CO_2CH_3$ | 5-$OCH_3$ | 195-197 |
| $CH_3$ | $CO_2CH_3$ | 5-$OCH_2CH_3$ | |
| $CH_3$ | $CO_2CH_3$ | 5-$SCH_3$ | |
| $CH_3$ | $CO_2CH_3$ | 5-$OCF_2H$ | |
| $CH_3$ | $CO_2CH_3$ | 3-$CH_3$ | |
| $CH_3$ | $CO_2CH_2CH_3$ | H | 186-187.5 (dec) |
| $CH_3$ | $CO_2CH_2CH_3$ | 3-Cl | |

Table 1 (continued)

| R | $R_1$ | $R_2$ | m.p. (°C) |
|---|---|---|---|
| $CH_3$ | $CO_2CH_2CH_3$ | 5-Cl | |
| $CH_3$ | $CO_2CH_2CH_3$ | 6-Cl | |
| $CH_3$ | $CO_2CH_2CH_3$ | 5-$CF_3$ | |
| $CH_3$ | $CO_2CH_2CH_3$ | 3-$CH_3$ | |
| $CH_3$ | $CO_2CH_2CH_3$ | 5-$CH_3$ | |
| $CH_3$ | $CO_2CH_2CH_3$ | 5-$OCH_3$ | |
| $CH_3$ | $CO_2CH_2CH_3$ | 5-$SCH_3$ | |
| $CH_3$ | $CO_2CH_2CH_3$ | 5-$OCF_2H$ | |
| $CH_3$ | $CO_2CH_2CH_3$ | 5-$OCH_2CH_3$ | |
| $CH_3$ | $CO_2CH(CH_3)_2$ | 3-Cl | |
| $CH_3$ | $CO_2CH(CH_3)_2$ | 5-Cl | |
| $CH_3$ | $CO_2CH(CH_3)_2$ | 6-Cl | |
| $CH_3$ | $CO_2CH(CH_3)_2$ | 5-$CF_3$ | |
| $CH_3$ | $CO_2CH(CH_3)_2$ | 3-$CH_3$ | |
| $CH_3$ | $CO_2CH(CH_3)_2$ | 5-$CH_3$ | |
| $CH_3$ | $CO_2CH(CH_3)_2$ | 5-$OCH_3$ | |
| $CH_3$ | $CO_2CH(CH_3)_2$ | 5-$OCF_2H$ | |
| $CH_3$ | $CO_2CH_2CH_2CH_3$ | H | |
| $CH_3$ | $CO_2CH_2CH_2CH_3$ | 5-$OCH_3$ | |
| $CH_3$ | $CO_2CH_2CH_2CH_3$ | 5-$CH_3$ | |
| $CH_3$ | $CO_2CH_2CH(CH_3)_2$ | H | |
| $CH_3$ | $CO_2CH(CH_3)CH_2CH_3$ | H | |
| $CH_3$ | $CO_2CH_2CH_2CH_2CH_3$ | H | |
| $CH_3$ | $CO_2CH_2CH_2OCH_3$ | H | |
| $CH_3$ | $CO_2CH_2CH_2OCH_3$ | 5-$CF_3$ | |
| $CH_3$ | $CO_2CH_2CH_2OCH_3$ | 5-Cl | |
| $CH_3$ | $CO_2CH_2CH_2OCH_3$ | 5-$OCH_3$ | |
| $CH_3$ | $CO_2CH_2CH_2Cl$ | 5-$CH_3$ | |
| $CH_3$ | $CO_2CH_2CH_2Cl$ | H | |
| $CH_3$ | $CO_2CH_2CH_2Cl$ | 5-$OCH_3$ | |
| $CH_3$ | $CO_2CH_2CH{=}CH_2$ | H | |
| $CH_3$ | $CO_2CH_2CH{=}CH_2$ | 5-$CF_3$ | |

Table 1 (continued)

| R | $R_1$ | $R_2$ | m.p. (°C) |
|---|---|---|---|
| $CH_3$ | $CO_2CH_2CH{=}CH_2$ | 5-$OCH_3$ | |
| $CH_3$ | $SO_2N(CH_3)_2$ | H | 231-232.5 (dec) |
| $CH_3$ | $SO_2N(CH_3)_2$ | 3-Cl | |
| $CH_3$ | $SO_2N(CH_3)CH_2CH_3$ | H | |
| $CH_3$ | $SO_2N(CH_2CH_3)_2$ | H | |
| $CH_3$ | $SO_2N(CH_3)CH(CH_3)_2$ | H | |
| $CH_3$ | $SO_2N(CH_3)CH_2CH_2CH_3$ | H | |
| $CH_3$ | $OSO_2CH_3$ | H | 217-219 (dec) |
| $CH_3$ | $OSO_2CH_3$ | 5-Cl | |
| $CH_3$ | $OSO_2CH_2CH_3$ | H | |
| $CH_3$ | $OSO_2CH(CH_3)_2$ | H | |
| $CH_3$ | $OSO_2CH_2CH_2CH_3$ | H | |
| $CH_3$ | $OSO_2N(CH_3)_2$ | H | |
| $CH_3$ | $SCH_3$ | H | 116-120 |
| $CH_3$ | $SCH_2CH_3$ | H | |
| $CH_3$ | $SCH_2CH_2CH_3$ | H | |
| $CH_3$ | $SCH(CH_3)_2$ | H | |
| $CH_3$ | $SCH_2CH{=}CH_2$ | H | |
| $CH_3$ | $SOCH_3$ | H | |
| $CH_3$ | $SOCH_2CH_3$ | H | |
| $CH_3$ | $SO_2CH_3$ | H | 239-241 (dec) |
| $CH_3$ | $SO_2CH_2CH_3$ | H | |
| $CH_3$ | $SO_2CH_2CH_2CH_3$ | H | |
| $CH_3$ | $SO_2CH(CH_3)_2$ | H | |
| $CH_3$ | $SO_2CH_2CH{=}CH_2$ | H | |
| $CH_3$ | $SCF_3$ | H | |
| $CH_3$ | $SO_2CF_3$ | H | |
| $CH_3$ | $OCF_3$ | H | |
| $CH_3$ | $SCF_2H$ | H | |
| $CH_3$ | $SO_2CF_2H$ | H | |
| $CH_3$ | $OCF_2H$ | H | |
| $CH_3$ | $OCH_2CH{=}CH_2$ | H | |

Table 1 (continued)

| R | $R_1$ | $R_2$ | m.p. (°C) |
|---|---|---|---|
| $CH_3$ | $OCH_2CH=CHCH_3$ | H | |
| $CH_3$ | $OCH(CH_3)CH=CH_2$ | H | |
| $CH_3$ | $OCH_2C\equiv CH$ | H | |
| $CH_3$ | $OCH_2C\equiv CCH_3$ | H | |
| $CH_3$ | $OCH(CH_3)C\equiv CH$ | H | |
| $CH_3$ | $CH_2OCH_3$ | H | |
| $CH_3$ | $CH_2OCH_2CH_3$ | H | |
| $CH_3$ | $CH_2CH_2OCH_3$ | H | |
| $CH_3$ | $CH(CH_3)OCH_3$ | H | |
| $CH_3$ | $C_6H_5$ | H | |
| $CH_3$ | 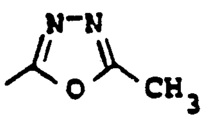 | H | |
| $CH_3$ | 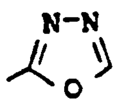 | H | |
| $CH_3$ | 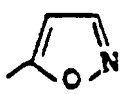 | H | |
| $CH_3$ | 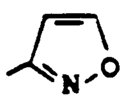 | H | |
| $CH_3$ | 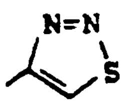 | H | |
| $CH_3$ | 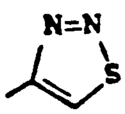 | 5-Cl | |
| $CH_3$ | 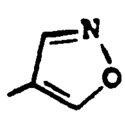 | H | |
| $CH_3$ | 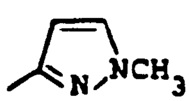 | H | |
| $CH_3$ | 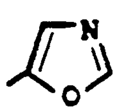 | H | |

Table 1 (continued)

| R | $R_1$ | $R_2$ | m.p. (°C) |
|---|---|---|---|
| $CH_3$ | 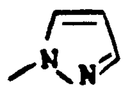 | H | |
| $CH_3$ | 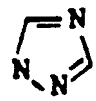 | H | |
| $CH_3$ | 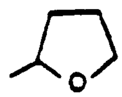 | H | |
| $CH_3$ | 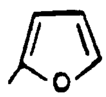 | H | |
| $CH_3$ | 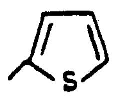 | H | |
| $CH_3$ | 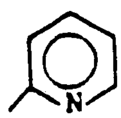 | H | |
| $CH_3$ | 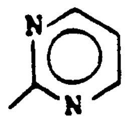 | H | |
| $CH_2CH_3$ | $CH_3$ | H | |
| $CH_2CH_3$ | $CH_2CH_3$ | H | |
| $CH_2CH_3$ | $CH_2CH_2CH_3$ | H | |
| $CH_2CH_3$ | $CH(CH_3)_2$ | H | |
| $CH_2CH_3$ | $CH(CH_3)CH_2CH_3$ | H | |
| $CH_2CH_3$ | $OCH_3$ | H | |
| $CH_2CH_3$ | $OCH_2CH_3$ | H | |
| $CH_2CH_3$ | $OCH_2CH_2CH_3$ | H | |
| $CH_2CH_3$ | $OCH(CH_3)_2$ | H | |
| $CH_2CH_3$ | $OCH_2CH_2CH_2CH_3$ | H | |
| $CH_2CH_3$ | $OCH_2CH_2OCH_3$ | H | |
| $CH_2CH_3$ | F | H | |

Table 1 (continued)

| R | $R_1$ | $R_2$ | m.p. (°C) |
|---|---|---|---|
| $CH_2CH_3$ | Cl | H | |
| $CH_2CH_3$ | Br | H | |
| $CH_2CH_3$ | $NO_2$ | H | |
| $CH_2CH_3$ | $CF_3$ | H | |
| $CH_2CH_3$ | $CO_2CH_3$ | H | |
| $CH_2CH_3$ | $CO_2CH_3$ | 5-F | |
| $CH_2CH_3$ | $CO_2CH_3$ | 3-Cl | |
| $CH_2CH_3$ | $CO_2CH_3$ | 5-Cl | |
| $CH_2CH_3$ | $CO_2CH_3$ | 6-Cl | |
| $CH_2CH_3$ | $CO_2CH_3$ | 5-Br | |
| $CH_2CH_3$ | $CO_2CH_3$ | 5-$CF_3$ | |
| $CH_2CH_3$ | $CO_2CH_3$ | 5-$CH_3$ | |
| $CH_2CH_3$ | $CO_2CH_3$ | 5-$OCH_3$ | |
| $CH_2CH_3$ | $CO_2CH_3$ | 5-$OCH_2CH_3$ | |
| $CH_2CH_3$ | $CO_2CH_3$ | 5-$SCH_3$ | |
| $CH_2CH_3$ | $CO_2CH_3$ | 5-$OCF_2H$ | |
| $CH_2CH_3$ | $CO_2CH_3$ | 3-$CH_3$ | |
| $CH_2CH_3$ | $CO_2CH_2CH_3$ | H | |
| $CH_2CH_3$ | $CO_2CH_2CH_3$ | 3-Cl | |
| $CH_2CH_3$ | $CO_2CH_2CH_3$ | 5-Cl | |
| $CH_2CH_3$ | $CO_2CH_2CH_3$ | 6-Cl | |
| $CH_2CH_3$ | $CO_2CH_2CH_3$ | 5-$CF_3$ | |
| $CH_2CH_3$ | $CO_2CH_2CH_3$ | 3-$CH_3$ | |
| $CH_2CH_3$ | $CO_2CH_2CH_3$ | 5-$CH_3$ | |
| $CH_2CH_3$ | $CO_2CH_2CH_3$ | 5-$OCH_3$ | |
| $CH_2CH_3$ | $CO_2CH_2CH_3$ | 5-$SCH_3$ | |
| $CH_2CH_3$ | $CO_2CH_2CH_3$ | 5-$OCF_2H$ | |
| $CH_2CH_3$ | $CO_2CH_2CH_3$ | 5-$OCH_2CH_3$ | |
| $CH_2CH_3$ | $CO_2CH(CH_3)_2$ | 3-Cl | |
| $CH_2CH_3$ | $CO_2CH(CH_3)_2$ | 5-Cl | |
| $CH_2CH_3$ | $CO_2CH(CH_3)_2$ | 6-Cl | |
| $CH_2CH_3$ | $CO_2CH(CH_3)_2$ | 5-$CF_3$ | |

Table 1 (continued)

| R | $R_1$ | $R_2$ | m.p. (°C) |
|---|---|---|---|
| $CH_2CH_3$ | $CO_2CH(CH_3)_2$ | $3-CH_3$ | |
| $CH_2CH_3$ | $CO_2CH(CH_3)_2$ | $5-CH_3$ | |
| $CH_2CH_3$ | $CO_2CH(CH_3)_2$ | $5-OCH_3$ | |
| $CH_2CH_3$ | $CO_2CH(CH_3)_2$ | $5-OCF_2H$ | |
| $CH_2CH_3$ | $CO_2CH_2CH_2CH_3$ | H | |
| $CH_2CH_3$ | $CO_2CH_2CH_2CH_3$ | $5-OCH_3$ | |
| $CH_2CH_3$ | $CO_2CH_2CH_2CH_3$ | $5-CH_3$ | |
| $CH_2CH_3$ | $CO_2CH_2CH(CH_3)_2$ | H | |
| $CH_2CH_3$ | $CO_2CH(CH_3)CH_2CH_3$ | H | |
| $CH_2CH_3$ | $CO_2CH_2CH_2CH_2CH_3$ | H | |
| $CH_2CH_3$ | $CO_2CH_2CH_2OCH_3$ | H | |
| $CH_2CH_3$ | $CO_2CH_2CH_2OCH_3$ | $5-CF_3$ | |
| $CH_2CH_3$ | $CO_2CH_2CH_2OCH_3$ | 5-Cl | |
| $CH_2CH_3$ | $CO_2CH_2CH_2OCH_3$ | $5-OCH_3$ | |
| $CH_2CH_3$ | $CO_2CH_2CH_2Cl$ | $5-CH_3$ | |
| $CH_2CH_3$ | $CO_2CH_2CH_2Cl$ | H | |
| $CH_2CH_3$ | $CO_2CH_2CH_2Cl$ | $5-OCH_3$ | |
| $CH_2CH_3$ | $CO_2CH_2CH=CH_2$ | H | |
| $CH_2CH_3$ | $CO_2CH_2CH=CH_2$ | $5-CF_3$ | |
| $CH_2CH_3$ | $CO_2CH_2CH=CH_2$ | $5-OCH_3$ | |
| $CH_2CH_3$ | $SO_2N(CH_3)_2$ | H | |
| $CH_2CH_3$ | $SO_2N(CH_3)_2$ | 3-Cl | |
| $CH_2CH_3$ | $SO_2N(CH_3)CH_2CH_3$ | H | |
| $CH_2CH_3$ | $SO_2N(CH_2CH_3)_2$ | H | |
| $CH_2CH_3$ | $SO_2N(CH_3)CH(CH_3)_2$ | H | |
| $CH_2CH_3$ | $SO_2N(CH_3)CH_2CH_2CH_3$ | H | |
| $CH_2CH_3$ | $OSO_2CH_3$ | H | |
| $CH_2CH_3$ | $OSO_2CH_3$ | 5-Cl | |
| $CH_2CH_3$ | $OSO_2CH_2CH_3$ | H | |
| $CH_2CH_3$ | $OSO_2CH(CH_3)_2$ | H | |
| $CH_2CH_3$ | $OSO_2CH_2CH_2CH_3$ | H | |
| $CH_2CH_3$ | $OSO_2N(CH_3)_2$ | H | |

Table 1 (continued)

| R | $R_1$ | $R_2$ | m.p. (°C) |
|---|---|---|---|
| $CH_2CH_3$ | $SCH_3$ | H | |
| $CH_2CH_3$ | $SCH_2CH_3$ | H | |
| $CH_2CH_3$ | $SCH_2CH_2CH_3$ | H | |
| $CH_2CH_3$ | $SCH(CH_3)_2$ | H | |
| $CH_2CH_3$ | $SCH_2CH=CH_2$ | H | |
| $CH_2CH_3$ | $SOCH_3$ | H | |
| $CH_2CH_3$ | $SOCH_2CH_3$ | H | |
| $CH_2CH_3$ | $SO_2CH_3$ | H | |
| $CH_2CH_3$ | $SO_2CH_2CH_3$ | H | |
| $CH_2CH_3$ | $SO_2CH_2CH_2CH_3$ | H | |
| $CH_2CH_3$ | $SO_2CH(CH_3)_2$ | H | |
| $CH_2CH_3$ | $SO_2CH_2CH=CH_2$ | H | |
| $CH_2CH_3$ | $SCF_3$ | H | |
| $CH_2CH_3$ | $SO_2CF_3$ | H | |
| $CH_2CH_3$ | $OCF_3$ | H | |
| $CH_2CH_3$ | $SCF_2H$ | H | |
| $CH_2CH_3$ | $SO_2CF_2H$ | H | |
| $CH_2CH_3$ | $OCF_2H$ | H | |
| $CH_2CH_3$ | $OCH_2CH=CH_2$ | H | |
| $CH_2CH_3$ | $OCH_2CH=CHCH_3$ | H | |
| $CH_2CH_3$ | $OCH(CH_3)CH=CH_2$ | H | |
| $CH_2CH_3$ | $OCH_2C\equiv CH$ | H | |
| $CH_2CH_3$ | $OCH_2C\equiv CCH_3$ | H | |
| $CH_2CH_3$ | $OCH(CH_3)C\equiv CH$ | H | |
| $CH_2CH_3$ | $CH_2OCH_3$ | H | |
| $CH_2CH_3$ | $CH_2OCH_2CH_3$ | H | |
| $CH_2CH_3$ | $CH_2CH_2OCH_3$ | H | |
| $CH_2CH_3$ | $CH(CH_3)OCH_3$ | H | |
| $CH_2CH_3$ | $C_6H_5$ | H | |
| $CH_2CH_3$ | 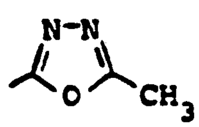 | H | |
| $CH_2CH_3$ | 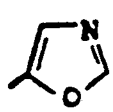 | H | |

Table 1 (continued)

| R | $R_1$ | $R_2$ | m.p. (°C) |
|---|---|---|---|
| $CH_2CH_3$ | 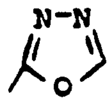 | H | |
| $CH_2CH_3$ | 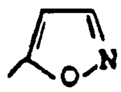 | H | |
| $CH_2CH_3$ | 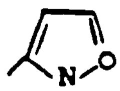 | H | |
| $CH_2CH_3$ | 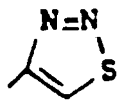 | H | |
| $CH_2CH_3$ | 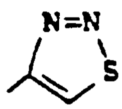 | 5-Cl | |
| $CH_2CH_3$ | 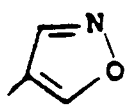 | H | |
| $CH_2CH_3$ | 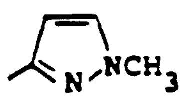 | H | |
| $CH_2CH_3$ | 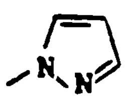 | H | |
| $CH_2CH_3$ | 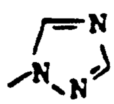 | H | |
| $CH_2CH_3$ | 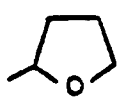 | H | |
| $CH_2CH_3$ | 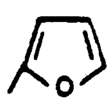 | H | |
| $CH_2CH_3$ | 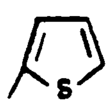 | H | |

Table 1 (continued)

| R | $R_1$ | $R_2$ | m.p. (°C) |
|---|---|---|---|
| $CH_2CH_3$ | 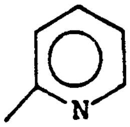 | H | |
| $CH_2CH_3$ | 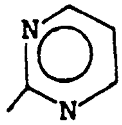 | H | |
| $CH_2CH_3$ | 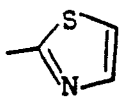 | H | |
| $CH_2CH_3$ | 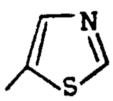 | H | |
| $CH_3$ | $CO_2CH_3$ | 5-$CH_2CH_3$ | |
| $CH_3$ | $CO_2CH_3$ | 5-$CH_2CH_2CH_2CH_3$ | |
| $CH_3$ | $CO_2CH_3$ | 5-$OCH_2CH_2CH_2CH_3$ | |
| $CH_3$ | $CO_2CH_3$ | 5-$OCH_2CH{=}CH_2$ | |
| $CH_3$ | $CO_2CH_3$ | 5-$OCH_2C{\equiv}CH$ | |
| $CH_3$ | $CO_2CH_3$ | 5-$OCH_2CHClCH_2CH_3$ | |
| $CH_3$ | $CO_2CH_3$ | 5-$SOCH_3$ | |
| $CH_3$ | $CO_2CH_3$ | 5-$SCH_2CH{=}CH_2$ | |
| $CH_3$ | $CO_2CH_3$ | 5-$SCH_2C{\equiv}CH$ | |
| $CH_3$ | $CO_2CH_3$ | 5-$SCH_2CH_2C{\equiv}CH$ | |
| $CH_3$ | $CO_2CH_3$ | 5-$CH_2OCH_3$ | |
| $CH_3$ | $CO_2CH_3$ | 5-$CH_2SCH_3$ | |
| $CH_3$ | $CO_2CH_3$ | 5-$CH_2CH_2CH_3$ | |
| $CH_3$ | $CO_2CH_3$ | 5-$OCH_2CH_2CH_3$ | |
| $CH_3$ | $CO_2CH_3$ | 5-$OCH_2CH_2CH{=}CH_2$ | |
| $CH_3$ | $CO_2CH_3$ | 5-$OCH_2CH_2C{\equiv}CH$ | |
| $CH_3$ | $CO_2CH_3$ | 5-$OCF_2CF_2H$ | |
| $CH_3$ | $CO_2CH_3$ | 5-$SO_2CH_3$ | |

Table 1 (continued)

| R | $R_1$ | $R_2$ | m.p. (°C) |
|---|---|---|---|
| $CH_3$ | $CO_2CH_3$ | 5-$SCH_2CH_2CH{=}CH_2$ | |
| $CH_3$ | $CO_2CH_3$ | 5-$CH_2CH_2OCH_3$ | |
| $CH_3$ | $CO_2CH_3$ | 5-$SO_2CH_2CH_3$ | |
| $CH_3$ | $CO_2CH_3$ | 5-$SO_2CH_2CH_2CH_2CH_3$ | |
| $CH_3$ | $CO_2CH_3$ | 5-$SO_2CH_2CH{=}CH_2$ | |
| $CH_3$ | $CO_2CH_3$ | 5-$SO_2CH_2C{\equiv}CH$ | |

Table 2

General Structure 2

| R | $R_3$ | m.p. (°C) |
|---|---|---|
| $CH_3$ | $CH_3$ | |
| $CH_2CH_3$ | $CH_3$ | |
| $CH_3$ | $OCH_3$ | |
| $CH_2CH_3$ | $OCH_3$ | |
| $CH_3$ | F | |
| $CH_3$ | H | |
| $CH_3$ | Cl | |
| $CH_2CH_3$ | Cl | |
| $CH_3$ | Br | |
| $CH_2CH_3$ | Br | |
| $CH_3$ | $SO_2N(CH_3)_2$ | |
| $CH_3$ | $OSO_2CH_3$ | |
| $CH_3$ | $SCH_3$ | |
| $CH_3$ | $SOCH_3$ | |
| $CH_3$ | $SO_2CH_3$ | |

Table 3

General Structure 3

| R | $R_4$ | m.p. (°C) |
|---|---|---|
| $CH_3$ | $CH_3$ | |
| $CH_3$ | $CH_2CH_3$ | |
| $CH_3$ | $OCH_3$ | |
| $CH_2CH_3$ | $OCH_3$ | |
| $CH_3$ | $OCH_2CH_3$ | |
| $CH_3$ | F | |
| $CH_3$ | Cl | |
| $CH_2CH_3$ | Cl | |
| $CH_3$ | Br | |
| $CH_3$ | $SO_2N(CH_3)_2$ | |
| $CH_3$ | $SO_2N(CH_2CH_3)_2$ | |
| $CH_3$ | $SO_2N(OCH_3)CH_3$ | |
| $CH_3$ | $SO_2CH_3$ | |
| $CH_3$ | $SCH_3$ | |
| $CH_3$ | $SOCH_3$ | |
| $CH_3$ | $SCH_2CH_3$ | |
| $CH_3$ | $SO_2CH_2CH_3$ | |
| $CH_3$ | $SO_2CH_2CH(CH_3)_2$ | |
| $CH_3$ | $SO_2CH_2CH_2CH_3$ | |
| $CH_3$ | $SCH_2CH{=}CH_2$ | |
| $CH_3$ | $SO_2CH_2CH{=}CH_2$ | |
| $CH_3$ | $OCH_2CH{=}CH_2$ | |
| $CH_2CH_3$ | $OCH_2CH{=}CH_2$ | |
| $CH_3$ | $OCH(CH_3)CH{=}CH_2$ | |
| $CH_3$ | $OCH_2C{\equiv}CH$ | |
| $CH_3$ | $OCH_2C{\equiv}CCH_3$ | |

Table 4

General Structure 4

| R | $R_5$ | $R_{20}$ | m.p. (°C) |
|---|---|---|---|
| $CH_3$ | $CH_3$ | H | |
| $CH_3$ | $CH_2CH_3$ | H | |
| $CH_3$ | $CH_2CH_2CH_3$ | H | |
| $CH_3$ | $CH(CH_3)_2$ | 5-Cl | |
| $CH_3$ | F | H | |
| $CH_3$ | Cl | H | |
| $CH_3$ | Br | H | |
| $CH_3$ | $NO_2$ | H | |
| $CH_3$ | $CO_2CH_3$ | H | |
| $CH_3$ | $CO_2CH_2CH_3$ | H | |
| $CH_3$ | $CO_2CH(CH_3)_2$ | H | |
| $CH_3$ | $CO_2CH_2CH_2CH_3$ | H | |
| $CH_3$ | $CO_2CH(CH_3)CH_2CH_3$ | H | |
| $CH_3$ | $CO_2CH_2CH_2OCH_3$ | H | |
| $CH_3$ | $CO_2CH_2CH_2Cl$ | H | |
| $CH_3$ | $CO_2CH_2CH=CH_2$ | H | |
| $CH_2CH_3$ | $CO_2CH_3$ | H | |
| $CH_2CH_3$ | $CO_2CH_2CH_3$ | 5-$CH_3$ | |
| $CH_3$ | $SO_2N(CH_3)_2$ | H | |
| $CH_3$ | $SO_2N(CH_2CH_3)_2$ | H | |
| $CH_3$ | $SO_2N(OCH_3)CH_3$ | H | |
| $CH_3$ | $SCH_3$ | H | |
| $CH_3$ | $SOCH_3$ | H | |
| $CH_3$ | $SO_2CH_3$ | H | |
| $CH_3$ | $SCH_2CH_3$ | H | |
| $CH_3$ | $SCH_2CH_2CH_3$ | H | |
| $CH_3$ | $SCH_2CH=CH_2$ | H | |

Table 5

General Structure 5

| R | $R_5$ | $R_{20}$ | m.p. (°C) |
|---|---|---|---|
| $CH_3$ | $CH_3$ | H | |
| $CH_3$ | $CH_2CH_3$ | H | |
| $CH_3$ | $CH_2CH_2CH_3$ | H | |
| $CH_3$ | $CH(CH_3)_2$ | 5-Cl | |
| $CH_3$ | F | H | |
| $CH_3$ | Cl | H | |
| $CH_3$ | Br | H | |
| $CH_3$ | $NO_2$ | H | |
| $CH_3$ | $CO_2CH_3$ | H | |
| $CH_3$ | $CO_2CH_2CH_3$ | H | |
| $CH_3$ | $CO_2CH(CH_3)_2$ | H | |
| $CH_3$ | $CO_2CH_2CH_2CH_3$ | H | |
| $CH_3$ | $CO_2CH(CH_3)CH_2CH_3$ | H | |
| $CH_3$ | $CO_2CH_2CH_2OCH_3$ | H | |
| $CH_3$ | $CO_2CH_2CH_2Cl$ | H | |
| $CH_3$ | $CO_2CH_2CH=CH_2$ | H | |
| $CH_2CH_3$ | $CO_2CH_3$ | H | |
| $CH_2CH_3$ | $CO_2CH_2CH_3$ | 5-$CH_3$ | |
| $CH_3$ | $SO_2N(CH_3)_2$ | H | |
| $CH_3$ | $SO_2N(CH_2CH_3)_2$ | H | |
| $CH_3$ | $SO_2N(OCH_3)CH_3$ | H | |
| $CH_3$ | $SCH_3$ | H | |
| $CH_3$ | $SOCH_3$ | H | |
| $CH_3$ | $SO_2CH_3$ | H | |
| $CH_3$ | $SCH_2CH_3$ | H | |
| $CH_3$ | $SCH_2CH_2CH_3$ | H | |
| $CH_3$ | $SCH_2CH=CH_2$ | H | |

Table 6

General Structure 6

| R | $R_5$ | $R_{20}$ | m.p. (°C) |
|---|---|---|---|
| $CH_3$ | $CH_3$ | H | |
| $CH_3$ | $CH_2CH_3$ | H | |
| $CH_3$ | $CH_2CH_2CH_3$ | H | |
| $CH_3$ | $CH(CH_3)_2$ | 2-Cl | |
| $CH_3$ | F | H | |
| $CH_3$ | Cl | H | |
| $CH_3$ | Br | H | |
| $CH_3$ | $NO_2$ | H | |
| $CH_3$ | $CO_2CH_3$ | H | |
| $CH_3$ | $CO_2CH_2CH_3$ | H | |
| $CH_3$ | $CO_2CH(CH_3)_2$ | H | |
| $CH_3$ | $CO_2CH_2CH_2CH_3$ | H | |
| $CH_3$ | $CO_2CH(CH_3)CH_2CH_3$ | H | |
| $CH_3$ | $CO_2CH_2CH_2OCH_3$ | H | |
| $CH_3$ | $CO_2CH_2CH_2Cl$ | H | |
| $CH_3$ | $CO_2CH_2CH=CH_2$ | H | |
| $CH_2CH_3$ | $CO_2CH_3$ | H | |
| $CH_2CH_3$ | $CO_2CH_2CH_3$ | 2-$CH_3$ | |
| $CH_3$ | $SO_2N(CH_3)_2$ | H | |
| $CH_3$ | $SO_2N(CH_2CH_3)_2$ | H | |
| $CH_3$ | $SO_2N(OCH_3)CH_3$ | H | |
| $CH_3$ | $SCH_3$ | H | |
| $CH_3$ | $SOCH_3$ | H | |
| $CH_3$ | $SO_2CH_3$ | H | |
| $CH_3$ | $SCH_2CH_3$ | H | |
| $CH_3$ | $SCH_2CH_2CH_3$ | H | |
| $CH_3$ | $SCH_2CH=CH_2$ | H | |

Table 7

General Structure 7

| $\underline{R}$ | $\underline{R_6}$ | m.p. (°C) |
|---|---|---|
| $CH_3$ | Cl | |
| $CH_3$ | $NO_2$ | |
| $CH_3$ | $CO_2CH_3$ | |
| $CH_2CH_3$ | $CO_2CH_3$ | |
| $CH_3$ | $CO_2CH_2CH_3$ | |
| $CH_2CH_3$ | $CO_2CH_2CH_3$ | |
| $CH_3$ | $SO_2N(CH_3)_2$ | |
| $CH_3$ | $OSO_2CH_3$ | |
| $CH_3$ | $SO_2CH_3$ | |
| $CH_3$ | $SO_2CH_2CH_3$ | |
| $CH_3$ | $OCH_3$ | |
| $CH_3$ | $OCH_2CH_3$ | |

Table 8

General Structure 8

| R | $R_2$ | $R_7$ | $R_8$ | $R_9$ | m | $Q_1$ | m.p. (°C) |
|---|---|---|---|---|---|---|---|
| $CH_3$ | H | H | H | H | 0 | O | |
| $CH_3$ | H | $CH_3$ | H | H | 0 | O | |
| $CH_3$ | H | $CH_3$ | $CH_3$ | H | 0 | O | |
| $CH_3$ | H | H | H | $CH_3$ | 0 | O | |
| $CH_3$ | H | H | $CH_3$ | H | 1 | O | |
| $CH_3$ | H | $CH_3$ | $CH_3$ | H | 1 | O | |
| $CH_3$ | H | $CH_3$ | H | H | 0 | S | |
| $CH_3$ | H | $CH_3$ | $CH_3$ | H | 0 | S | |
| $CH_3$ | H | H | H | $CH_3$ | 0 | S | |
| $CH_3$ | H | $CH_3$ | H | H | 1 | S | |
| $CH_3$ | H | $CH_3$ | $CH_3$ | H | 1 | S | |
| $CH_3$ | H | H | H | H | 0 | $SO_2$ | |
| $CH_3$ | H | $CH_3$ | H | H | 0 | $SO_2$ | |
| $CH_2CH_3$ | H | $CH_3$ | H | H | 0 | $SO_2$ | |
| $CH_3$ | H | $CH_3$ | $CH_3$ | H | 0 | $SO_2$ | |
| $CH_2CH_3$ | H | $CH_3$ | $CH_3$ | H | 0 | $SO_2$ | |
| $CH_3$ | H | $CH_3$ | H | $CH_3$ | 0 | $SO_2$ | |
| $CH_3$ | H | $CH_3$ | H | H | 1 | $SO_2$ | |
| $CH_3$ | H | $CH_3$ | $CH_3$ | H | 1 | $SO_2$ | |
| $CH_3$ | H | $CH_2CH_3$ | H | H | 0 | $SO_2$ | |
| $CH_3$ | H | $CH_2CH_3$ | H | H | 1 | $SO_2$ | |
| $CH_3$ | 6-Cl | $CH_3$ | H | H | 0 | $SO_2$ | |
| $CH_3$ | 7-Cl | $CH_3$ | H | H | 1 | $SO_2$ | |
| $CH_3$ | 5-Br | $CH_3$ | H | H | 0 | O | |
| $CH_3$ | 6-$CH_3$ | $CH_3$ | H | H | 0 | $SO_2$ | |
| $CH_3$ | 7-Cl | $CH_3$ | H | H | 1 | S | |
| $CH_3$ | 6-Cl | $CH_3$ | $CH_3$ | H | 0 | $SO_2$ | |

Table 9

General Structure 9

| R | $R_2$ | $R_{10}$ | $R_{11}$ | $Q_2$ | m.p. (°C) |
|---|---|---|---|---|---|
| $CH_3$ | H | H | H | O | |
| $CH_3$ | H | $CH_3$ | H | O | |
| $CH_3$ | H | $CH_3$ | $CH_3$ | O | |
| $CH_2CH_3$ | H | H | H | O | |
| $CH_2CH_3$ | H | $CH_3$ | H | O | |
| $CH_2CH_3$ | H | $CH_3$ | $CH_3$ | O | |
| $CH_3$ | H | H | H | S | |
| $CH_3$ | H | $CH_3$ | H | S | |
| $CH_3$ | H | $CH_3$ | $CH_3$ | S | |
| $CH_2CH_3$ | H | H | H | S | |
| $CH_2CH_3$ | H | $CH_3$ | H | S | |
| $CH_2CH_3$ | H | $CH_3$ | $CH_3$ | S | |

Table 10

General Structure 10

| R | $R_2$ | $R_9$ | $R_{12}$ | m | m.p. (°C) |
|---|---|---|---|---|---|
| $CH_3$ | H | H | $CH_3$ | 0 | |
| $CH_3$ | H | $CH_3$ | $CH_3$ | 0 | |
| $CH_3$ | H | H | $CH_3$ | 1 | |
| $CH_2CH_3$ | H | H | $CH_3$ | 0 | |
| $CH_2CH_3$ | H | $CH_3$ | $CH_3$ | 0 | |
| $CH_2CH_3$ | H | H | $CH_3$ | 1 | |
| $CH_3$ | H | H | $CH_2CH_3$ | 0 | |
| $CH_3$ | H | H | $CH_2CH_2CH_3$ | 0 | |
| $CH_3$ | H | H | $CH(CH_3)_2$ | 0 | |
| $CH_3$ | H | H | $CH_2CH_2CH_2CH_3$ | 0 | |
| $CH_3$ | H | H | $CH(CH_3)CH_2CH_3$ | 0 | |
| $CH_3$ | H | H | $CH_2CH(CH_3)_2$ | 0 | |
| $CH_3$ | H | H | $CH_2CH_3$ | 1 | |
| $CH_3$ | H | H | $CH(CH_3)_2$ | 1 | |

Table 10 (continued)

| R | $R_2$ | $R_9$ | $R_{12}$ | m | m.p. (°C) |
|---|---|---|---|---|---|
| $CH_3$ | H | H | $CH_2CH_2CH_3$ | 1 | |
| $CH_3$ | 6-Cl | H | $CH_3$ | 0 | |
| $CH_3$ | H | H | $CH_2CH_2F$ | 0 | |
| $CH_3$ | H | H | $CH_2CH_2OCH_3$ | 0 | |
| $CH_3$ | H | H | $CH_2CH=CH_2$ | 0 | |
| $CH_3$ | H | H | $CH_2C\equiv CH$ | 0 | |
| $CH_3$ | H | H | $CH_2C_6H_5$ | 0 | |
| $CH_3$ | H | H | $C(O)CH_3$ | 0 | |
| $CH_3$ | H | H | $C(O)CH_2CH_2CH_3$ | 1 | |
| $CH_3$ | H | H | $CO_2CH_3$ | 0 | |
| $CH_3$ | H | H | $CH_2CN$ | 0 | |
| $CH_3$ | H | H | $CH_2C(O)CH_3$ | 0 | |
| $CH_3$ | H | H | $CH_2CO_2CH_3$ | 0 | |
| $CH_3$ | H | H | $CH_2CF=CF_2$ | 1 | |
| $CH_3$ | H | H | $CH_2CH=CCl_2$ | 0 | |
| $CH_3$ | H | H | $CH_2CH_2F$ | 0 | |
| $CH_3$ | H | H | $CH_2OCH_3$ | 0 | |

Table 11

General Structure 11

| R | $R_2$ | $R_9$ | m | m.p. (°C) |
|---|---|---|---|---|
| $CH_3$ | H | H | 0 | |
| $CH_3$ | H | $CH_3$ | 0 | |
| $CH_3$ | H | H | 1 | 183-186 |
| $CH_2CH_3$ | H | H | 0 | |
| $CH_2CH_3$ | H | $CH_3$ | 0 | |
| $CH_2CH_3$ | H | H | 1 | |
| $CH_3$ | 6-Cl | H | 0 | |
| $CH_3$ | 7-Cl | H | 1 | |
| $CH_3$ | 6-Cl | H | 1 | |
| $CH_3$ | 6-$CH_3$ | H | 1 | |

Table 12

General Structure 12

| R | $R_2$ | $R_9$ | m | m.p. (°C) |
|---|---|---|---|---|
| $CH_3$ | H | H | 0 | |
| $CH_3$ | H | $CH_3$ | 0 | |
| $CH_3$ | H | H | 1 | |
| $CH_2CH_3$ | H | H | 0 | |
| $CH_2CH_3$ | H | $CH_3$ | 0 | |
| $CH_2CH_3$ | H | H | 1 | |

Table 13

General Structure 13

| R | $R_2$ | $R_9$ | m | m.p. (°C) |
|---|---|---|---|---|
| $CH_3$ | H | H | 0 | |
| $CH_3$ | H | $CH_3$ | 0 | |
| $CH_3$ | H | H | 1 | |
| $CH_2CH_3$ | H | H | 0 | |
| $CH_2CH_3$ | H | $CH_3$ | 0 | |
| $CH_2CH_3$ | H | H | 1 | |

Table 14

General Structure 14

| R | $R_2$ | $R_{12}$ | m.p. (°C) |
|---|---|---|---|
| $CH_3$ | H | $CH_3$ | |
| $CH_2CH_3$ | H | $CH_3$ | |
| $CH_3$ | H | $CH_2CH_3$ | |
| $CH_2CH_3$ | H | $CH_2CH_3$ | |
| $CH_3$ | H | $CH_2CH_2CH_3$ | |
| $CH_3$ | H | $CH(CH_3)_2$ | |
| $CH_3$ | H | $CH_2CH_2CH_2CH_3$ | |
| $CH_3$ | H | $CH(CH_3)CH_2CH_3$ | |
| $CH_3$ | H | $CH_2CH(CH_3)_2$ | |

Table 15

General Structure 15

| R | $R_2$ | $R_{13}$ | m.p. (°C) |
|---|---|---|---|
| $CH_3$ | H | H | |
| $CH_3$ | H | $CH_3$ | |
| $CH_2CH_3$ | H | H | |
| $CH_2CH_3$ | H | $CH_3$ | |

Table 16

General Structure 16

| R | $R_2$ | $R_{13}$ | m.p. (°C) |
|---|---|---|---|
| $CH_3$ | H | H | |
| $CH_3$ | H | $CH_3$ | |
| $CH_2CH_3$ | H | H | |
| $CH_2CH_3$ | H | $CH_3$ | |

Table 17

General Structure 17

| R | $R_2$ | $R_{13}$ | $R_{14}$ | m.p. (°C) |
|---|---|---|---|---|
| $CH_3$ | H | H | $CH_3$ | |
| $CH_3$ | H | H | $CH_2CH_3$ | |
| $CH_3$ | H | $CH_3$ | $CH_3$ | |
| $CH_3$ | H | $CH_3$ | $CH_2CH_3$ | |
| $CH_2CH_3$ | H | H | $CH_3$ | |
| $CH_2CH_3$ | H | H | $CH_2CH_3$ | |
| $CH_2CH_3$ | H | $CH_3$ | $CH_3$ | |
| $CH_2CH_3$ | H | $CH_3$ | $CH_2CH_3$ | |
| $CH_3$ | 6-Cl | H | $CH_3$ | |
| $CH_3$ | 6-$CH_3$ | H | $CH_3$ | |
| $CH_3$ | 6-Cl | $CH_3$ | $CH_3$ | |
| $CH_3$ | 7-Cl | H | $CH_3$ | |

Table 18

General Structure 18

| q | $Q_3$ | $R_{21}$ | $R_{22}$ | $R_{23}$ | m.p.(°C) |
|---|---|---|---|---|---|
| 1 | O | H | H | H | 197-201 |
| 1 | O | H | $CH_3$ | H | |
| 1 | O | H | $CH_2CH_3$ | H | |
| 1 | O | H | $CH_2CH_2CH_3$ | H | |
| 1 | O | H | $CH_2CH_2CH_2CH_3$ | H | |
| 1 | O | H | $CH(CH_3)_2$ | H | |
| 1 | O | H | $CH(CH_3)CH_2CH_3$ | H | |
| 1 | O | H | $CH_2CH(CH_3)_2$ | H | |
| 1 | O | o-Cl | H | H | |
| 1 | O | o-Cl | $CH_3$ | H | |
| 1 | O | o-Cl | $CH_2CH_3$ | H | |
| 1 | O | o-Cl | $CH_2CH_2CH_3$ | H | |
| 1 | O | o-Cl | $CH_2CH_2CH_2CH_3$ | H | |
| 1 | O | o-Cl | $CH(CH_3)_2$ | H | |
| 1 | O | o-Cl | $CH(CH_3)CH_2CH_3$ | H | |
| 1 | O | o-Cl | $CH_2CH(CH_3)_2$ | H | |
| 1 | O | m-Cl | H | H | |
| 1 | O | m-Cl | $CH_3$ | H | 208-211 |
| 1 | O | m-Cl | $CH_2CH_3$ | H | |
| 1 | O | m-Cl | $CH_2CH_2CH_3$ | H | |
| 1 | O | m-Cl | $CH_2CH_2CH_2CH_3$ | H | |
| 1 | O | m-Cl | $CH(CH_3)_2$ | H | |
| 1 | O | m-Cl | $CH(CH_3)CH_2CH_3$ | H | |
| 1 | O | m-Cl | $CH_2CH(CH_3)_2$ | H | |

Table 18 (continued)

| q | $Q_3$ | $R_{21}$ | $R_{22}$ | $R_{23}$ | m.p.(°C) |
|---|---|---|---|---|---|
| 1 | O | o-$CH_3$ | H | H | |
| 1 | O | o-$CH_3$ | $CH_3$ | H | |
| 1 | O | o-$CH_3$ | $CH_2CH_3$ | H | |
| 1 | O | o-$CH_3$ | $CH_2CH_2CH_3$ | H | |
| 1 | O | o-$CH_3$ | $CH_2CH_2CH_2CH_3$ | H | |
| 1 | O | o-$CH_3$ | $CH(CH_3)_2$ | H | |
| 1 | O | o-$CH_3$ | $CH(CH_3)CH_2CH_3$ | H | |
| 1 | O | o-$CH_3$ | $CH_2CH(CH_3)_2$ | H | |
| 1 | O | m-$CH_3$ | H | H | |
| 1 | O | m-$CH_3$ | $CH_3$ | H | |
| 1 | O | m-$CH_3$ | $CH_2CH_3$ | H | |
| 1 | O | m-$CH_3$ | $CH_2CH_2CH_3$ | H | |
| 1 | O | m-$CH_3$ | $CH_2CH_2CH_2CH_3$ | H | |
| 1 | O | m-$CH_3$ | $CH(CH_3)_2$ | H | |
| 1 | O | m-$CH_3$ | $CH(CH_3)CH_2CH_3$ | H | |
| 1 | O | m-$CH_3$ | $CH_2CH(CH_3)_2$ | H | |
| 1 | O | o-$OCH_3$ | H | H | |
| 1 | O | o-$OCH_3$ | $CH_3$ | H | |
| 1 | O | o-$OCH_3$ | $CH_2CH_3$ | H | |
| 1 | O | o-$OCH_3$ | $CH_2CH_2CH_3$ | H | |
| 1 | O | o-$OCH_3$ | $CH_2CH_2CH_2CH_3$ | H | |
| 1 | O | o-$OCH_3$ | $CH(CH_3)_2$ | H | |
| 1 | O | o-$OCH_3$ | $CH(CH_3)CH_2CH_3$ | H | |
| 1 | O | o-$OCH_3$ | $CH_2CH(CH_3)_2$ | H | |
| 1 | O | m-$OCH_3$ | H | H | |
| 1 | O | m-$OCH_3$ | $CH_3$ | H | |
| 1 | O | m-$OCH_3$ | $CH_2CH_3$ | H | |
| 1 | O | m-$OCH_3$ | $CH_2CH_2CH_3$ | H | |
| 1 | O | m-$OCH_3$ | $CH_2CH_2CH_2CH_3$ | H | |
| 1 | O | m-$OCH_3$ | $CH(CH_3)_2$ | H | |
| 1 | O | m-$OCH_3$ | $CH(CH_3)CH_2CH_3$ | H | |
| 1 | O | m-$OCH_3$ | $CH_2CH(CH_3)_2$ | H | |

Table 18 (continued)

| q | $Q_3$ | $R_{21}$ | $R_{22}$ | $R_{23}$ | m.p.(°C) |
|---|---|---|---|---|---|
| 1 | S | H | H | H | |
| 1 | S | H | $CH_3$ | H | |
| 1 | S | H | $CH_2CH_3$ | H | |
| 1 | S | H | $CH_2CH_2CH_3$ | H | |
| 1 | S | H | $CH_2CH_2CH_2CH_3$ | H | |
| 1 | S | H | $CH(CH_3)_2$ | H | |
| 1 | S | H | $CH(CH_3)CH_2CH_3$ | H | |
| 1 | S | H | $CH_2CH(CH_3)_2$ | H | |
| 1 | S | o-Cl | H | H | |
| 1 | S | o-Cl | $CH_3$ | H | |
| 1 | S | o-Cl | $CH_2CH_3$ | H | |
| 1 | S | o-Cl | $CH_2CH_2CH_3$ | H | |
| 1 | S | o-Cl | $CH_2CH_2CH_2CH_3$ | H | |
| 1 | S | o-Cl | $CH(CH_3)_2$ | H | |
| 1 | S | o-Cl | $CH(CH_3)CH_2CH_3$ | H | |
| 1 | S | o-Cl | $CH_2CH(CH_3)_2$ | H | |
| 1 | S | m-Cl | H | H | |
| 1 | S | m-Cl | $CH_3$ | H | |
| 1 | S | m-Cl | $CH_2CH_3$ | H | |
| 1 | S | m-Cl | $CH_2CH_2CH_3$ | H | |
| 1 | S | m-Cl | $CH_2CH_2CH_2CH_3$ | H | |
| 1 | S | m-Cl | $CH(CH_3)_2$ | H | |
| 1 | S | m-Cl | $CH(CH_3)CH_2CH_3$ | H | |
| 1 | S | m-Cl | $CH_2CH(CH_3)_2$ | H | |
| 1 | S | o-$CH_3$ | H | H | |
| 1 | S | o-$CH_3$ | $CH_3$ | H | |
| 1 | S | o-$CH_3$ | $CH_2CH_3$ | H | |
| 1 | S | o-$CH_3$ | $CH_2CH_2CH_3$ | H | |
| 1 | S | o-$CH_3$ | $CH_2CH_2CH_2CH_3$ | H | |
| 1 | S | o-$CH_3$ | $CH(CH_3)_2$ | H | |
| 1 | S | o-$CH_3$ | $CH(CH_3)CH_2CH_3$ | H | |
| 1 | S | o-$CH_3$ | $CH_2CH(CH_3)_2$ | H | |

Table 18 (continued)

| q | $Q_3$ | $R_{21}$ | $R_{22}$ | $R_{23}$ | m.p.(°C) |
|---|---|---|---|---|---|
| 1 | S | m-$CH_3$ | H | H | |
| 1 | S | m-$CH_3$ | $CH_3$ | H | |
| 1 | S | m-$CH_3$ | $CH_2CH_3$ | H | |
| 1 | S | m-$CH_3$ | $CH_2CH_2CH_3$ | H | |
| 1 | S | m-$CH_3$ | $CH_2CH_2CH_2CH_3$ | H | |
| 1 | S | m-$CH_3$ | $CH(CH_3)_2$ | H | |
| 1 | S | m-$CH_3$ | $CH(CH_3)CH_2CH_3$ | H | |
| 1 | S | m-$CH_3$ | $CH_2CH(CH_3)_2$ | H | |
| 1 | S | o-$OCH_3$ | H | H | |
| 1 | S | o-$OCH_3$ | $CH_3$ | H | |
| 1 | S | o-$OCH_3$ | $CH_2CH_3$ | H | |
| 1 | S | o-$OCH_3$ | $CH_2CH_2CH_3$ | H | |
| 1 | S | o-$OCH_3$ | $CH_2CH_2CH_2CH_3$ | H | |
| 1 | S | o-$OCH_3$ | $CH(CH_3)_2$ | H | |
| 1 | S | o-$OCH_3$ | $CH(CH_3)CH_2CH_3$ | H | |
| 1 | S | o-$OCH_3$ | $CH_2CH(CH_3)_2$ | H | |
| 1 | S | m-$OCH_3$ | H | H | |
| 1 | S | m-$OCH_3$ | $CH_3$ | H | |
| 1 | S | m-$OCH_3$ | $CH_2CH_3$ | H | |
| 1 | S | m-$OCH_3$ | $CH_2CH_2CH_3$ | H | |
| 1 | S | m-$OCH_3$ | $CH_2CH_2CH_2CH_3$ | H | |
| 1 | S | m-$OCH_3$ | $CH(CH_3)_2$ | H | |
| 1 | S | m-$OCH_3$ | $CH(CH_3)CH_2CH_3$ | H | |
| 1 | S | m-$OCH_3$ | $CH_2CH(CH_3)_2$ | H | |
| 1 | SO | H | H | H | |
| 1 | SO | H | $CH_3$ | H | |
| 1 | SO | H | $CH_2CH_3$ | H | |
| 1 | SO | H | $CH_2CH_2CH_3$ | H | |
| 1 | SO | H | $CH_2CH_2CH_2CH_3$ | H | |
| 1 | SO | H | $CH(CH_3)_2$ | H | |
| 1 | SO | H | $CH(CH_3)CH_2CH_3$ | H | |
| 1 | SO | H | $CH_2CH(CH_3)_2$ | H | |

Table 18 (continued)

| q | $Q_3$ | $R_{21}$ | $R_{22}$ | $R_{23}$ | m.p.(°C) |
|---|---|---|---|---|---|
| 1 | SO | o-Cl | H | H | |
| 1 | SO | o-Cl | $CH_3$ | H | |
| 1 | SO | o-Cl | $CH_2CH_3$ | H | |
| 1 | SO | o-Cl | $CH_2CH_2CH_3$ | H | |
| 1 | SO | o-Cl | $CH_2CH_2CH_2CH_3$ | H | |
| 1 | SO | o-Cl | $CH(CH_3)_2$ | H | |
| 1 | SO | o-Cl | $CH(CH_3)CH_2CH_3$ | H | |
| 1 | SO | o-Cl | $CH_2CH(CH_3)_2$ | H | |
| 1 | SO | m-Cl | H | H | |
| 1 | SO | m-Cl | $CH_3$ | H | |
| 1 | SO | m-Cl | $CH_2CH_3$ | H | |
| 1 | SO | m-Cl | $CH_2CH_2CH_3$ | H | |
| 1 | SO | m-Cl | $CH_2CH_2CH_2CH_3$ | H | |
| 1 | SO | m-Cl | $CH(CH_3)_2$ | H | |
| 1 | SO | m-Cl | $CH(CH_3)CH_2CH_3$ | H | |
| 1 | SO | m-Cl | $CH_2CH(CH_3)_2$ | H | |
| 1 | SO | o-$CH_3$ | H | H | |
| 1 | SO | o-$CH_3$ | $CH_3$ | H | |
| 1 | SO | o-$CH_3$ | $CH_2CH_3$ | H | |
| 1 | SO | o-$CH_3$ | $CH_2CH_2CH_3$ | H | |
| 1 | SO | o-$CH_3$ | $CH_2CH_2CH_2CH_3$ | H | |
| 1 | SO | o-$CH_3$ | $CH(CH_3)_2$ | H | |
| 1 | SO | o-$CH_3$ | $CH(CH_3)CH_2CH_3$ | H | |
| 1 | SO | o-$CH_3$ | $CH_2CH(CH_3)_2$ | H | |
| 1 | SO | m-$CH_3$ | H | H | |
| 1 | SO | m-$CH_3$ | $CH_3$ | H | |
| 1 | SO | m-$CH_3$ | $CH_2CH_3$ | H | |
| 1 | SO | m-$CH_3$ | $CH_2CH_2CH_3$ | H | |
| 1 | SO | m-$CH_3$ | $CH_2CH_2CH_2CH_3$ | H | |
| 1 | SO | m-$CH_3$ | $CH(CH_3)_2$ | H | |
| 1 | SO | m-$CH_3$ | $CH(CH_3)CH_2CH_3$ | H | |
| 1 | SO | m-$CH_3$ | $CH_2CH(CH_3)_2$ | H | |

Table 18 (continued)

| q | $Q_3$ | $R_{21}$ | $R_{22}$ | $R_{23}$ | m.p.(°C) |
|---|---|---|---|---|---|
| 1 | SO | o-$OCH_3$ | H | H | |
| 1 | SO | o-$OCH_3$ | $CH_3$ | H | |
| 1 | SO | o-$OCH_3$ | $CH_2CH_3$ | H | |
| 1 | SO | o-$OCH_3$ | $CH_2CH_2CH_3$ | H | |
| 1 | SO | o-$OCH_3$ | $CH_2CH_2CH_2CH_3$ | H | |
| 1 | SO | o-$OCH_3$ | $CH(CH_3)_2$ | H | |
| 1 | SO | o-$OCH_3$ | $CH(CH_3)CH_2CH_3$ | H | |
| 1 | SO | o-$OCH_3$ | $CH_2CH(CH_3)_2$ | H | |
| 1 | SO | m-$OCH_3$ | H | H | |
| 1 | SO | m-$OCH_3$ | $CH_3$ | H | |
| 1 | SO | m-$OCH_3$ | $CH_2CH_3$ | H | |
| 1 | SO | m-$OCH_3$ | $CH_2CH_2CH_3$ | H | |
| 1 | SO | m-$OCH_3$ | $CH_2CH_2CH_2CH_3$ | H | |
| 1 | SO | m-$OCH_3$ | $CH(CH_3)_2$ | H | |
| 1 | SO | m-$OCH_3$ | $CH(CH_3)CH_2CH_3$ | H | |
| 1 | SO | m-$OCH_3$ | $CH_2CH(CH_3)_2$ | H | |
| 1 | $SO_2$ | H | H | H | 238-240 |
| 1 | $SO_2$ | H | $CH_3$ | H | 225-227 |
| 1 | $SO_2$ | H | $CH_3$ | $CH_3$ | 235-237 |
| 1 | $SO_2$ | H | $CH_2CH_3$ | H | |
| 1 | $SO_2$ | H | $CH_2CH_2CH_3$ | H | |
| 1 | $SO_2$ | H | $CH_2CH_2CH_2CH_3$ | H | |
| 1 | $SO_2$ | H | $CH(CH_3)_2$ | H | |
| 1 | $SO_2$ | H | $CH(CH_3)CH_2CH_3$ | H | |
| 1 | $SO_2$ | H | $CH_2CH(CH_3)_2$ | H | |
| 1 | $SO_2$ | o-Cl | H | H | |
| 1 | $SO_2$ | o-Cl | $CH_3$ | H | |
| 1 | $SO_2$ | o-Cl | $CH_3$ | $CH_3$ | |
| 1 | $SO_2$ | o-Cl | $CH_2CH_3$ | H | |
| 1 | $SO_2$ | o-Cl | $CH_2CH_2CH_3$ | H | |
| 1 | $SO_2$ | o-Cl | $CH_2CH_2CH_2CH_3$ | H | |
| 1 | $SO_2$ | o-Cl | $CH(CH_3)_2$ | H | |

Table 18 (continued)

| q | $Q_3$ | $R_{21}$ | $R_{22}$ | $R_{23}$ | m.p.(°C) |
|---|---|---|---|---|---|
| 1 | $SO_2$ | o-Cl | $CH(CH_3)CH_2CH_3$ | H | |
| 1 | $SO_2$ | o-Cl | $CH_2CH(CH_3)_2$ | H | |
| 1 | $SO_2$ | m-Cl | H | H | |
| 1 | $SO_2$ | m-Cl | $CH_3$ | H | |
| 1 | $SO_2$ | m-Cl | $CH_3$ | $CH_3$ | |
| 1 | $SO_2$ | m-Cl | $CH_2CH_3$ | H | |
| 1 | $SO_2$ | m-Cl | $CH_2CH_2CH_3$ | H | |
| 1 | $SO_2$ | m-Cl | $CH_2CH_2CH_2CH_3$ | H | |
| 1 | $SO_2$ | m-Cl | $CH(CH_3)_2$ | H | |
| 1 | $SO_2$ | m-Cl | $CH(CH_3)CH_2CH_3$ | H | |
| 1 | $SO_2$ | m-Cl | $CH_2CH(CH_3)_2$ | H | |
| 1 | $SO_2$ | o-$CH_3$ | H | H | |
| 1 | $SO_2$ | o-$CH_3$ | $CH_3$ | H | |
| 1 | $SO_2$ | o-$CH_3$ | $CH_3$ | $CH_3$ | |
| 1 | $SO_2$ | o-$CH_3$ | $CH_2CH_3$ | H | |
| 1 | $SO_2$ | o-$CH_3$ | $CH_2CH_2CH_3$ | H | |
| 1 | $SO_2$ | o-$CH_3$ | $CH_2CH_2CH_2CH_3$ | H | |
| 1 | $SO_2$ | o-$CH_3$ | $CH(CH_3)_2$ | H | |
| 1 | $SO_2$ | o-$CH_3$ | $CH(CH_3)CH_2CH_3$ | H | |
| 1 | $SO_2$ | o-$CH_3$ | $CH_2CH(CH_3)_2$ | H | |
| 1 | $SO_2$ | m-$CH_3$ | H | H | |
| 1 | $SO_2$ | m-$CH_3$ | $CH_3$ | H | |
| 1 | $SO_2$ | m-$CH_3$ | $CH_3$ | $CH_3$ | |
| 1 | $SO_2$ | m-$CH_3$ | $CH_2CH_3$ | H | |
| 1 | $SO_2$ | m-$CH_3$ | $CH_2CH_2CH_3$ | H | |
| 1 | $SO_2$ | m-$CH_3$ | $CH_2CH_2CH_2CH_3$ | H | |
| 1 | $SO_2$ | m-$CH_3$ | $CH(CH_3)_2$ | H | |
| 1 | $SO_2$ | m-$CH_3$ | $CH(CH_3)CH_2CH_3$ | H | |
| 1 | $SO_2$ | m-$CH_3$ | $CH_2CH(CH_3)_2$ | H | |
| 1 | $SO_2$ | o-$OCH_3$ | H | H | |
| 1 | $SO_2$ | o-$OCH_3$ | $CH_3$ | H | |
| 1 | $SO_2$ | o-$OCH_3$ | $CH_3$ | $CH_3$ | |

Table 18 (continued)

| q | $Q_3$ | $R_{21}$ | $R_{22}$ | $R_{23}$ | m.p.(°C) |
|---|---|---|---|---|---|
| 1 | $SO_2$ | o-$OCH_3$ | $CH_2CH_3$ | H | |
| 1 | $SO_2$ | o-$OCH_3$ | $CH_2CH_2CH_3$ | H | |
| 1 | $SO_2$ | o-$OCH_3$ | $CH_2CH_2CH_2CH_3$ | H | |
| 1 | $SO_2$ | o-$OCH_3$ | $CH(CH_3)_2$ | H | |
| 1 | $SO_2$ | o-$OCH_3$ | $CH(CH_3)CH_2CH_3$ | H | |
| 1 | $SO_2$ | o-$OCH_3$ | $CH_2CH(CH_3)_2$ | H | |
| 1 | $SO_2$ | m-$OCH_3$ | H | H | |
| 1 | $SO_2$ | m-$OCH_3$ | $CH_3$ | H | |
| 1 | $SO_2$ | m-$OCH_3$ | $CH_3$ | $CH_3$ | |
| 1 | $SO_2$ | m-$OCH_3$ | $CH_2CH_3$ | H | |
| 1 | $SO_2$ | m-$OCH_3$ | $CH_2CH_2CH_3$ | H | |
| 1 | $SO_2$ | m-$OCH_3$ | $CH_2CH_2CH_2CH_3$ | H | |
| 1 | $SO_2$ | m-$OCH_3$ | $CH(CH_3)_2$ | H | |
| 1 | $SO_2$ | m-$OCH_3$ | $CH(CH_3)CH_2CH_3$ | H | |
| 1 | $SO_2$ | m-$OCH_3$ | $CH_2CH(CH_3)_2$ | H | |
| 2 | O | H | H | H | |
| 2 | O | H | $CH_3$ | H | |
| 2 | O | H | $CH_2CH_3$ | H | |
| 2 | O | H | $CH_2CH_2CH_3$ | H | |
| 2 | O | H | $CH_2CH_2CH_2CH_3$ | H | |
| 2 | O | H | $CH(CH_3)_2$ | H | |
| 2 | O | H | $CH(CH_3)CH_2CH_3$ | H | |
| 2 | O | H | $CH_2CH(CH_3)_2$ | H | |
| 2 | O | o-Cl | H | H | |
| 2 | O | o-Cl | $CH_3$ | H | |
| 2 | O | o-Cl | $CH_2CH_3$ | H | |
| 2 | O | o-Cl | $CH_2CH_2CH_3$ | H | |
| 2 | O | o-Cl | $CH_2CH_2CH_2CH_3$ | H | |
| 2 | O | o-Cl | $CH(CH_3)_2$ | H | |
| 2 | O | o-Cl | $CH(CH_3)CH_2CH_3$ | H | |
| 2 | O | o-Cl | $CH_2CH(CH_3)_2$ | H | |
| 2 | O | m-Cl | H | H | |

Table 18 (continued)

| q | $Q_3$ | $R_{21}$ | $R_{22}$ | $R_{23}$ | m.p.(°C) |
|---|---|---|---|---|---|
| 2 | O | m-Cl | $CH_3$ | H | |
| 2 | O | m-Cl | $CH_2CH_3$ | H | |
| 2 | O | m-Cl | $CH_2CH_2CH_3$ | H | |
| 2 | O | m-Cl | $CH_2CH_2CH_2CH_3$ | H | |
| 2 | O | m-Cl | $CH(CH_3)_2$ | H | |
| 2 | O | m-Cl | $CH(CH_3)CH_2CH_3$ | H | |
| 2 | O | m-Cl | $CH_2CH(CH_3)_2$ | H | |
| 2 | O | o-$CH_3$ | H | H | |
| 2 | O | o-$CH_3$ | $CH_3$ | H | |
| 2 | O | o-$CH_3$ | $CH_2CH_3$ | H | |
| 2 | O | o-$CH_3$ | $CH_2CH_2CH_3$ | H | |
| 2 | O | o-$CH_3$ | $CH_2CH_2CH_2CH_3$ | H | |
| 2 | O | o-$CH_3$ | $CH(CH_3)_2$ | H | |
| 2 | O | o-$CH_3$ | $CH(CH_3)CH_2CH_3$ | H | |
| 2 | O | o-$CH_3$ | $CH_2CH(CH_3)_2$ | H | |
| 2 | O | m-$CH_3$ | H | H | |
| 2 | O | m-$CH_3$ | $CH_3$ | H | |
| 2 | O | m-$CH_3$ | $CH_2CH_3$ | H | |
| 2 | O | m-$CH_3$ | $CH_2CH_2CH_3$ | H | |
| 2 | O | m-$CH_3$ | $CH_2CH_2CH_2CH_3$ | H | |
| 2 | O | m-$CH_3$ | $CH(CH_3)_2$ | H | |
| 2 | O | m-$CH_3$ | $CH(CH_3)CH_2CH_3$ | H | |
| 2 | O | m-$CH_3$ | $CH_2CH(CH_3)_2$ | H | |
| 2 | O | o-$OCH_3$ | H | H | |
| 2 | O | o-$OCH_3$ | $CH_3$ | H | |
| 2 | O | o-$OCH_3$ | $CH_2CH_3$ | H | |
| 2 | O | o-$OCH_3$ | $CH_2CH_2CH_3$ | H | |
| 2 | O | o-$OCH_3$ | $CH_2CH_2CH_2CH_3$ | H | |
| 2 | O | o-$OCH_3$ | $CH(CH_3)_2$ | H | |
| 2 | O | o-$OCH_3$ | $CH(CH_3)CH_2CH_3$ | H | |
| 2 | O | o-$OCH_3$ | $CH_2CH(CH_3)_2$ | H | |
| 2 | O | m-$OCH_3$ | H | H | |

Table 18 (continued)

| q | $Q_3$ | $R_{21}$ | $R_{22}$ | $R_{23}$ | m.p.(°C) |
|---|---|---|---|---|---|
| 2 | O | m-$OCH_3$ | $CH_3$ | H | |
| 2 | O | m-$OCH_3$ | $CH_2CH_3$ | H | |
| 2 | O | m-$OCH_3$ | $CH_2CH_2CH_3$ | H | |
| 2 | O | m-$OCH_3$ | $CH_2CH_2CH_2CH_3$ | H | |
| 2 | O | m-$OCH_3$ | $CH(CH_3)_2$ | H | |
| 2 | O | m-$OCH_3$ | $CH(CH_3)CH_2CH_3$ | H | |
| 2 | O | m-$OCH_3$ | $CH_2CH(CH_3)_2$ | H | |
| 2 | S | H | H | H | |
| 2 | S | H | $CH_3$ | H | |
| 2 | S | H | $CH_2CH_3$ | H | |
| 2 | S | H | $CH_2CH_2CH_3$ | H | |
| 2 | S | H | $CH_2CH_2CH_2CH_3$ | H | |
| 2 | S | H | $CH(CH_3)_2$ | H | |
| 2 | S | H | $CH(CH_3)CH_2CH_3$ | H | |
| 2 | S | H | $CH_2CH(CH_3)_2$ | H | |
| 2 | S | o-Cl | H | H | |
| 2 | S | o-Cl | $CH_3$ | H | |
| 2 | S | o-Cl | $CH_2CH_3$ | H | |
| 2 | S | o-Cl | $CH_2CH_2CH_3$ | H | |
| 2 | S | o-Cl | $CH_2CH_2CH_2CH_3$ | H | |
| 2 | S | o-Cl | $CH(CH_3)_2$ | H | |
| 2 | S | o-Cl | $CH(CH_3)CH_2CH_3$ | H | |
| 2 | S | o-Cl | $CH_2CH(CH_3)_2$ | H | |
| 2 | S | m-Cl | H | H | |
| 2 | S | m-Cl | $CH_3$ | H | |
| 2 | S | m-Cl | $CH_2CH_3$ | H | |
| 2 | S | m-Cl | $CH_2CH_2CH_3$ | H | |
| 2 | S | m-Cl | $CH_2CH_2CH_2CH_3$ | H | |
| 2 | S | m-Cl | $CH(CH_3)_2$ | H | |
| 2 | S | m-Cl | $CH(CH_3)CH_2CH_3$ | H | |
| 2 | S | m-Cl | $CH_2CH(CH_3)_2$ | H | |
| 2 | S | o-$CH_3$ | H | H | |

Table 18 (continued)

| q | $Q_3$ | $R_{21}$ | $R_{22}$ | $R_{23}$ | m.p.(°C) |
|---|---|---|---|---|---|
| 2 | S | o-$CH_3$ | $CH_3$ | H | |
| 2 | S | o-$CH_3$ | $CH_2CH_3$ | H | |
| 2 | S | o-$CH_3$ | $CH_2CH_2CH_3$ | H | |
| 2 | S | o-$CH_3$ | $CH_2CH_2CH_2CH_3$ | H | |
| 2 | S | o-$CH_3$ | $CH(CH_3)_2$ | H | |
| 2 | S | o-$CH_3$ | $CH(CH_3)CH_2CH_3$ | H | |
| 2 | S | o-$CH_3$ | $CH_2CH(CH_3)_2$ | H | |
| 2 | S | m-$CH_3$ | H | H | |
| 2 | S | m-$CH_3$ | $CH_3$ | H | |
| 2 | S | m-$CH_3$ | $CH_2CH_3$ | H | |
| 2 | S | m-$CH_3$ | $CH_2CH_2CH_3$ | H | |
| 2 | S | m-$CH_3$ | $CH_2CH_2CH_2CH_3$ | H | |
| 2 | S | m-$CH_3$ | $CH(CH_3)_2$ | H | |
| 2 | S | m-$CH_3$ | $CH(CH_3)CH_2CH_3$ | H | |
| 2 | S | m-$CH_3$ | $CH_2CH(CH_3)_2$ | H | |
| 2 | S | o-$OCH_3$ | H | H | |
| 2 | S | o-$OCH_3$ | $CH_3$ | H | |
| 2 | S | o-$OCH_3$ | $CH_2CH_3$ | H | |
| 2 | S | o-$OCH_3$ | $CH_2CH_2CH_3$ | H | |
| 2 | S | o-$OCH_3$ | $CH_2CH_2CH_2CH_3$ | H | |
| 2 | S | o-$OCH_3$ | $CH(CH_3)_2$ | H | |
| 2 | S | o-$OCH_3$ | $CH(CH_3)CH_2CH_3$ | H | |
| 2 | S | o-$OCH_3$ | $CH_2CH(CH_3)_2$ | H | |
| 2 | S | m-$OCH_3$ | H | H | |
| 2 | S | m-$OCH_3$ | $CH_3$ | H | |
| 2 | S | m-$OCH_3$ | $CH_2CH_3$ | H | |
| 2 | S | m-$OCH_3$ | $CH_2CH_2CH_3$ | H | |
| 2 | S | m-$OCH_3$ | $CH_2CH_2CH_2CH_3$ | H | |
| 2 | S | m-$OCH_3$ | $CH(CH_3)_2$ | H | |
| 2 | S | m-$OCH_3$ | $CH(CH_3)CH_2CH_3$ | H | |
| 2 | S | m-$OCH_3$ | $CH_2CH(CH_3)_2$ | H | |
| 2 | SO | H | H | H | |

Table 18 (continued)

| q | $Q_3$ | $R_{21}$ | $R_{22}$ | $R_{23}$ | m.p.(°C) |
|---|---|---|---|---|---|
| 2 | SO | H | $CH_3$ | H | |
| 2 | SO | H | $CH_2CH_3$ | H | |
| 2 | SO | H | $CH_2CH_2CH_3$ | H | |
| 2 | SO | H | $CH_2CH_2CH_2CH_3$ | H | |
| 2 | SO | H | $CH(CH_3)_2$ | H | |
| 2 | SO | H | $CH(CH_3)CH_2CH_3$ | H | |
| 2 | SO | H | $CH_2CH(CH_3)_2$ | H | |
| 2 | SO | o-Cl | H | H | |
| 2 | SO | o-Cl | $CH_3$ | H | |
| 2 | SO | o-Cl | $CH_2CH_3$ | H | |
| 2 | SO | o-Cl | $CH_2CH_2CH_3$ | H | |
| 2 | SO | o-Cl | $CH_2CH_2CH_2CH_3$ | H | |
| 2 | SO | o-Cl | $CH(CH_3)_2$ | H | |
| 2 | SO | o-Cl | $CH(CH_3)CH_2CH_3$ | H | |
| 2 | SO | o-Cl | $CH_2CH(CH_3)_2$ | H | |
| 2 | SO | m-Cl | H | H | |
| 2 | SO | m-Cl | $CH_3$ | H | |
| 2 | SO | m-Cl | $CH_2CH_3$ | H | |
| 2 | SO | m-Cl | $CH_2CH_2CH_3$ | H | |
| 2 | SO | m-Cl | $CH_2CH_2CH_2CH_3$ | H | |
| 2 | SO | m-Cl | $CH(CH_3)_2$ | H | |
| 2 | SO | m-Cl | $CH(CH_3)CH_2CH_3$ | H | |
| 2 | SO | m-Cl | $CH_2CH(CH_3)_2$ | H | |
| 2 | SO | o-$CH_3$ | H | H | |
| 2 | SO | o-$CH_3$ | $CH_3$ | H | |
| 2 | SO | o-$CH_3$ | $CH_2CH_3$ | H | |
| 2 | SO | o-$CH_3$ | $CH_2CH_2CH_3$ | H | |
| 2 | SO | o-$CH_3$ | $CH_2CH_2CH_2CH_3$ | H | |
| 2 | SO | o-$CH_3$ | $CH(CH_3)_2$ | H | |
| 2 | SO | o-$CH_3$ | $CH(CH_3)CH_2CH_3$ | H | |
| 2 | SO | o-$CH_3$ | $CH_2CH(CH_3)_2$ | H | |
| 2 | SO | m-$CH_3$ | H | H | |

Table 18 (continued)

| q | $Q_3$ | $R_{21}$ | $R_{22}$ | $R_{23}$ | m.p.(°C) |
|---|---|---|---|---|---|
| 2 | SO | m-$CH_3$ | $CH_3$ | H | |
| 2 | SO | m-$CH_3$ | $CH_2CH_3$ | H | |
| 2 | SO | m-$CH_3$ | $CH_2CH_2CH_3$ | H | |
| 2 | SO | m-$CH_3$ | $CH_2CH_2CH_2CH_3$ | H | |
| 2 | SO | m-$CH_3$ | $CH(CH_3)_2$ | H | |
| 2 | SO | m-$CH_3$ | $CH(CH_3)CH_2CH_3$ | H | |
| 2 | SO | m-$CH_3$ | $CH_2CH(CH_3)_2$ | H | |
| 2 | SO | o-$OCH_3$ | H | H | |
| 2 | SO | o-$OCH_3$ | $CH_3$ | H | |
| 2 | SO | o-$OCH_3$ | $CH_2CH_3$ | H | |
| 2 | SO | o-$OCH_3$ | $CH_2CH_2CH_3$ | H | |
| 2 | SO | o-$OCH_3$ | $CH_2CH_2CH_2CH_3$ | H | |
| 2 | SO | o-$OCH_3$ | $CH(CH_3)_2$ | H | |
| 2 | SO | o-$OCH_3$ | $CH(CH_3)CH_2CH_3$ | H | |
| 2 | SO | o-$OCH_3$ | $CH_2CH(CH_3)_2$ | H | |
| 2 | SO | m-$OCH_3$ | H | H | |
| 2 | SO | m-$OCH_3$ | $CH_3$ | H | |
| 2 | SO | m-$OCH_3$ | $CH_2CH_3$ | H | |
| 2 | SO | m-$OCH_3$ | $CH_2CH_2CH_3$ | H | |
| 2 | SO | m-$OCH_3$ | $CH_2CH_2CH_2CH_3$ | H | |
| 2 | SO | m-$OCH_3$ | $CH(CH_3)_2$ | H | |
| 2 | SO | m-$OCH_3$ | $CH(CH_3)CH_2CH_3$ | H | |
| 2 | SO | m-$OCH_3$ | $CH_2CH(CH_3)_2$ | H | |
| 2 | $SO_2$ | H | H | H | |
| 2 | $SO_2$ | H | $CH_3$ | H | |
| 2 | $SO_2$ | H | $CH_3$ | $CH_3$ | |
| 2 | $SO_2$ | H | $CH_2CH_3$ | H | |
| 2 | $SO_2$ | H | $CH_2CH_2CH_3$ | H | |
| 2 | $SO_2$ | H | $CH_2CH_2CH_2CH_3$ | H | |
| 2 | $SO_2$ | H | $CH(CH_3)_2$ | H | |
| 2 | $SO_2$ | H | $CH(CH_3)CH_2CH_3$ | H | |
| 2 | $SO_2$ | H | $CH_2CH(CH_3)_2$ | H | |

Table 18 (continued)

| q | $Q_3$ | $R_{21}$ | $R_{22}$ | $R_{23}$ | m.p.(°C) |
|---|---|---|---|---|---|
| 2 | $SO_2$ | o-Cl | H | H | |
| 2 | $SO_2$ | o-Cl | $CH_3$ | H | |
| 2 | $SO_2$ | o-Cl | $CH_3$ | $CH_3$ | |
| 2 | $SO_2$ | o-Cl | $CH_2CH_3$ | H | |
| 2 | $SO_2$ | o-Cl | $CH_2CH_2CH_3$ | H | |
| 2 | $SO_2$ | o-Cl | $CH_2CH_2CH_2CH_3$ | H | |
| 2 | $SO_2$ | o-Cl | $CH(CH_3)_2$ | H | |
| 2 | $SO_2$ | o-Cl | $CH(CH_3)CH_2CH_3$ | H | |
| 2 | $SO_2$ | o-Cl | $CH_2CH(CH_3)_2$ | H | |
| 2 | $SO_2$ | m-Cl | H | H | |
| 2 | $SO_2$ | m-Cl | $CH_3$ | H | |
| 2 | $SO_2$ | m-Cl | $CH_3$ | $CH_3$ | |
| 2 | $SO_2$ | m-Cl | $CH_2CH_3$ | H | |
| 2 | $SO_2$ | m-Cl | $CH_2CH_2CH_3$ | H | |
| 2 | $SO_2$ | m-Cl | $CH_2CH_2CH_2CH_3$ | H | |
| 2 | $SO_2$ | m-Cl | $CH(CH_3)_2$ | H | |
| 2 | $SO_2$ | m-Cl | $CH(CH_3)CH_2CH_3$ | H | |
| 2 | $SO_2$ | m-Cl | $CH_2CH(CH_3)_2$ | H | |
| 2 | $SO_2$ | o-$CH_3$ | H | H | |
| 2 | $SO_2$ | o-$CH_3$ | $CH_3$ | H | |
| 2 | $SO_2$ | o-$CH_3$ | $CH_3$ | $CH_3$ | |
| 2 | $SO_2$ | o-$CH_3$ | $CH_2CH_3$ | H | |
| 2 | $SO_2$ | o-$CH_3$ | $CH_2CH_2CH_3$ | H | |
| 2 | $SO_2$ | o-$CH_3$ | $CH_2CH_2CH_2CH_3$ | H | |
| 2 | $SO_2$ | o-$CH_3$ | $CH(CH_3)_2$ | H | |
| 2 | $SO_2$ | o-$CH_3$ | $CH(CH_3)CH_2CH_3$ | H | |
| 2 | $SO_2$ | o-$CH_3$ | $CH_2CH(CH_3)_2$ | H | |
| 2 | $SO_2$ | m-$CH_3$ | H | H | |
| 2 | $SO_2$ | m-$CH_3$ | $CH_3$ | H | |
| 2 | $SO_2$ | m-$CH_3$ | $CH_3$ | $CH_3$ | |
| 2 | $SO_2$ | m-$CH_3$ | $CH_2CH_3$ | H | |
| 2 | $SO_2$ | m-$CH_3$ | $CH_2CH_2CH_3$ | H | |

Table 18 (continued)

| q | $Q_3$ | $R_{21}$ | $R_{22}$ | $R_{23}$ | m.p.(°C) |
|---|---|---|---|---|---|
| 2 | $SO_2$ | m-$CH_3$ | $CH_2CH_2CH_2CH_3$ | H | |
| 2 | $SO_2$ | m-$CH_3$ | $CH(CH_3)_2$ | H | |
| 2 | $SO_2$ | m-$CH_3$ | $CH(CH_3)CH_2CH_3$ | H | |
| 2 | $SO_2$ | m-$CH_3$ | $CH_2CH(CH_3)_2$ | H | |
| 2 | $SO_2$ | o-$OCH_3$ | H | H | |
| 2 | $SO_2$ | o-$OCH_3$ | $CH_3$ | H | |
| 2 | $SO_2$ | o-$OCH_3$ | $CH_3$ | $CH_3$ | |
| 2 | $SO_2$ | o-$OCH_3$ | $CH_2CH_3$ | H | |
| 2 | $SO_2$ | o-$OCH_3$ | $CH_2CH_2CH_3$ | H | |
| 2 | $SO_2$ | o-$OCH_3$ | $CH_2CH_2CH_2CH_3$ | H | |
| 2 | $SO_2$ | o-$OCH_3$ | $CH(CH_3)_2$ | H | |
| 2 | $SO_2$ | o-$OCH_3$ | $CH(CH_3)CH_2CH_3$ | H | |
| 2 | $SO_2$ | o-$OCH_3$ | $CH_2CH(CH_3)_2$ | H | |
| 2 | $SO_2$ | m-$OCH_3$ | H | H | |
| 2 | $SO_2$ | m-$OCH_3$ | $CH_3$ | H | |
| 2 | $SO_2$ | m-$OCH_3$ | $CH_3$ | $CH_3$ | |
| 2 | $SO_2$ | m-$OCH_3$ | $CH_2CH_3$ | H | |
| 2 | $SO_2$ | m-$OCH_3$ | $CH_2CH_2CH_3$ | H | |
| 2 | $SO_2$ | m-$OCH_3$ | $CH_2CH_2CH_2CH_3$ | H | |
| 2 | $SO_2$ | m-$OCH_3$ | $CH(CH_3)_2$ | H | |
| 2 | $SO_2$ | m-$OCH_3$ | $CH(CH_3)CH_2CH_3$ | H | |
| 2 | $SO_2$ | m-$OCH_3$ | $CH_2CH(CH_3)_2$ | H | |

Table 19

General Structure 19

| $R_{21}$ | $R_{24}$ |
|---|---|
| H | H |
| H | $CH_3$ |
| o-Cl | H |
| o-Cl | $CH_3$ |
| m-Cl | H |
| m-Cl | $CH_3$ |
| o-$CH_3$ | H |
| o-$CH_3$ | $CH_3$ |
| m-$CH_3$ | H |
| m-$CH_3$ | $CH_3$ |
| o-$OCH_3$ | H |
| o-$OCH_3$ | $CH_3$ |
| m-$OCH_3$ | H |
| m-$OCH_3$ | $CH_3$ |

Formulations

Useful formulations of the compounds of Formula I can be prepared in conventional ways. They include dusts, granules, pellets, solutions, suspensions, emulsions, wettable powders, emulsifiable concentrates and the like. Many of these may be applied directly. Sprayable formulations can be extended in suitable media and used at spray volumes of from a few liters to several hundred liters per hectare. High strength compositions are primarily used as intermediates for further formulation. The formulations, broadly, contain about 0.1% to 99% by weight of active ingredient(s) and at least one of (a) about 0.1% to 20% surfactant(s) and (b) about 1% to 99.9% solid or liquid inert diluent(s). More specifically, they will contain these ingredients in the following approximate proportions:

Table 20

| | Weight Percent* | | |
|---|---|---|---|
| | Active Ingredient | Diluent(s) | Surfactant(s) |
| Wettable Powders | 20-90 | 0-74 | 1-10 |
| Oil Suspensions, Emulsions, Solutions, (including Emulsifiable Concentrates) | 3-50 | 40-95 | 0-15 |
| Aqueous Suspension | 10-50 | 40-84 | 1-20 |
| Dusts | 1-25 | 70-99 | 0-5 |
| Granules and Pellets | 0.1-95 | 5-99.9 | 0-15 |
| High Strength Compositions | 90-99 | 0-10 | 0-2 |

* Active ingredient plus at least one of a Surfactant or a Diluent equals 100 weight percent.

Lower or higher levels of active ingredient can, of course, be present depending on the intended use and the physical properties of the compound. Higher ratios of surfactant to active ingredient are sometimes desirable, and are achieved by incorporation into the formulation or by tank mixing.

Typical solid diluents are described in Watkins, et al., "Handbook of Insecticide Dust Diluents and Carriers", 2nd Ed., Dorland Books, Caldwell, New Jersey, but other solids, either mined or manufactured, may be used. The more absorptive diluents are preferred for wettable powders and the denser ones for dusts. Typical liquid diluents and solvents are described in Marsden, "Solvents Guide", 2nd Ed., Interscience, New York, 1950. Solubility under 0.1% is preferred for suspension concentrates; solution concentrates are preferably stable against phase separation at 0°C. "McCutcheon's Detergents and Emulsifiers Annual", MC Publishing Corp., Ridgewood, New Jersey, as well as Siseley and Wood, "Encyclopedia of Surface Active Agents", Chemical Publishing Co., Inc., New York, 1964, list surfactants and recommended uses. All formulations can contain minor amounts of additives to reduce foaming, caking, corrosion, microbiological growth, etc.

The methods of making such compositions are well known. Solutions are prepared by simply mixing the ingredients. Fine solid compositions are made by blending and, usually, grinding as in a hammer or fluid energy mill. Suspensions are prepared by wet milling (see, for example, Littler, U.S. Patent 3,060,084). Granules and pellets may be made by spraying the active material upon preformed granular carriers or by agglomeration techniques. See J. E. Browning, "Agglomeration", *Chemical Engineering,* December 4, 1976, pp. 147ff. and "Perry's Chemical Engineer's Handbook", 5th Ed., McGraw-Hill, New York, 1973, pp. 8—57ff.

For further information regarding the art of formulation, see for example:

H. M. Loux, U.S. Patent 3,235,361, February 15, 1966, Col. 6, line 16 through Col. 7, line 19 and Examples 10 through 41;

R. W. Luckenbaugh, U.S. Patent 3,309,192, March 14, 1967, Col. 5, line 43 through Col. 7, line 62 and Examples 8, 12, 15, 39, 41, 52, 53, 58, 132, 138—140, 162—164, 166, 167 and 169—182;

H. Gysin and E. Knusli, U.S. Patent 2,891,855, June 23, 1959, Col. 3, line 66 through Col. 5, line 17 and Examples 1—4;

G. C. Klingman, "Weed Control as a Science", John Wiley and Sons, Inc., New York, 1961, pp. 81—96; and

J. D. Fryer and S. A. Evans, "Weed Control Handbook", 5th Ed., Blackwell Scientific Publications, Oxford, 1968, pp. 101—103.

In the following examples, all parts are by weight unless otherwise indicated.

Example 4

Wettable Powder

| | |
|---|---|
| 2-[[(4-iodo-6-methoxypyrimidin-2-yl)aminocarbonyl]aminosulfonyl]benzoic acid, methyl ester | 80% |
| sodium alkylnaphthalenesulfonate | 2% |
| sodium ligninsulfonate | 2% |
| synthetic amorphous silica | 3% |
| kaolinite | 13% |

The ingredients are blended, hammer-milled until all the solids are essentially under 50 microns, reblended, and packaged.

Example 5

Wettable Powder

| | |
|---|---|
| 2-[[(4-iodo-6-methoxypyrimidin-2-yl)aminocarbonyl]aminosulfonyl]benzoic acid, methyl ester | 50% |
| sodium alkylnaphthalenesulfonate | 2% |
| low viscosity methyl cellulose | 2% |
| diatomaceous earth | 46% |

The ingredients are blended, coarsely hammer-milled and then air-milled to produce particles essentially all below 10 microns in diameter. The product is reblended before packaging.

Example 6

Granule

| | |
|---|---|
| Wettable Powder of Example 3 | 5% |
| attapulgite granules (U.S.S. 20—40 mesh; 0.84—0.42 mm) | 95% |

A slurry of wettable powder containing 25% solids is sprayed on the surface of attapulgite granules while tumbling in a double-cone blender. The granules are dried and packaged.

Example 7

Extruded Pellet

| | |
|---|---|
| 2-[[(4-iodo-6-methoxypyrimidin-2-yl)aminocarbonyl]aminosulfonyl]benzoic acid, methyl ester | 25% |
| anhydrous sodium sulfate | 10% |
| crude calcium ligninsulfonate | 5% |
| sodium alkylnaphthalenesulfonate | 1% |
| calcium/magnesium bentonite | 59% |

The ingredients are blended, hammer-milled and then moistened with about 12% water. The mixture is extruded as cylinders about 3 mm diameter which are cut to produce pellets about 3 mm long. These may be used directly after drying, or the dried pellets may be crushed to pass a U.S.S. No. 20 sieve (0.84 mm openings). The granules held on a U.S.S. No. 40 sieve (0.42 mm openings) may be packaged for use and the fines recycled.

Example 8

Oil Suspension

| | |
|---|---|
| 2-[[(4-iodo-6-methoxypyrimidin-2-yl)aminocarbonyl]aminosulfonyl]benzoic acid, methyl ester | 25% |
| polyoxyethylene sorbitol hexaoleate | 5% |
| highly aliphatic hydrocarbon oil | 70% |

The ingredients are ground together in a sand mill until the solid particles have been reduced to under about 5-microns. The resulting thick suspension may be applied directly, but preferably after being extended with oils or emulsified in water.

Example 9

Wettable Powder

| | |
|---|---|
| N'[(4-iodo-6-methoxypyrimidin-2-yl)aminocarbonyl]-N,N-dimethyl-1,2-benzenedisulfonamide | 20% |
| sodium alkylnaphthalenesulfonate | 4% |
| sodium ligninsulfonate | 4% |
| low viscosity methyl cellulose | 3% |
| attapulgite | 69% |

The ingredients are thoroughly blended. After grinding in a hammer-mill to produce particles essentially all below 100 microns, the material is reblended and sifted through a U.S.S. No. 50 sieve (0.3 mm opening) and packaged.

Example 10

Low Strength Granule

| | |
|---|---|
| 2-[[(4-iodo-6-methoxypyrimidin-2-yl)aminocarbonyl]aminosulfonyl]benzoic acid, methyl ester | 1% |
| N,N-dimethylformamide | 9% |
| attapulgite granules (U.S.S. 20—40 sieve) | 90% |

The active ingredient is dissolved in the solvent and the solution is sprayed upon dedusted granules in a double cone blender. After spraying of the solution has been completed, the blender is allowed to run for a short period and then the granules are packaged.

Example 11

Aqueous Suspension

| | |
|---|---|
| 2-[[(4-iodo-6-methoxypyrimidin-2-yl)aminocarbonyl]aminosulfonyl]benzoic acid, methyl ester | 40% |
| polyacrylic acid thickener | 0.3% |
| dodecylphenol polyethylene glycol ether | 0.5% |
| disodium phosphate | 1.0% |
| monosodium phosphate | 0.5% |
| polyvinyl alcohol | 1.0% |
| water | 56.7% |

The ingredients are blended and ground together in a sand mill to produce particles essentially all under 5 microns in size.

Example 12

Solution

| | |
|---|---|
| 2-[[(4-iodo-6-methoxypyrimidin-2-yl)aminocarbonyl]aminosulfonyl]benzoic acid, methyl ester, sodium salt | 5% |
| water | 95% |

The salt is added directly to the water with stirring to produce the solution, which may then be packaged for use.

Example 13

Low Strength Granule

| | |
|---|---|
| 2-[[(4-iodo-6-methoxypyrimidin-2-yl)aminocarbonyl]aminosulfonyl]benzoic acid, methyl ester | 0.1% |
| attapulgite granules (U.S.S. 20—40 mesh) | 99.9% |

The active ingredient is dissolved in a solvent and the solution is sprayed upon dedusted granules in a double-cone blender. After spraying of the solution has been completed, the material is warmed to evaporate the solvent. The material is allowed to cool and then packaged.

Example 14

Granule

| | |
|---|---|
| N'-[(4-iodo-6-methoxypyrimidin-2-yl)aminocarbonyl]-N,N-dimethyl-1,2-benzenedisulfonamide | 80% |
| wetting agent | 1% |
| crude ligninsulfonate salt (containing 5—20% of the natural sugars) | 10% |
| attapulgite clay | 9% |

The ingredients are blended and milled to pass through a 100 mesh screen. This material is then added to a fluid bed granulator, the air flow is adjusted to gently fluidize the material, and a fine spray of water is sprayed onto the fluidized material. The fluidization and spraying are continued until granules of the desired size range are made. The spraying is stopped, but fluidization is continued, optionally with heat, until the water content is reduced to the desired level, generally less than 1%. The material is then discharged, screened to the desired size range, generally 14—100 mesh (1410—149 microns), and packaged for use.

Example 15

High Strength Concentrate

| | |
|---|---|
| 2-[[(4-iodo-6-methoxypyrimidin-2-yl)aminocarbonyl]aminosulfonyl]benzoic acid, methyl ester | 99% |
| silica aerogel | 0.5% |
| synthetic amorphous silica | 0.5% |

The ingredients are blended and ground in a hammer-mill to produce a material essentially all passing a U.S.S. No. 50 screen (0.3 mm opening). The concentrate may be formulated further if necessary.

Example 16

Wettable Powder

| | |
|---|---|
| 2-[[(4-iodo-6-methoxypyrimidin-2-yl)aminocarbonyl]aminosulfonyl]benzoic acid, methyl ester | 90% |
| dioctyl sodium sulfosuccinate | 0.1% |
| synthetic fine silica | 9.9% |

The ingredients are blended and ground in a hammer-mill to produce particles essentially all below 100 microns. The material is sifted through a U.S.S. No. 50 screen and then packaged.

Example 17

Wettable Powder

| | |
|---|---|
| 2-[[(4-iodo-6-methoxypyrimidin-2-yl)aminocarbonyl]aminosulfonyl]benzoic acid, methyl ester | 40% |
| sodium ligninsulfonate | 20% |
| montmorillonite clay | 40% |

The ingredients are thoroughly blended, coarsely hammer-milled and then air-milled to produce particles essentially all below 10 microns in size. The material is reblended and then packaged.

Example 18

Oil Suspension

| | |
|---|---|
| 2-[[(4-iodo-6-methoxypyrimidin-2-yl)aminocarbonyl]aminosulfonyl]benzoic acid, methyl ester | 35% |
| blend of polyalcohol carboxylic esters and oil soluble petroleum sulfonates | 6% |
| xylene | 59% |

The ingredients are combined and ground together in a sand mill to produce particles essentially all below 5 microns. The product can be used directly, extended with oils, or emulsified in water.

Example 19

Dust

| | |
|---|---|
| 2-[[(4-iodo-6-methoxypyrimidin-2-yl)aminocarbonyl]aminosulfonyl]benzoic acid, methyl ester | 10% |
| attapulgite | 10% |
| Pyrophyllite | 80% |

The active ingredient is blended with attapulgite and then passed through a hammer-mill to produce particles substantially all below 200 microns. The ground concentrate is then blended with powdered pyrophyllite until homogeneous.

Example 20

High Strength Concentrate

| | |
|---|---|
| N-[(4-bromo-6-methoxypyrimidin-2-yl)aminocarbonyl]-2,3-dihydrobenzo[b]-thiophene-7-sulfonamide, 1,1-dioxide | 99% |
| silica aerogel | 0.5% |
| synthetic amorphous silica | 0.5% |

The ingredients are blended and ground in a hammer-mill to produce a material essentially all passing a U.S.S. No. 50 screen (0.3 mm opening). The concentrate may be formulated further if necessary.

Example 21

Wettable Powder

| | |
|---|---|
| N-[(4-bromo-6-methoxypyrimidin-2-yl)aminocarbonyl]-2,3-dihydrobenzo[b]thiophene-7-sulfonamide, 1,1-dioxide | 90% |
| dioctyl sodium sulfosuccinate | 0.1% |
| synthetic fine silica | 9.9% |

The ingredients are blended and ground in a hammer-mill to produce particles essentially all below 100 microns. The material is sifted through a U.S.S. No. 50 screen and then packaged.

Example 22

Wettable Powder

| | |
|---|---|
| N-[(4-bromo-6-methoxypyrimidin-2-yl)aminocarbonyl]-2,3-dihydro-2-methylbenzo[b]thiophene-7-sulfonamide, 1,1-dioxide | 40% |
| sodium ligninsulfonate | 20% |
| montmorillonite clay | 40% |

The ingredients are thoroughly blended, coarsely hammer-milled and then air-milled to produce particles essentially all below 10 microns in size. The material is reblended and then packaged.

Utility

Test results indicate that the compounds of the present invention are active herbicides. They have utility for broad-spectrum pre- and/or post-emergence weed control in areas where complete control of all vegetation is desired, such as around fuel storage tanks, ammunition depots, industrial storage areas, parking lots, drive-in theaters, around billboards, highway and railroad structures. Some of the compounds have utility for selective weed control in crops such as rice, wheat, barley, cotton, corn and soybeans. Alternatively, the subject compounds are useful to modify plant growth.

The rates of application for the compounds of the invention are determined by a number of factors, including their use as selective or general herbicides, the crop species involved, the type of weeds to be controlled, weather and climate, formulations selected, mode of application, amount of foliage present, etc. In general terms, the subject compounds should be applied at levels of around 0.01 to 5 kg/ha, the lower rates being suggested for use on lighter soils and/or those having a low organic matter content, for selective weed control or for situations where only short-term persistence is required.

The compounds of the invention may be used in combination with any other commercial herbicide; examples of which are those of the triazine, triazole, uracil, urea, amide, diphenylether, carbamate and bipyridylium types.

The herbicidal properties of the subject compounds were discovered in a number of greehouse tests. The test procedures and results follow.

Compounds

Compound 1

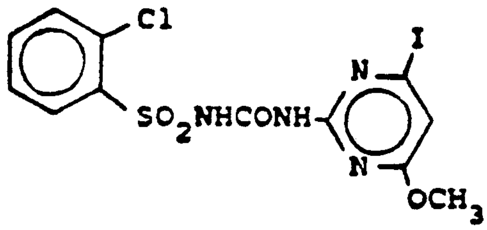

Compound 2

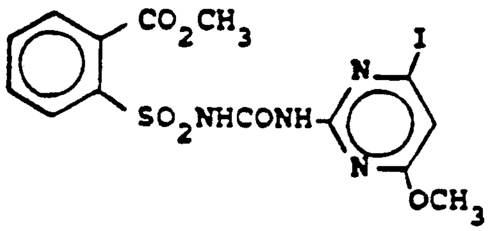

Compound 3

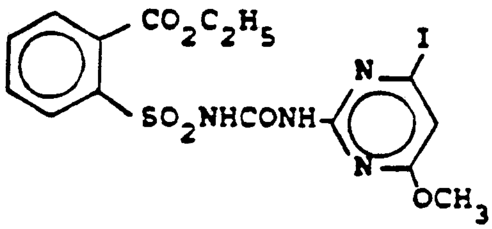

Compound 4

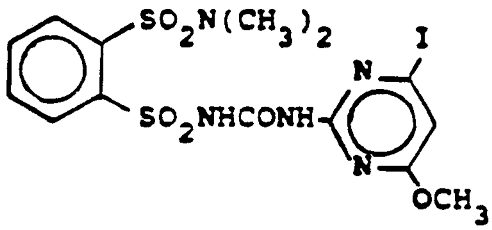

Compound 5

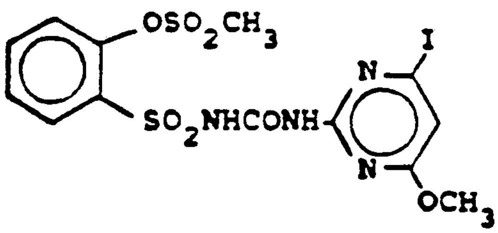

Compounds (Cont'd)

Compound 6

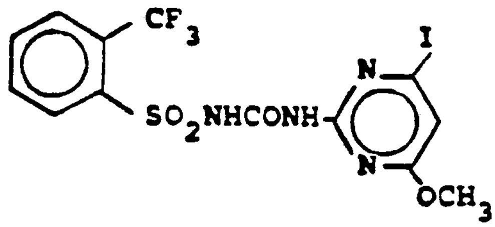

Compound 7

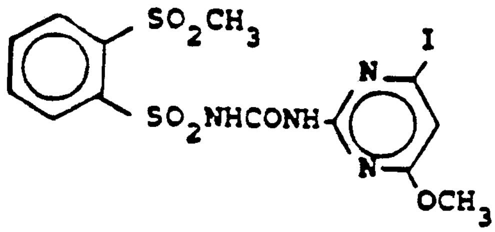

Compound 8

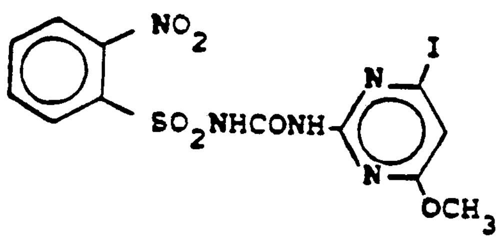

Compound 9

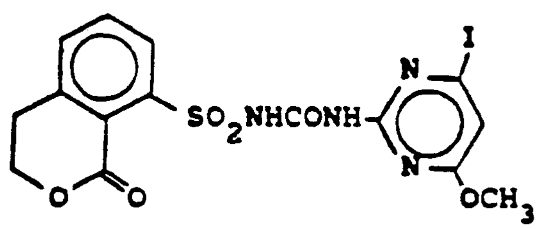

Compound 10

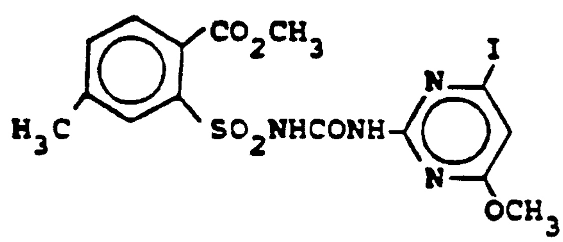

Compound 11

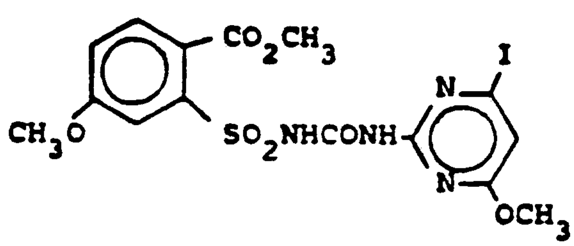

Compounds (Cont'd)

Compound 12

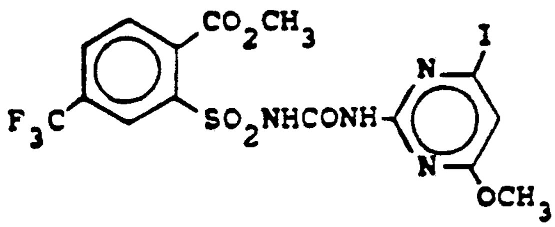

Compound 13

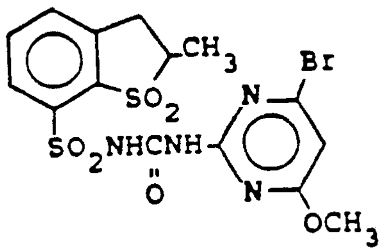

Compound 14

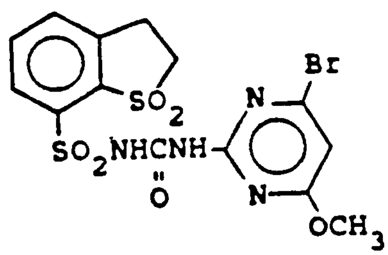

Test A

Seeds of crabgrass (*Digitaria* spp.), barnyardgrass (*Echinochloa crusqalli*), wild oats (*Avena fatua*), sicklepod (*Cassia obtusifolia*), morningglory (Ipomoea spp.), cocklebur (*Xanthium pensylvanicum*), sorghun, corn, soybean, sugar beet, cotton, rice, wheat, and purple nutsedge (*Cyperus rotundus*) tubers were planted and treated preemergence with the test chemicals dissolved in a non-phytoxic solvent. At the same time, these crop and weed species were treated with a soil/foliage application. At the time of treatment, the plants ranged in height from 2 to 18 cm. Treated plants and controls were maintained in greenhouse for sixteen days, after which all aspects were compared to controls and visually rated for response to treatment. The ratings, summarized in Table A, are based on a numerical scale extending from 0 = no injury, to 10 = complete kill. The accompanying descriptive symbols have the following meanings:

C = chlorosis/necrosis
B = burn
D = defoliation
E = emergence inhibition
G = growth retardation
H = formative effect
U = unusual pigmentation
X = axilliary stimulation
S = albinism
6Y = abscised buds or flowers

Table A

| | Cmpd. 1 | Cmpd. 2 | Cmpd. 3 | Cmpd. 4 |
|---|---|---|---|---|
| Rate (kg/ha) | 0.05 | 0.05 | 0.05 | 0.05 |
| POSTEMERGENCE | | | | |
| Morningglory | 2C | 5C, 9G | 1C,4G | 4C,9G |
| Cocklebur | 9C | 10C | 10C | 10C |
| Cassia | 2C | 6C, 9G | 2C,6G | 4C,8G |
| Nutsedge | 2C | 3C, 8G | 7G | 2C,5G |
| Crabgrass | 0 | 2C, 6G | 2G | 4G |
| Barnyardgrass | 0 | 3C, 9H | 2C,9H | 3C,9H |
| Wild Oats | 0 | 2C, 6G | 2G | 0 |
| Wheat | 0 | 2C, 4G | 2G | 1C,4G |
| Corn | 3G | 1U, 7H | 2C,6H | 3H |
| Soybean | 0 | 3C, 5G | 3G | 1C,1H |
| Rice | 2G | 3C, 8G | 2C,8G | 2C,8G |
| Sorghum | 0 | 2C, 8G | 9G | 7H |
| Sugar Beets | 5C,9G | 4C, 9G | 3C,8G | 3C,5G |
| Cotton | 2C,4G | 9C | 5C,9G | 5C,9G |
| PREEMERGENCE | | | | |
| Morningglory | 2C,8G | 9G | 5G | 7G |
| Cocklebur | 9H | 5G | 7H | 8G |
| Cassia | 6G | 5G | 0 | 5G |
| Nutsedge | 0 | 2G | 7G | 10E |
| Crabgrass | 0 | 0 | 0 | 0 |
| Barnyardgrass | 1C | 3G | 4G | 5G |
| Wild Oats | 0 | 5G | 3G | 0 |
| Wheat | 0 | 3G | 0 | 0 |
| Corn | 2C,6G | 6G | 4G | 5G |
| Soybean | 0 | 0 | 0 | 0 |
| Rice | 4G | 8G | 9G | 2C,7H |
| Sorghum | 3G | 7G | 5G | 6G |
| Sugar Beets | 9G | 7G | 8G | 6G |
| Cotton | 9G | 8G | 4G | 10E |

Table A (continued)

| | Cmpd. 5 | Cmpd. 6 | Cmpd. 7 | Cmpd. 8 |
|---|---|---|---|---|
| Rate (kg/ha) | 0.05 | 0.05 | 0.05 | 0.05 |
| POSTEMERGENCE | | | | |
| Morningglory | 5C,9G | 5C,9G | 3C,8H | 2C,8G |
| Cocklebur | 9C | 9C | 9C | 10C |
| Cassia | 5C,9G | 3C,5G | 3C,8H | 4C,9G |
| Nutsedge | 2C,9G | 2C,9G | 2C,8G | 9C |
| Crabgrass | 4G | 3G | 2C,5G | 2C,4G |
| Barnyardgrass | 9H | 3C,8G | 3C,9H | 3C,8H |
| Wild Oats | 1C | 2C | 2G | 2G |
| Wheat | 0 | 0 | 2G | 2G |
| Corn | 2C,9H | 3C,6G | 2C,8H | 2C,5G |
| Soybean | 3C,7G | 3C,5G | 2C,2H | 3H |
| Rice | 3C,8G | 2C,8G | 4C,9G | 2C,7G |
| Sorghum | 9G | 5G | 1C,9G | 5G |
| Sugar Beets | 4C,8G | 4C,8G | 3C,7G | 9C |
| Cotton | 9C | 6C,9G | 10C | 10C |
| PREEMERGENCE | | | | |
| Morningglory | 9G | 9G | 9G | 9G |
| Cocklebur | 9H | - | 8H | - |
| Cassia | 7G | 8G | 8G | 7G |
| Nutsedge | 10E | 0 | 10E | 5G |
| Crabgrass | 5G | 2G | 7G | 0 |
| Barnyardgrass | 4G | 3C,3H | 2C,8G | 0 |
| Wild Oats | 2C,4G | 2C,3G | 5G | 0 |
| Wheat | 0 | 0 | 0 | 2G |
| Corn | 2C,7G | 3C,6G | 8G | 1C,6G |
| Soybean | 2C,4H | 2C,3H | 3H | 2H |
| Rice | 7G | 3C,7G | 10E | 5G |
| Sorghum | 8G | 2C,6G | 2C,9G | 3G |
| Sugar Beets | 9G | 9G | 8G | 8G |
| Cotton | 9G | 8G | 9G | 9G |

Table A (continued)

| | Cmpd. 9 | Cmpd. 10 | Cmpd. 11 | Cmpd. 12 |
|---|---|---|---|---|
| Rate (kg/ha) | 0.05 | 0.05 | 0.05 | 0.05 |
| POSTEMERGENCE | | | | |
| Morningglory | 9C | 5C,9G | 5C,9G | 2C |
| Cocklebur | 10C | 9C | 10C | 5C,9G |
| Cassia | 9C | 9C | 4C,9G | 1C |
| Nutsedge | 2C,9G | 5C,9G | 9C | 2C,5G |
| Crabgrass | 5C,9G | 5C,8G | 3C,8G | 2G |
| Barnyardgrass | 9C | 5C,9H | 3C,7H | 2C,2G |
| Wild Oats | 5C,9H | 1C | 3G | 0 |
| Wheat | 9C | 0 | 2G | 0 |
| Corn | 9C | 2C,8H | 9H | 0 |
| Soybean | 2C,4H | 3C,8G | 4C,9G | 1H |
| Rice | 5C,9G | 9G | 2C,8G | 0 |
| Sorghum | 5C,9G | 2C,9G | 3C,8H | 0 |
| Sugar Beets | 10C | 9C | 5C,9G | 3C,7G |
| Cotton | 10C | 9C | 5C,9G | 3C,7G |
| PREEMERGENCE | | | | |
| Morningglory | 9G | 9G | 9G | 6G |
| Cocklebur | 8G | - | 9G | 9H |
| Cassia | 8G | 7G | 8G | 6G |
| Nutsedge | 9G | 10E | 5G | 0 |
| Crabgrass | 3C,8G | 3G | 2G | 0 |
| Barnyardgrass | 2C,8G | 2C,7G | 3G | 0 |
| Wild Oats | 5G | 2C | 2C,6G | 0 |
| Wheat | 4G | 0 | 4G | 0 |
| Corn | 2C,8G | 3C,8G | 2C,8G | 2C,5G |
| Soybean | 2H | 0 | 3C,6G | 0 |
| Rice | 10E | 2C,7G | 6G | 0 |
| Sorghum | 2C,9G | 2C,9H | 8G | 0 |
| Sugar Beets | 4C,9G | 2C,8G | 9G | 3G |
| Cotton | 9G | 9G | 9G | 7G |

Table A (continued)

| | Cmpd. 13 | Cmpd. 14 |
|---|---|---|
| Rate kg/ha | 0.05 | 0.05 |
| POSTEMERGENCE | | |
| Morningglory | 3C,6G | 5C,9G |
| Cocklebur | 10C | 6C,9G |
| Sicklepod | 1C,2H | 3C,7G |
| Nutsedge | 3C,8G | 2C,8G |
| Crabgrass | 4C,8G | 6C,9G |
| Barnyardgrass | 10C | 10C |
| Wild Oats | 0 | 0 |
| Wheat | 0 | 2G |
| Corn | 10C | 3C,9G |
| Soybean | 2H,3G | 5G |
| Rice | 5C,9G | 5C,9G |
| Sorghum | 10C | 9C |
| Sugar beet | 9C | 5C,9G |
| Cotton | 4C,9G | 2C,9G |
| PREEMERGENCE | | |
| Morningglory | 9G | 8G |
| Cocklebur | 8G | 9H |
| Sicklepod | 2C,7G | 9G |
| Nutsedge | 10E | 9G |
| Crabgrass | 2G | 2C,8G |
| Barnyardgrass | 4C,8H | 3C,9H |
| Wild Oats | 1C | 1C |
| Wheat | 2G | 2G |
| Corn | 2C,8G | 10E |
| Soybean | 1C,3G | 1H |
| Rice | 10E | 10E |
| Sorghum | 3C,9G | 3C,9H |
| Sugar beet | 10E | 9G |
| Cotton | 9G | 8G |

Test B

Postemergence

Two round pans (25 cm diameter by 12.5 cm deep) were filled with sassafras sandy loam soil. One pan was planted with blackgrass (*Alopecurus myosuroides*), sugar beets, nutsedge (*Cyperus rotundus*) tubers, crabgrass, (*Digitaria sanquinalis*), sicklepod (*Cassia obtusifolia*), teaweed (*Sida spinosa*), jimsonweed (*Datura stramonium*), velvetleaf (*Abutilon theophrasti*), and giant foxtail (*Setaria faverii*). The other pan was planted with wheat, cotton, rice, corn, soybean, wild oats (*Avena fatua*), cocklebur (*Xanthium pensylvanicum*), morningglory (*Ipomoea hederacea*), johnsongrass (*Sorghum halepense*) and barnyardgrass (*Echinochloa crusgalli*). The plants were grown for approximately fourteen days, then sprayed postemergence with the chemicals dissolved in a non-phytotoxic solvent.

Preemergence

Two round pans (25 cm diameter by 12.5 cm deep) were filled with sassafras sandy loam soil. One pan was planted with blackgrass, sugar beets, nutsedge, crabgrass, sicklepod, teaweed, jimsonweed, velvetleaf, and giant foxtail. The other pan was planted with wheat, cotton, rice, corn, soybeans, wild oats, cocklebur, morningglory, jonsongrass, and barnyardgrass. The two pans were sprayed preemergence with the chemicals dissolved in a non-phytoxic solvent.

Treated plants and controls were maintained in the greenhouse for 28 days, then all treated plants were compared to controls and visually rated for plant response. The response rating system was described for Test A.

Response ratings are contained in Table B.

Table B

| | Compound 2 | | | Compound 4 | | | |
|---|---|---|---|---|---|---|---|
| Rate (g/ha) | 62 | 16 | 04 | 250 | 62 | 16 | 04 |
| POSTEMERGENCE | | | | | | | |
| Corn | 5G | 0 | 0 | 3G | 2G | 0 | 0 |
| Wheat | 0 | 0 | 0 | 0 | 0 | 0 | 0 |
| Rice | 9G | 5G | 2G | 9G | 8G | 5G | 0 |
| Soybean | 2G | 0 | 0 | 2G | 0 | 0 | 0 |
| Cotton | 9G | 7G | 2G | 9G | 8G | 6G | 2G |
| Sugar beet | 9G | 8G | 2G | 9G | 7G | 3G | 0 |
| Crabgrass | 7G | 3G | 0 | 9G | 9G | 8G | 4G |
| Johnsongrass | 8G | 7G | 3G | 9G | 7G | 5G | 0 |
| Blackgrass | 8G | 7G | 2G | 10G | 9G | 5G | 0 |
| Barnyardgrass | 9G | 8G | 4G | 9G | 8G | 4G | 0 |
| Nutsedge | 9G | 4G | 0 | 9G | 8G | 5G | 2G |
| Giant Foxtail | 6G | 2G | 0 | 8G | 6G | 2G | 0 |
| Wild Oats | 4G | 0 | 0 | 0 | 0 | 0 | 0 |
| Cocklebur | 9G | 7G | 0 | 10G | 9G | 4G | 0 |
| Morningglory | 9G | 8G | 2G | 10G | 7G | 3G | 0 |
| Teaweed | 9G | 5G | 0 | 9G | 9G | 8G | 2G |
| Cassia | 9G | 5G | 0 | 9G | 9G | 6G | 2G |
| Jimsonweed | 9G | 7G | 3G | 10G | 8G | 6G | 2G |
| Velvetleaf | 9G | 7G | 4G | 9G | 9G | 7G | 3G |
| Rate (g/ha) | 250 | 62 | 16 | 250 | 62 | | |
| PREEMERGENCE | | | | | | | |
| Corn | 3G | 0 | 0 | 0 | 0 | | |
| Wheat | 3G | 0 | 0 | 0 | 0 | | |
| Rice | 9G | 9G | 8G | 9G | 8G | | |
| Soybean | 3G | 0 | 0 | 0 | 0 | | |
| Cotton | 6G | 3G | 0 | 2G | 0 | | |
| Sugar beet | 8G | 7G | 3G | 5G | 3G | | |
| Crabgrass | 8G | 7G | 2G | 9G | 3G | | |
| Johnsongrass | 9G | 8G | 3G | 8G | 6G | | |
| Blackgrass | 9G | 9G | 8G | 9G | 9G | | |
| Barnyardgrass | 9G | 7G | 3G | 9G | 7G | | |
| Nutsedge | 9G | 8G | 0 | 9G | 8G | | |
| Giant Foxtail | 10E | 7G | 2G | 9G | 8G | | |
| Wild Oats | 6G | 4G | 0 | 0 | 0 | | |
| Cocklebur | 7G | 3G | 0 | 6G | 0 | | |
| Morningglory | 8G | 4G | 0 | 7G | 0 | | |
| Teaweed | 9G | 9G | 4G | 9G | 7G | | |
| Cassia | 8G | 6G | 2G | 7G | 2G | | |
| Jimsonweed | 9G | 7G | 4G | 9G | 9G | | |
| Velvetleaf | 9G | 8G | 4G | 7G | 5G | | |

Table B (continued)

| | Compound 5 | | | Compound 6 | | |
|---|---|---|---|---|---|---|
| Rate (g/ha) | 62 | 16 | 04 | 250 | 62 | 16 |
| POSTEMERGENCE | | | | | | |
| Corn | 4G | 0 | 0 | 0 | 0 | 0 |
| Wheat | 0 | 0 | 0 | 0 | 0 | 0 |
| Rice | 5G | 0 | 0 | 7G | 3G | 0 |
| Soybean | 0 | 0 | 0 | 2G | 0 | 0 |
| Cotton | 7G | 4G | 0 | 6G | 2G | 0 |
| Sugar beet | 7G | 2G | 0 | 9G | 7G | 3G |
| Crabgrass | 6G | 2G | 0 | 6G | 2G | 0 |
| Johnsongrass | 8G | 3G | 0 | 5G | 2G | 0 |
| Blackgrass | 2G | 0 | 0 | 4G | 0 | 0 |
| Barnyardgrass | 7G | 3G | 0 | 7G | 2G | 0 |
| Nutsedge | 9G | 4G | 0 | 6G | 0 | 0 |
| Giant Foxtail | 9G | 2G | 0 | 4G | 0 | 0 |
| Wild Oats | 0 | 0 | 0 | 0 | 0 | 0 |
| Cocklebur | 9G | 5G | 0 | 9G | 6G | 0 |
| Morningglory | 9G | 4G | 0 | 10G | 9G | 2G |
| Teaweed | 9G | 7G | 2G | 9G | 7G | 2G |
| Cassia | 10G | 9G | 3G | 9G | 8G | 3G |
| Jimsonweed | 10G | 5G | 0 | 9G | 9G | 2G |
| Velvetleaf | 9G | 4G | 0 | 9G | 8G | 3G |
| Rate (g/ha) | 250 | 62 | 16 | 250 | 62 | |
| PREEMERGENCE | | | | | | |
| Corn | 5G | 2G | 0 | 0 | 0 | |
| Wheat | 0 | 0 | 0 | 0 | 0 | |
| Rice | 9G | 6G | 4G | 9G | 7G | |
| Soybean | 0 | 0 | 0 | 0 | 0 | |
| Cotton | 5G | 2G | 0 | 2G | 0 | |
| Sugar beet | 6G | 3G | 0 | 7G | 3G | |
| Crabgrass | 7G | 3G | 0 | 0 | 0 | |
| Johnsongrass | 8G | 6G | 2G | 3G | 0 | |
| Blackgrass | 8G | 6G | 2G | 8G | 6G | |
| Barnyardgrass | 8G | 4G | 0 | 6G | 2G | |
| Nutsedge | 9G | 9G | 3G | 2G | 0 | |
| Giant Foxtail | 8G | 3G | 0 | 8G | 2G | |
| Wild Oats | 0 | 0 | 0 | 0 | 0 | |
| Cocklebur | 6G | 3G | 0 | 6G | 0 | |
| Morningglory | 7G | 3G | 0 | 3G | 0 | |
| Teaweed | 8G | 5G | 0 | 8G | 3G | |
| Cassia | 7G | 2G | 0 | 4G | 0 | |
| Jimsonweed | 9G | 8G | 3G | 9G | 7G | |
| Velvetleaf | 8G | 4G | 0 | 8G | 6G | |

Table B (continued)

| | Compound 7 | | | Compound 10 | |
|---|---|---|---|---|---|
| Rate (g/ha) | 250 | 62 | 16 | 62 | 04 |
| POSTEMERGENCE | | | | | |
| Corn | 8G | 3G | 0 | 2G | 0 |
| Wheat | 0 | 0 | 0 | 0 | 0 |
| Rice | 9G | 5G | 2G | 5G | 0 |
| Soybean | 4G | 2G | 0 | 0 | 0 |
| Cotton | 9G | 7G | 3G | 7G | 0 |
| Sugar beet | 7G | 0 | 0 | 9G | 6G |
| Crabgrass | 9G | 3G | 0 | 6G | 2G |
| Johnsongrass | 10G | 9G | 9G | 10G | 5G |
| Blackgrass | 8G | 4G | 0 | 4G | 0 |
| Barnyardgrass | 9G | 8G | 7G | 8G | 2G |
| Nutsedge | 9G | 8G | 3G | 9G | 4G |
| Giant Foxtail | 9G | 9G | 6G | 8G | 0 |
| Wild Oats | 0 | 0 | 0 | 0 | 0 |
| Cocklebur | 9G | 9G | 3G | 10G | 5G |
| Morningglory | 8G | 4G | 0 | 10G | 2G |
| Teaweed | 10G | 6G | 2G | 9G | 4G |
| Cassia | 9G | 5G | 3G | 10G | 5G |
| Jimsonweed | 10G | 8G | 6G | 9G | 3G |
| Velvetleaf | 10G | 9G | 3G | 9G | 6G |
| Rate (g/ha) | 250 | 62 | 16 | 250 | 62 |
| PREEMERGENCE | | | | | |
| Corn | 7G | 5G | 0 | 2G | 0 |
| Wheat | 0 | 0 | 0 | 0 | 0 |
| Rice | 9G | 9G | 8G | 9G | 8G |
| Soybean | 0 | 0 | 0 | 4G | 0 |
| Cotton | 3G | 0 | 0 | 5G | 0 |
| Sugar beet | 3G | 0 | 0 | 7G | 3G |
| Crabgrass | 9G | 4G | 2G | 9G | 5G |
| Johnsongrass | 9G | 9G | 4G | 10G | 9G |
| Blackgrass | 9G | 9G | 6G | 8G | 7G |
| Barnyardgrass | 9G | 8G | 4G | 9G | 7G |
| Nutsedge | 10E | 9G | 3G | 9G | 8G |
| Giant Foxtail | 8G | 7G | 3G | 9G | 7G |
| Wild Oats | 0 | 0 | 0 | 2G | 0 |
| Cocklebur | 0 | 0 | 0 | 9G | 6G |
| Morningglory | 3G | 0 | 0 | 8G | 5G |
| Teaweed | 9G | 6G | 0 | 9G | 8G |
| Cassia | 4G | 0 | 0 | 7G | 0 |
| Jimsonweed | 9G | 8G | 7G | 9G | 7G |
| Velvetleaf | 7G | 0 | 0 | 9G | 7G |

Table B (continued)

Compound 11

| Rate (g/ha) | 62 | 16 | 04 | 01 |
|---|---|---|---|---|
| POSTEMERGENCE | | | | |
| Corn | 7G | 3G | 0 | 0 |
| Wheat | 0 | 0 | 0 | 0 |
| Rice | 2G | 0 | 0 | 0 |
| Soybean | 8G | 3G | 0 | 0 |
| Cotton | 10C | 10C | 8G | 0 |
| Sugar beet | 10C | 9G | 5G | 2G |
| Crabgrass | 3G | 0 | 0 | 0 |
| Johnsongrass | 6G | 2G | 0 | 0 |
| Blackgrass | 2G | 0 | 0 | 0 |
| Barnyardgrass | 5G | 0 | 0 | 0 |
| Nutsedge | 5G | 2G | 0 | 0 |
| Giant Foxtail | 3G | 0 | 0 | 0 |
| Wild Oats | 0 | 0 | 0 | 0 |
| Cocklebur | 10C | 8G | 6G | 2G |
| Morningglory | 10G | 10G | 4G | 0 |
| Teaweed | 8G | 7G | 4G | 2G |
| Cassia | 10C | 9G | 7G | 5G |
| Jimsonweed | 10C | 9G | 8G | 3G |
| Velvetleaf | 10C | 7G | 5G | 0 |

| Rate (g/ha) | 250 | 62 | 16 |
|---|---|---|---|
| PREEMERGENCE | | | |
| Corn | 3G | 0 | 0 |
| Wheat | 0 | 0 | 0 |
| Rice | 8G | 4G | 0 |
| Soybean | 3G | 0 | 0 |
| Cotton | 6G | 2G | 0 |
| Sugar beet | 10G | 10G | 6G |
| Crabgrass | 5G | 2G | 0 |
| Johnsongrass | 5G | 0 | 0 |
| Blackgrass | 8G | 6G | 2G |
| Barnyardgrass | 4G | 0 | 0 |
| Nutsedge | 8G | 3G | 0 |
| Giant Foxtail | 5G | 0 | 0 |
| Wild Oats | 0 | 0 | 0 |
| Cocklebur | 4G | 2G | 0 |
| Morningglory | 5G | 2G | 0 |
| Teaweed | 10G | 8G | 5G |
| Cassia | 10G | 9G | 4G |
| Jimsonweed | 10G | 9G | 3G |
| Velvetleaf | 10G | 9G | 3G |

Table B (continued)

Compound 13

| POSTEMERGENCE Rate g/ha | 62 | 16 | 4 | 1 |
|---|---|---|---|---|
| Corn | 10U | 10U | 9G | 7G |
| Wheat | 0 | 0 | 0 | 0 |
| Rice | 10C | 9G | 6G | 4G |
| Soybean | 6G | 4G | 3G | 2G |
| Cotton | 10C | 8G | 4G | 2G |
| Sugar beet | 10C | 10G | 9G | 9G |
| Crabgrass | 10G | 9G | 5G | 2G |
| Johnsongrass | 10C | 9G | 7G | 3G |
| Blackgrass | 9C | 8G | 2G | 0 |
| Barnyardgrass | 10C | 9G | 5G | 2G |
| Nutsedge | 4G | 2G | 0 | 0 |
| Giant Foxtail | 9G | 6G | 4G | 0 |
| Wild Oats | 0 | 0 | 0 | 0 |
| Cocklebur | 8G | 6G | 3G | 0 |
| Morningglory | 9G | 8G | 6G | 2G |
| Teaweed | 10G | 7G | 5G | 2G |
| Sicklepod | 7G | 4G | 4G | 0 |
| Jimsonweed | 10G | 5G | 3G | 2G |
| Velvetleaf | 10C | 6G | 6G | 4G |

| PREEMERGENCE Rate g/ha | 250 | 62 | 16 | 4 |
|---|---|---|---|---|
| Corn | 10G | 7G | 3G | 0 |
| Wheat | 0 | 0 | 0 | 0 |
| Rice | 10G | 10G | 8G | 4G |
| Soybean | 5G | 2G | 0 | 0 |
| Cotton | 8G | 3G | 0 | 0 |
| Sugar beet | 8G | 6G | 2G | 0 |
| Crabgrass | 10G | 9G | 3G | 0 |
| Johnsongrass | 10G | 10G | 8G | 3G |
| Blackgrass | 10G | 10G | 8G | 3G |
| Barnyardgrass | 10G | 10G | 7G | 2G |
| Nutsedge | 10G | 9G | 3G | 0 |
| Giant Foxtail | - | - | - | - |
| Wild Oats | 2G | 0 | 0 | 0 |
| Cocklebur | 7G | 3G | 0 | 0 |
| Morningglory | 9G | 6G | 3G | 0 |
| Teaweed | 9G | 8G | 4G | 0 |
| Sicklepod | 10G | 8G | 4G | 0 |
| Jimsonweed | 10G | 10G | 8G | 3G |
| Velvetleaf | 9G | 8G | 3G | 0 |

Table B (continued)

Compound 14

| POSTEMERGENCE Rate g/ha | 62 | 16 | 4 | 1 |
|---|---|---|---|---|
| Corn | 10G | 10G | 9G | 7G |
| Wheat | 0 | 0 | 0 | 0 |
| Rice | 10G | 10G | 9G | 7G |
| Soybean | 3G | 1G | 0 | 0 |
| Cotton | 9G | 6G | 3G | 0 |
| Sugar beet | 10G | 7G | 2G | 0 |
| Crabgrass | 10G | 10G | 8G | 3G |
| Johnsongrass | 10G | 9G | 9G | 6G |
| Blackgrass | 10G | 9G | 6G | 2G |
| Barnyardgrass | 10G | 10G | 7G | 4G |
| Nutsedge | 0 | 0 | 0 | 0 |
| Giant Foxtail | 10G | 9G | 7G | 4G |
| Wild Oats | 0 | 0 | 0 | 0 |
| Cocklebur | 10G | 6G | 2G | 0 |
| Morningglory | 10G | 6G | 2G | 0 |
| Teaweed | 8G | 4G | 0 | 0 |
| Sicklepod | 4G | 2G | 0 | 0 |
| Jimsonweed | 10G | 7G | 2G | 0 |
| Velvetleaf | 10G | 6G | 3G | 0 |

| PREEMERGENCE Rate g/ha | 250 | 62 | 16 | 4 |
|---|---|---|---|---|
| Corn | 10G | 9G | 4G | 0 |
| Wheat | 8G | 2G | 0 | 0 |
| Rice | 10G | 10G | 10G | 8G |
| Soybean | 6G | 1G | 0 | 0 |
| Cotton | 9G | 7G | 2G | 0 |
| Sugar beet | 10G | 9G | 3G | 0 |
| Crabgrass | 10G | 9G | 8G | 3G |
| Johnsongrass | 10G | 10G | 9G | 8G |
| Blackgrass | 10G | 10G | 8G | 7G |
| Barnyardgrass | 10G | 9G | 9G | 5G |
| Nutsedge | 10G | 9G | 7G | 3G |
| Giant Foxtail | - | - | - | - |
| Wild Oats | 4G | 0 | 0 | 0 |
| Cocklebur | 6G | 2G | 0 | 0 |
| Morningglory | 8G | 3G | 0 | 0 |
| Teaweed | 10G | 9G | 8G | 2G |
| Sicklepod | 9G | 7G | 4G | 0 |
| Jimsonweed | 10G | 9G | 7G | 2G |
| Velvetleaf | 10G | 8G | 5G | 2G |

Test C

Three 25-cm diameter plastic pots were filled with a light soil. One pan was planted to short rows of corn, soybeans, and cotton, planting depth 2.5 cm. The other two pots were planted to the following weed species, 8 or 9 species per pot: crabgrass (*Digitaria* sp.), johnsongrass (*Sorghum halepense*), barnyardgrass (*Echinochloa crusgalli*), nutsedge (*Cyperus rotundus*), giant foxtail (*Setaria faberii*), green foxtail (*Setaria viridis*), cocklebur (*Xanthium pensylvanicum*), morningglory (*Ipomoea hederacea*), teaweed (*Sida spinosa*), sicklepod (*Cassia obtusfolia*), jimsonweed (*Datura stramonium*), velvetleaf (*Abutilon theophrasti*), bindweed (*Convolvulus arvensis*), coffeeweed (*Sesbania exaltata*), lambsquarters

(*Chenopodium album*), pigweed (*Amaranthus retroflexus*), and purslane (*Portulaca oleracea*). All of the foregoing weed species were planted at a depth of 0.5 to 1.0 cm, except for cockbur and nutsedge which were planted at a depth of 2.5 cm. The plantings were treated preemergence with the test chemicals dissolved in a non-phytotoxic solvent. At the same time, the same crops and weed species, from plantings made 10 to 21 days previously, were treated with a soil/foliage application. Treated plants and controls were maintained in a greenhouse for 3 to 4 weeks after which all species were compared to controls and visually rated for response to treatment with 0 indicating no injury and 100 indicating that the plants were dead. The data are summarized in Table C.

Table C

Compound 13

| Rate gm/ha | 250 | 125 | 62 | 31 | 16 | 8 | 4 |
|---|---|---|---|---|---|---|---|
| POSTEMERGENCE | | | | | | | |
| Corn | 100 | 100 | 100 | 100 | 90 | 85 | 80 |
| Cotton | - | - | - | - | - | - | - |
| Soybean | 55 | 40 | 35 | 30 | 25 | 20 | 20 |
| Crabgrass | 100 | 100 | 95 | 85 | 75 | 65 | 40 |
| Johnsongrass | 100 | 100 | 100 | 100 | 100 | 100 | 80 |
| Barnyardgrass | 100 | 100 | 100 | 95 | 80 | 75 | 50 |
| Nutsedge | 100 | 95 | 80 | 75 | 55 | 35 | 30 |
| Giant foxtail | 100 | 100 | 100 | 95 | 85 | 80 | 70 |
| Green foxtail | 100 | 100 | 100 | 95 | 85 | 85 | 70 |
| Cocklebur | - | 100 | 100 | 85 | 85 | 65 | 40 |
| Morningglory | - | 95 | 90 | 80 | 75 | 55 | 35 |
| Teaweed | - | 90 | 85 | 80 | 80 | 60 | 40 |
| Sicklepod | - | 90 | 85 | 80 | 65 | 50 | 40 |
| Jimsonweed | - | 85 | 80 | 85 | 75 | 50 | 40 |
| Velvetleaf | 95 | 90 | 85 | 80 | 65 | 40 | 30 |
| Bindweed | 85 | 60 | 40 | 35 | 25 | 15 | 0 |
| Coffeeweed | 100 | 100 | 100 | 85 | 80 | 65 | 45 |
| Lambsquarter | 100 | 95 | 90 | 90 | 70 | 60 | 35 |
| Pigweed | 100 | 100 | 100 | 100 | 85 | 75 | 40 |
| Purslane | 100 | 100 | 95 | 85 | 75 | 65 | 50 |
| PREEMERGENCE | | | | | | | |
| Corn | 100 | 100 | 95 | 85 | 75 | 45 | 20 |
| Cotton | 100 | 100 | 85 | 75 | 35 | 15 | 0 |
| Soybean | 75 | 40 | 25 | 0 | 0 | 0 | 0 |
| Crabgrass | 100 | 100 | 100 | 100 | 85 | 60 | 20 |
| Johnsongrass | 100 | 100 | 100 | 100 | 100 | 95 | 75 |
| Barnyardgrass | 100 | 100 | 100 | 100 | 95 | 85 | 35 |
| Nutsedge | 100 | 100 | 100 | 100 | 95 | 85 | 45 |
| Giant foxtail | 100 | 100 | 100 | 95 | 90 | 30 | 0 |
| Green foxtail | 100 | 100 | 95 | 95 | 80 | 15 | 0 |
| Cocklebur | 100 | 100 | 85 | 55 | 35 | 25 | 0 |
| Morningglory | 100 | 100 | 95 | 90 | 85 | 35 | 0 |
| Teaweed | 100 | 100 | 100 | 85 | 75 | 45 | 15 |
| Sicklepod | 100 | 100 | 95 | 85 | 65 | 45 | 15 |
| Jimsonweed | 100 | 100 | 100 | 95 | 90 | 60 | 20 |
| Velvetleaf | 100 | 100 | 95 | 95 | 80 | 65 | 15 |
| Bindweed | 100 | 100 | 90 | 85 | 75 | 55 | 25 |
| Coffeeweed | 100 | 100 | 85 | 80 | 60 | 35 | 15 |
| Lambsquarter | 100 | 100 | 100 | 100 | 90 | 75 | 50 |
| Pigweed | 100 | 100 | 100 | 100 | 90 | 75 | 50 |
| Purslane | 100 | 100 | 100 | 100 | 100 | 80 | 50 |

Table C (continued)

Compound 14

| Rate gm/ha | 250 | 125 | 62 | 31 | 16 | 8 | 4 |
|---|---|---|---|---|---|---|---|
| POSTEMERGENCE | | | | | | | |
| Corn | 100 | 100 | 100 | 100 | 90 | 85 | 75 |
| Cotton | - | - | - | - | - | - | - |
| Soybean | 45 | 40 | 30 | 25 | 20 | 15 | 0 |
| Crabgrass | 100 | 95 | 95 | 85 | 75 | 50 | 35 |
| Johnsongrass | 100 | 100 | 100 | 100 | 100 | 95 | 85 |
| Barnyardgrass | 100 | 100 | 100 | 90 | 85 | 80 | 65 |
| Nutsedge | 95 | 85 | 80 | 75 | 50 | 20 | 0 |
| Giant foxtail | 100 | 100 | 95 | 90 | 85 | 80 | 70 |
| Green foxtail | 100 | 100 | 100 | 90 | 90 | 85 | 70 |
| Cocklebur | 100 | 90 | 85 | 70 | 65 | 40 | 20 |
| Morningglory | 95 | 85 | 80 | 70 | 70 | 50 | 30 |
| Teaweed | 100 | 90 | 90 | 65 | 60 | 50 | 35 |
| Sicklepod | 100 | 95 | 85 | 70 | 55 | 25 | 15 |
| Jimsonweed | 100 | 90 | 80 | 50 | 40 | 20 | 15 |
| Velvetleaf | 95 | 85 | 75 | 75 | 55 | 25 | 20 |
| Bindweed | 70 | 60 | 60 | 45 | 40 | 25 | 15 |
| Coffeeweed | 100 | 100 | 90 | 65 | 60 | 35 | 25 |
| Lambsquarter | 100 | 100 | 95 | 90 | 80 | 40 | 15 |
| Pigweed | 100 | 100 | 100 | 100 | 95 | 80 | 45 |
| Purslane | 100 | 100 | 100 | 85 | 85 | 40 | 35 |
| PREEMERGENCE | | | | | | | |
| Corn | 100 | 100 | 95 | 80 | 60 | 20 | 0 |
| Cotton | 100 | 90 | 70 | 25 | 15 | 0 | 0 |
| Soybean | 50 | 35 | 20 | 0 | 0 | 0 | 0 |
| Crabgrass | 100 | 100 | 100 | 100 | 85 | 75 | 55 |
| Johnsongrass | 100 | 100 | 100 | 100 | 100 | 90 | 85 |
| Barnyardgrass | 100 | 100 | 100 | 100 | 90 | 85 | 55 |
| Nutsedge | 100 | 100 | 100 | 95 | 75 | 45 | 25 |
| Giant foxtail | 100 | 100 | 100 | 90 | 85 | 80 | 20 |
| Green foxtail | 100 | 100 | 100 | 90 | 90 | 55 | 15 |
| Cocklebur | - | - | - | - | - | - | - |
| Morningglory | 100 | 100 | 100 | 85 | 60 | 20 | 0 |
| Teaweed | 100 | 100 | 95 | 85 | 55 | 25 | 15 |
| Sicklepod | 100 | 90 | 90 | 75 | 40 | 20 | 15 |
| Jimsonweed | 100 | 100 | 100 | 100 | 100 | 90 | 75 |
| Velvetleaf | 100 | 95 | 90 | 80 | 50 | 25 | 15 |
| Bindweed | 100 | 95 | 90 | 85 | 80 | 60 | 45 |
| Coffeeweed | 100 | 90 | 90 | 80 | 45 | 30 | 20 |
| Lambsquarter | 100 | 100 | 100 | 100 | 90 | 80 | 75 |
| Pigweed | 100 | 100 | 100 | 100 | 90 | 80 | 75 |
| Purslane | 100 | 100 | 100 | 100 | 100 | 85 | 80 |

Test D

Two plastic pans lined with polyethylene liners were filled with prepared Woodstown sandy loam soil. One pan was planted with seeds of wheat (*Triticum aestivum*), barley (*Hordeum vulgare*), wild oats (*Avena fatua*), cheatgrass (*Bromus secalinus*), blackgrass (*Alopecurus myosuroides*), annual bluegrass (*Poa annua*), green foxtail (*Setaria viridis*), Italian ryegrass (*Lolium multiflorum*) and rapeseed (*Brassica napus*). The other pan was planted with seeds of Russian thistle (*Salsola kali*), cleavers (*Galium aparine*), speedwell (*Veronica persica*), kochia (*Kochia scoparia*), shepherdspurse (*Capsella bursa-pastoris*), *Matricaria inodora*, black nightshade (*Solanum nigrum*), wild buckwheat (*Polygonum convolvulus*) and sugar beets (*Beta*

*vulgaris*). The above two pans were treated preemergence. At the same time two pans in which the above plant species were already growing were treated postemergence. Plant height at the time of treatment ranged from 1—20 cm depending on plant species.

The compounds applied were diluted with a non-phytotoxic solvent and sprayed over-the-top of the pans. An untreated control and a solvent alone control were included for comparison. All treatments were maintained in the greenhouse for 19—22 days at which time the treatments were compared to the controls and the effects visually rated utilizing the same rating system as described for Test A. The recorded data are presented in Table D.

Table D

Compound 13

| Rate kg/ha | .125 | .06 | .03 | .015 | .008 | .004 | .002 | .001 |
|---|---|---|---|---|---|---|---|---|
| POSTEMERGENCE | | | | | | | | |
| wheat | 2G | 0 | 0 | 0 | 0 | 0 | 0 | 0 |
| barley | 8G | 5G | 4G | 3G | 2G | 0 | 0 | 0 |
| wild oats | 0 | 0 | 0 | 0 | 0 | 0 | 0 | 0 |
| cheatgrass | 6G | 5G | 3G | 0 | 0 | 0 | 0 | 0 |
| blackgrass | 10C | 9G | 7G | 5G | 3G | 0 | 0 | - |
| annual bluegrass | 10C | 9G | 9G | 4G | 4G | 0 | 0 | - |
| green foxtail | 10C | 10C | 10C | 10C | 8G | 6G | 4G | - |
| Italian ryegrass | 10C | 8G | 8G | 5G | 3G | 2G | 2G | - |
| rapeseed | 10C | 10C | 10C | 10C | 10C | 9G | 7G | - |
| *Matricaria inodora* | - | - | - | - | - | - | - | - |
| Galium aparine | - | - | - | - | - | - | - | - |
| Russian thistle | - | - | - | - | - | - | - | - |
| shepherdspurse | - | - | - | - | - | - | - | - |
| kochia | - | - | - | - | - | - | - | - |
| black nightshade | - | - | - | - | - | - | - | - |
| speedwell | 10C | 10C | 10C | 3G | 0 | 0 | 0 | 0 |
| wild buckwheat | 10C | 5G | 2G | 0 | - | 2G | 2G | 0 |
| sugar beets | 10C | 9G | 9G | 4G | 5G | 3G | 3G | 3G |
| PREEMERGENCE | | | | | | | | |
| wheat | 0 | 0 | 0 | 0 | 0 | 0 | 0 | 0 |
| barley | 6G | 0 | 2G | 0 | 0 | 0 | 0 | 0 |
| wild oats | 0 | 0 | 0 | 0 | 0 | 0 | 0 | 0 |
| cheatgrass | 5G | 2G | 0 | 0 | 0 | 0 | 0 | 0 |
| blackgrass | 8G | 8G | 8G | 8G | 7G | 3G | 2G | 0 |
| annual bluegrass | 9G | 9G | 9G | 8G | 8G | 7G | 5G | 3G |
| green foxtail | 9G | 8G | 5G | 2G | 0 | 0 | 0 | 0 |
| Italian ryegrass | 8G | 8G | 7G | 4G | 4G | 3G | 3G | 0 |
| rapeseed | 9G | 9G | 9G | 8G | 7G | 4G | 4G | 2G |
| *Matricaria inodora* | 9G | 9G | 8G | 8G | 8G | 6G | 6G | 5G |
| Galium aparine | 10C | 9G | 9G | 5G | 5G | 4G | 4G | 0 |
| Russian thistle | 8G | 8G | 3G | 2G | 2G | 2G | 0 | 0 |
| shepherdspurse | 10C | 10C | 9G | 9G | 9G | 7G | 6G | 0 |
| kochia | 10C | 10C | 10C | 10C | 8G | 8G | 4G | 4G |
| black nightshade | 9G | 9G | 9G | 8G | 8G | 6G | 3G | 0 |
| speedwell | 10C | 10C | 10C | 10C | 9G | 7G | 5G | 0 |
| wild buckwheat | 8G | 8G | 7G | 2G | 0 | 0 | 0 | 0 |
| sugar beets | 10C | 10C | 10C | 10C | 8G | 8G | 6G | 6G |

Table D (continued)

Compound 13

| Rate kg/ha | .125 | .06 | .03 | .015 | .008 | .004 | .002 | .001 |
|---|---|---|---|---|---|---|---|---|
| POSTEMERGENCE | | | | | | | | |
| wheat | 0 | 0 | 0 | 0 | 0 | 0 | 0 | 0 |
| barley | 6G | 5G | 4G | 4G | 5G | 2G | 2G | 0 |
| wild oats | 0 | 0 | 0 | 0 | 0 | 0 | 0 | 0 |
| cheatgrass | 6G | 4G | 4G | 2G | 0 | 0 | 0 | 0 |
| blackgrass | 9G | 9G | 8G | 5G | 3G | 0 | 0 | - |
| annual bluegrass | 10C | 9G | 8G | 5G | 2G | 2G | 0 | - |
| green foxtail | 10C | 10C | 10C | 10C | 8G | 7G | 5G | - |
| Italian ryegrass | 9G | 6G | 5G | 3G | 2G | 0 | 0 | - |
| rapeseed | 10C | 10C | 10C | 10C | 9G | 8G | 5G | - |
| *Matricaria inodora* | - | - | - | - | - | - | - | - |
| Galium aparine | - | - | - | - | - | - | - | - |
| Russian thistle | - | - | - | - | - | - | - | - |
| shepherdspurse | - | - | - | - | - | - | - | - |
| kochia | - | - | - | - | - | - | - | - |
| black nightshade | - | - | - | - | - | - | - | - |
| speedwell | 7G | 0 | 0 | 0 | 0 | 0 | 0 | 0 |
| wild buckwheat | 7G | 2G | 2G | - | 0 | 0 | 0 | 0 |
| sugar beets | 10C | 10C | 10C | 4G | 3G | 3G | 0 | 0 |
| PREEMERGENCE | | | | | | | | |
| wheat | 0 | 0 | 0 | 0 | 0 | 0 | 0 | 0 |
| barley | 8G | 7G | 7G | 7G | 3G | 3G | 0 | 0 |
| wild oats | 0 | 0 | 0 | 0 | 0 | 0 | 0 | 0 |
| cheatgrass | 7G | 3G | 0 | 0 | 0 | 0 | 0 | 0 |
| blackgrass | 9G | 8G | 8G | 7G | 6G | 4G | 2G | 1G |
| annual bluegrass | 9G | 9G | 9G | 8G | 5G | 5G | 3G | 2G |
| green foxtail | 9G | 7G | 7G | 6G | 4G | 2G | 0 | 0 |
| Italian ryegrass | 8G | 7G | 6G | 5G | 4G | 2G | 2G | 2G |
| rapeseed | 9G | 9G | 9G | 7G | 5G | 2G | 2G | 2G |
| *Matricaria inodora* | 8G | 7G | 7G | 7G | 6G | 5G | 4G | 3G |
| Galium aparine | 9G | 8G | 9G | 6G | 5G | 0 | 0 | 0 |
| Russian thistle | 6G | 6G | 3G | 0 | 0 | 0 | 0 | 0 |
| shepherdspurse | 10C | 10C | 9G | 9G | 8G | 6G | 5G | 3G |
| kochia | 10C | 10C | 10C | 9G | 7G | 6G | 6G | 3G |
| black nightshade | 9G | 9G | 9G | 8G | 8G | 8G | 6G | 4G |
| speedwell | 9G | 6G | 3G | 0 | 0 | 0 | 0 | 0 |
| wild buckwheat | 8G | 5G | - | 0 | 0 | 0 | 0 | 0 |
| sugar beets | 9G | 9G | 9G | 9G | 7G | 8G | 5G | 5G |

**Claims**

1. A compound of the formula

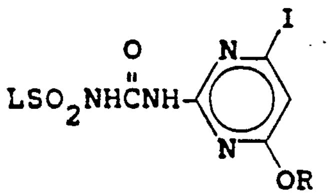

or

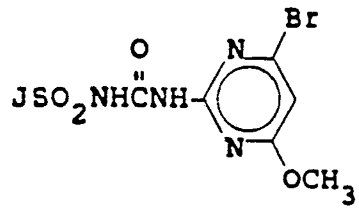

I

Ia

wherein

R is $CH_3$ or $CH_2CH_3$:

L is 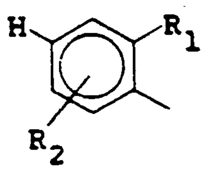 , 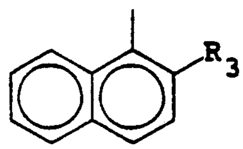 , 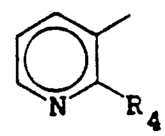 ,

L-1 L-2 L-3

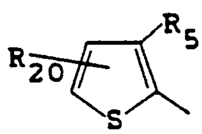 , 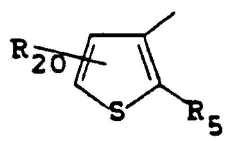 , 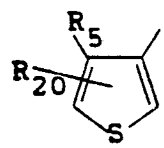 ,

L-4 L-5 L-6

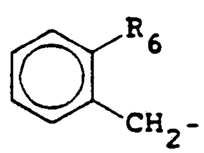 , 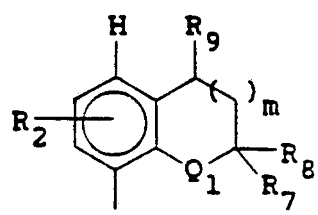 ,

L-7 L-8

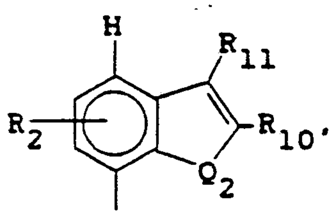 , 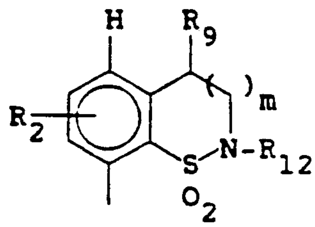 ,

L-9 L-10

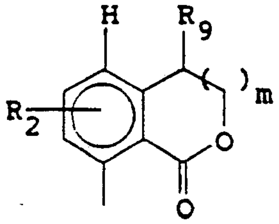,

L-11

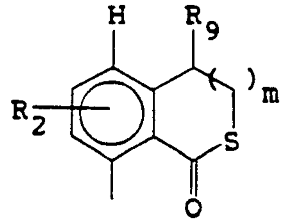,

L-12

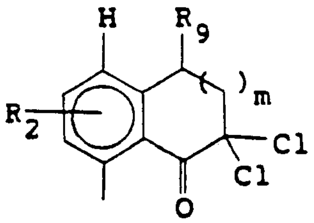,

L-13

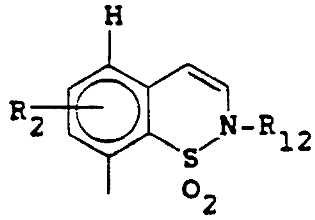,

L-14

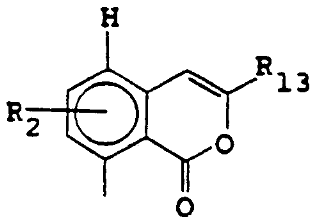,

L-15

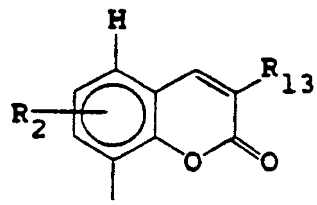

L-16

or 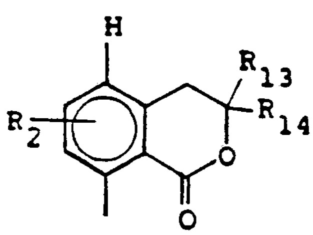 ;

L-17

J is 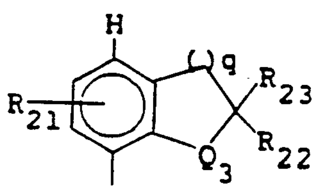 or 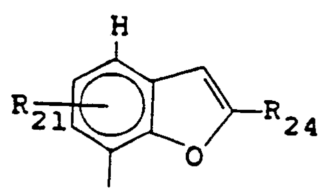 ;

J-1

J-2

$R_1$ is $C_1$—$C_4$ akyl, $C_1$—$C_4$ alkoxy, $OCH_2CH_2OCH_3$, F, Cl, Br, $NO_2$, $CF_3$, $CO_2R_{15}$, $SO_2NR_{16}R_{17}$, $C(O)R_{25}$, $CR_{25}(OR_{26})$, $SO_2N(OCH_3)CH_3$, $OSO_2R_{18}$, $S(O)_nR_{19}$, $WCF_3$, $WCHF_2$, $C_3$—$C_4$ alkenyloxy, $C_3$—$C_4$ alkynyloxy, $C_1$—$C_2$ alkyl substituted with $OCH_3$ or $OCH_2CH_3$, $C_6H_5$,

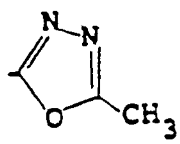 , 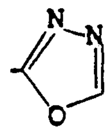 , 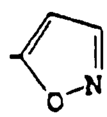 , 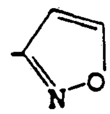 , 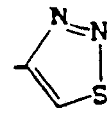 ,

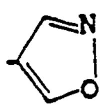 , 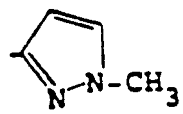 , 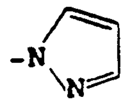 , 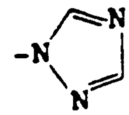 , 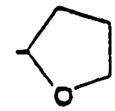 ,

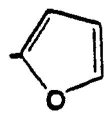 , 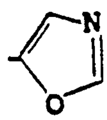 , 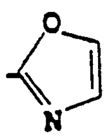 , 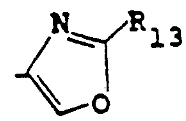 , 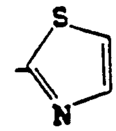 ,

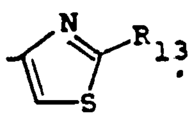 , 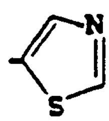 , 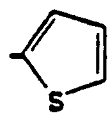 , 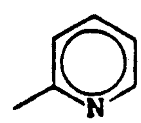 or 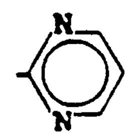 ;

$R_2$ is H, F, Cl, Br, $C_1$—$C_4$ alkyl, $C_1$—$C_4$ alkoxy, $C_3$—$C_4$ alkenyloxy, $C_3$—$C_4$ alkinyloxy, $C_1$—$C_4$ haloalkoxy, $C_1$—$C_4$ haloalkyl, $S(O)_pC_1$—$C_4$ alkyl, $S(O)_pC_3$—$C_4$ alkenyl $S(O)_pC_3$—$C_4$ alkynyl or $C_1$—$C_2$ alkyl substituted with $OCH_3$ or $SCH_3$;

$R_3$ is H, $CH_3$, $OCH_3$, F, Cl, Br, $SO_2N(CH_3)_2$, $OSO_2CH_3$ or $S(O)_nCH_3$;

$R_4$ is $CH_3$, $CH_2CH_3$, $OCH_3$, $OCH_2CH_3$, F, Cl, Br, $SO_2NR_{16}R_{17}$, $SO_2N(OCH_3)CH_3$, $S(O)_nR_{19}$, $C_3$—$C_4$ alkenyloxy or $C_3$—$C_4$ alkynyloxy;

$R_5$ is $C_1$—$C_3$ alkyl, F, Cl, Br, $NO_2$, $CO_2R_{15}$, $SO_2NR_{16}R_{17}$, $SO_2N(OCH_3)CH_3$ or $S(O)_nR_{19}$;

$R_6$ is Cl, $NO_2$, $CO_2CH_3$, $CO_2CH_2CH_3$, $SO_2N(CH_3)_2$, $OSO_2CH_3$, $SO_2CH_3$, $SO_2CH_2CH_3$, $OCH_3$ or $OCH_2CH_3$;

$R_7$ is H, $CH_3$ or $CH_2CH_3$;

$R_8$ is H, $CH_3$ or $CH_2CH_3$;

$R_9$ is H or $CH_3$;

$R_{10}$ is H or $CH_3$;

$R_{11}$ is H or $CH_3$;

$R_{12}$ is H, $C_1$—$C_4$ alkyl, $C_3$—$C_4$ alkenyl, $C_3$—$C_4$ alkynyl, benzyl, $C_1$—$C_4$ alkylcarbonyl, $C_1$—$C_4$ alkoxycarbonyl, $CH_2CN$, $CH_2C(O)CH_3$, $CH_2CO_2(C_1$—$C_2$ alkyl), $C_1$—$C_4$ alkyl substituted with 0—3 F, 0—1 Cl, OH, $OCH_3$ or $OC_2H_5$ or $C_3$—$C_4$ alkenyl substituted with 1—3 F or 1—3 Cl;

$R_{13}$ is H or $CH_3$;

$R_{14}$ is $CH_3$ or $C_2H_5$;

$R_{15}$ is $C_1$—$C_4$ alkyl, $CH_2CH_2OCH_3$, $CH_2CH_2Cl$ or $CH_2CH{=}CH_2$;

$R_{16}$ is H, $C_1$—$C_3$ alkyl;

$R_{17}$ is $C_1$—$C_3$ alkyl;

$R_{18}$ is $C_1$—$C_3$ alkyl or $N(CH_3)_2$;

$R_{19}$ is $C_1$—$C_3$ alkyl or $CH_2CH{=}CH_2$;

$R_{20}$ is H, Cl or $CH_3$;

$R_{21}$ is H, Cl, $CH_3$ or $OCH_3$;

$R_{22}$ is H or $C_1$—$C_4$ alkyl;

$R_{23}$ is H or $CH_3$;

$R_{24}$ is H or $CH_3$;

$R_{25}$ is H, $C_1$—$C_4$ alkyl, $C_3$—$C_4$ alkenyl, $C_3$—$C_4$ alkynyl or $C_3$—$C_6$ cycloalkyl;

$R_{26}$ is $C_1$—$C_2$ alkyl;

m is 0 or 1;

n is 0 or 2;

p is 0, 1 or 2;

q is 1 or 2;

$Q_1$ is O, S or $SO_2$;

$Q_2$ is O or S;
$Q_3$ is O, S, SO or $SO_2$; and
W is O, S or $SO_2$;

provided that

1) the total number of carbon atoms of $R_{16}$ and $R_{17}$ is less than or equal to four;
2) when m is 1, then $R_9$ is H; and
3) when $R_{23}$ is $CH_3$, then $Q_3$ is $SO_2$ and $R_{22}$ is $CH_3$;

and their agriculturally suitable salts.

2. A compound of Claim 1 where R is $CH_3$.

3. A compound of Claim 2 where L is L-1, L-2, L-3, L-5, L-8, L-10, L-11, L-16 or L-17.

4. A compound of Claim 3 where L is L-1; $R_1$ is $OCH_3$, $OC_2H_5$, Cl, $NO_2$, $CF_3$, $CO_2CH_3$, $CO_2C_2H_5$, $SO_2N(CH_3)_2$, $SO_2N(OCH_3)CH_3$, $OSO_2R_{18}$, $C(O)CH_3$, $C(O)C_2H_5$, $S(O)_nR_{19}$, $OCF_2H$, $SCF_2H$, $OCH_2CH{=}CH_2$, $OCH_2C{\equiv}CH$,

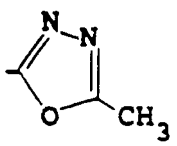 , 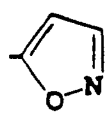 , 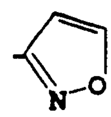 or 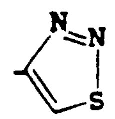 ;

$R_2$ is H, F, Cl, Br, $CH_3$, $C_2H_5$, $S(O)_pC_1$—$C_2$ alkyl, $S(O)_pCH_2CH{=}CH_2$, $S(O)_pCH_2C{\equiv}CH$, $C_1$—$C_3$ alkoxy, $OCH_2CH{=}CH_2$, $OCH_2{\equiv}CH$, $C_1$—$C_2$ haloalkoxy, $C_1$—$C_2$ haloalkyl, $CH_2OCH_3$ or $CH_2SCH_3$; $R_{18}$ is $C_1$—$C_3$ alkyl; $R_{19}$ is $CH_3$ or $C_2H_5$; and n is 2.

5. A compound of Claim 4 where

L is 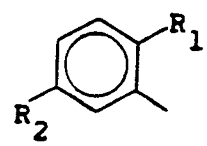 ;

and $R_2$ is H, F, Cl, Br, $CH_3$, $CH_2CH_3$, $SCH_3$, $SC_2H_5$, $OCH_3$, $OC_2H_5$, $CF_3$, $CH_2OCH_3$ or $CH_2SCH_3$.

6. A compound of Claim 3 where L is L-2; and $R_3$ is Cl, $CH_3$, $OCH_3$, $SCH_3$ or Br.

7. A compound of Claim 3 where L is L-3; and $R_4$ is Cl, $SO_2CH_3$ or $SO_2N(CH_3)_2$.

8. A compound of Claim 3 where L is L-5; $R_5$ is $CO_2CH_3$ or $CO_2C_2H_5$; and $R_{20}$ is H.

9. A compound of Claim 3 where L is L-8; m is 0; $R_2$ is H, F, Cl, Br, $CH_3$, $CH_2CH_3$, $SCH_3$, $SC_2H_5$, $OCH_3$, $OC_2H_5$, $CF_3$, $CH_2OCH_3$ or $CH_2SCH_3$; and $R_9$ is H.

10. A compound of Claim 3 wherein L is L-10; $R_2$ is H, F, Cl, Br, $CH_3$, $CH_2CH_3$, $SCH_3$, $SC_2H_5$, $OCH_3$, $OC_2H_5$, $CF_3$, $CH_2OCH_3$ or $CH_2SCH_3$; $R_9$ is H; and $R_{12}$ is H, $C_1$—$C_4$ alkyl, $C_1$—$C_4$ alkylcarbonyl, $CH_2F$ or $C_2$—$C_3$ alkyl substituted with 1F or 1Cl.

11. A compound of Claim 3 where L is L-11; $R_2$ is H, F, Cl, Br, $CH_3$, $CH_2CH_3$, $SCH_3$, $SC_2H_5$, $OCH_3$, $OC_2H_5$, $CF_3$, $CH_2OCH_3$ or $CH_2SCH_3$; and $R_9$ is H.

12. A compound of Claim 3 where L is L-16; and $R_2$ is H, F, Cl, Br, $CH_3$, $CH_2CH_3$, $SCH_3$, $SC_2H_5$, $OCH_3$, $OC_2H_5$, $CF_3$, $CH_2OCH_3$ or $CH_2SCH_3$.

13. A compound of Claim 3 where L is L-17; and $R_2$ is H, F, Cl, Br, $CH_3$, $CH_2CH_3$, $SCH_3$, $SC_2H_5$, $OCH_3$, $OC_2H_5$, $CF_3$, $CH_2OCH_3$ or $CH_2SCH_3$.

14. A compound of Claim 1 which is 2-[[(4-iodo-6-methoxypyrimidin-2-yl)aminocarbonyl]aminosulfonyl]benzoic acid, methyl ester.

15. A compound of Claim 1 where J is J-1, $R_{22}$ is H, $CH_3$ or $C_2H_5$ and $R_{23}$ is H.

16. A compound of Claim 1 which is N'-[(4-iodo-6-methoxypyrimidin-2-yl)aminocarbonyl]-N,N-dimethyl-1,2-benzenedisulfonamide.

17. A compound of Claim 1 which is 2-[[(4-iodo-6-methoxypyrimidin-2-yl)aminocarbonyl]aminosulfonyl)-4-methylbenzoic acid, methyl ester.

18. A compound of Claim 1 which is N-[(4-bromo-6-methoxypyrimidin-2-yl)aminocarbonyl]-2,3-dihydrobenzo[b]thiophene-7-sulfonamide, 1,1-dioxide.

19. A compound of Claim 1 which is N-[(4-bromo-6-methoxypyrimidin-2-yl)aminocarbonyl]-2,3-dihydro-2-methylbenzo[b]thiophene-7-sulfonamide, 1,1-dioxide.

20. A composition suitable for controlling the growth of undesired vegetation which comprises an effective amount of a compound of any of claims 1 to 19 and at least one of the following: surfactant, solid or liquid diluent.

21. A method for controlling the growth of undesired vegetation which comprises applying to the locus to be protected an effective amount of the compound of any of claims 1 to 19.

22. A method for regulating the growth of plants which comprises applying to the locus of such plants an effective but substantially non-phytotoxic amount of a plant growth regulant selected from compounds of any of claims 1 to 19.

23. A process for the production of a compound of claim 1 which comprises:
(a) reacting an appropriately substituted sulfonyl isocyanate of formula

$LSO_2NCO$ or $JSO_2NCO$

(II) (IIa)

with an aminoheterocycle of formula

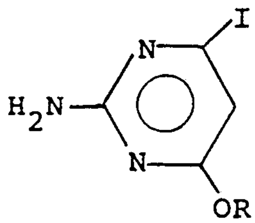

(III)

or

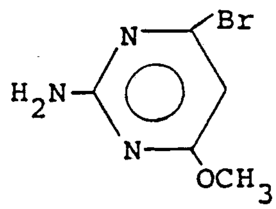

(IIIa)

respectively, wherein L, J and R are as defined in claim 1; or
(b) reacting a phenylcarbamate of formula

$LSO_2NHC(=O)OC_6H_5$ or $JSO_2NHC(=O)OC_6H_5$

(VI) (VIa)

with said aminoheterocycle of formula (III) or (IIIa) respectively; wherein L and J are as defined in claim 1; or
(c) reacting a sulfonamide of formula

$LSO_2NH_2$ or $JSO_2NH_2$

(IV) (IVa)

with a heterocyclic phenylcarbamate of formula

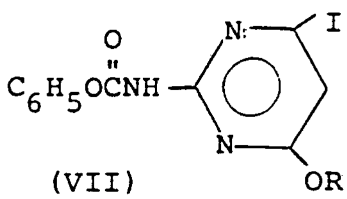

(VII)

or

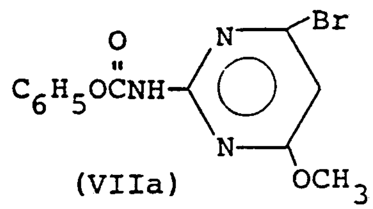

(VIIa)

respectively wherein L, J and R are as defined in claim 1.

24. Iodopyrimidines of formula

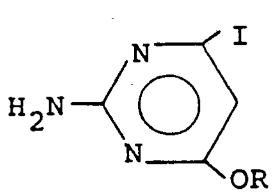

and phenylcarbamates thereof; wherein R is $CH_3$ or $C_2H_5$.

**Patentansprüche**

1. Verbindung der Formel

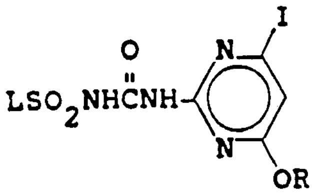

I

oder

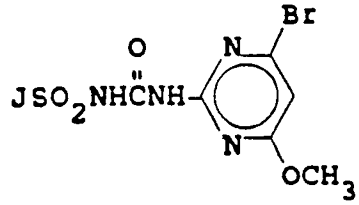

Ia

worin

R $CH_3$ oder $CH_2CH_3$ ist;

L is

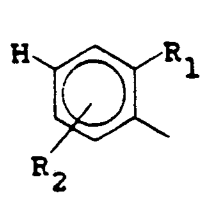

L-1

,

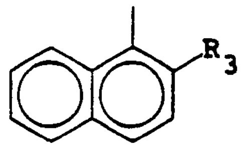

L-2

,

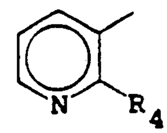

L-3

,

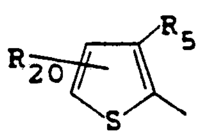

L-4

,

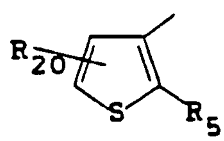

L-5

,

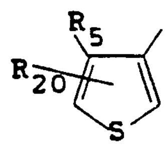

L-6

,

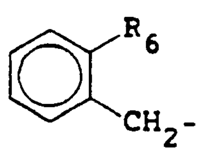

L-7

,

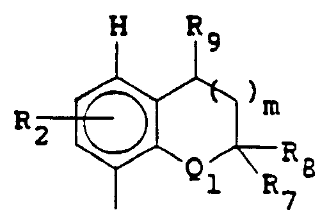

L-8

,

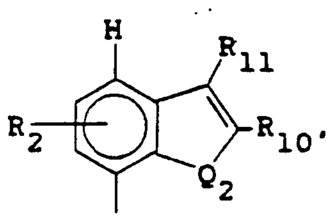

L-9

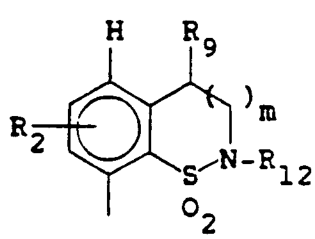

L-10

,

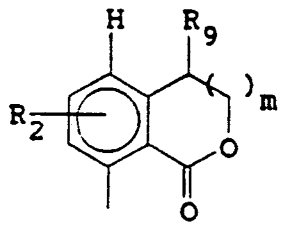 ,
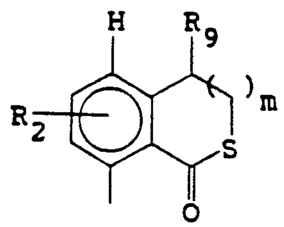 ,

L-11 L-12

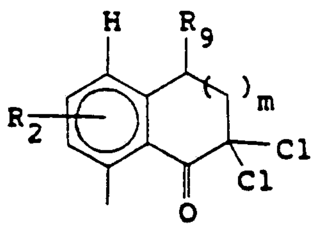 ,
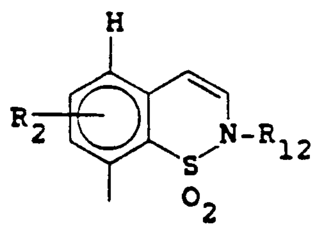 ,

L-13 L-14

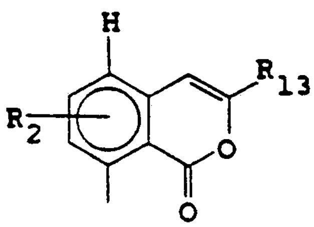 ,
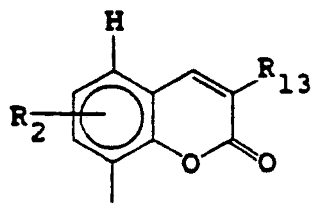

L-15 L-16

oder 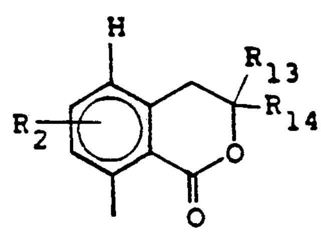 ist;

L-17

J is 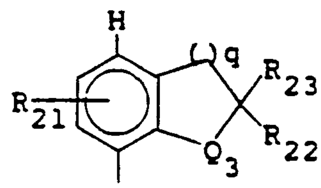 oder 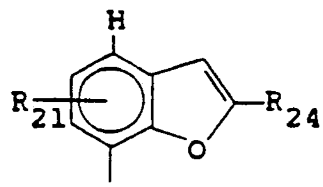 ist;

J-1 J-2

$R_1$ $C_1$—$C_4$-Alkyl, $C_1$—$C_4$-Alkoxy, $OCH_2CH_2OCH_3$, F, Cl, Br, $NO_2$, $CF_3$, $CO_2R_{15}$, $SO_2NR_{16}R_{17}$, $C(O)R_{25}$, $CR_{25}(OR_{26})$, $SO_2N(OCH_3)CH_3$, $OSO_2R_{18}$, $S(O)_nR_{19}$, $WCF_3$, $WCHF_2$, $C_3$—$C_4$-Alkenyloxy, $C_3$—$C_4$-Alkinyloxy, mit $OCH_3$ oder $OCH_2CH_3$ substituiertes $C_1$—$C_2$-Alkyl, $C_6H_5$,

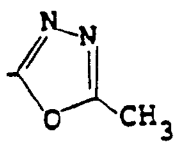 , 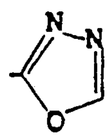 , 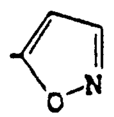 , 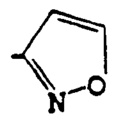 , 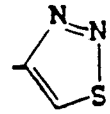 ,

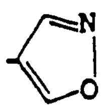 , 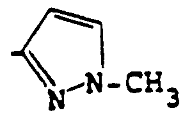 , 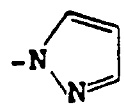 , 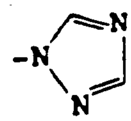 , 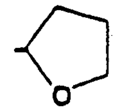 ,

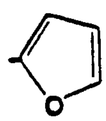 , 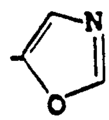 , 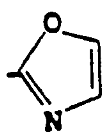 , 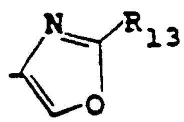 , 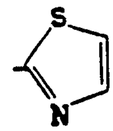 ,

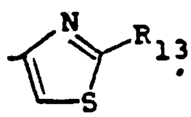 , 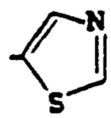 , 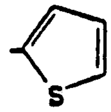 , 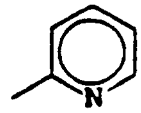 oder 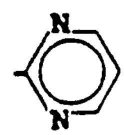 ist;

$R_2$ H, Cl, Br, $C_1$—$C_4$-Alkyl, $C_1$—$C_4$-Alkoxy, $C_3$—$C_4$-Alkenyloxy, $C_3$—$C_4$-Alkinyloxy, $C_1$—$C_4$-Halogenalkoxy, $C_1$—$C_4$-Halogenalkyl, $S(O)_pC_1$—$C_4$-Alkyl, $S(O)_pC_3$—$C_4$-Alkenyl, $S(O)_pC_3$—$C_4$-Alkinyl oder mit $OCH_3$ oder $SCH_3$ substituiertes $C_1$—$C_2$-Alkyl ist;

$R_3$ H, $CH_3$, $OCH_3$, F, Cl, Br, $SO_2N(CH_3)_2$, $OSO_2CH_3$ oder $S(O)_nCH_3$ ist;

$R_4$ $CH_3$, $CH_2CH_3$, $OCH_3$, $OCH_2CH_3$, F, Cl, Br, $SO_2NR_{16}R_{17}$, $SO_2N(OCH_3)CH_3$, $S(O)_nR_{19}$, $C_3$—$C_4$-Alkenyloxy oder $C_3$—$C_4$-Alkinyloxy ist;

$R_5$ $C_1$—$C_3$-Alkyl, F, Cl, Br, $NO_2$, $CO_2R_{15}$, $SO_2NR_{16}R_{17}$, $SO_2N(OCH_3)CH_3$ oder $S(O)_nR_{19}$ ist;

$R_6$ Cl, $NO_2$, $CO_2CH_3$, $CO_2CH_2CH_3$, $SO_2N(CH_3)_2$, $OSO_2CH_3$, $SO_2CH_3$, $SO_2CH_2CH_3$, $OCH_3$ oder $OCH_2CH_3$ ist;

$R_7$ H, $CH_3$ oder $CH_2CH_3$ ist;

$R_8$ H, $CH_3$ oder $CH_2CH_3$ ist;

$R_9$ H oder $CH_3$ ist;

$R_{10}$ H oder $CH_3$ ist;

$R_{11}$ H oder $CH_3$ ist;

$R_{12}$ H, $C_1$—$C_4$-Alkyl, $C_3$—$C_4$-Alkenyl, $C_3$—$C_4$-Alkinyl, Benzyl, $C_1$—$C_4$-Alkylcarbonyl, $C_1$—$C_4$-Alkoxycarbonyl, $CH_2CN$, $CH_2C(O)CH_3$, $CH_2CO_2(C_1$—$C_2$-Alkyl), mit 0—3 F, 0—1 Cl, OH, $OCH_3$ oder $OC_2H_5$ substituiertes $C_1$—$C_4$-Alkyl oder mit 1—3 F oder 1—3 Cl substituiertes Alkenyl ist;

$R_{13}$ H oder $CH_3$ ist;

$R_{14}$ $CH_3$ oder $C_2H_5$ ist;

$R_{15}$ $C_1$—$C_4$-Alkyl, $CH_2CH_2OCH_3$, $CH_2CH_2Cl$ oder $CH_2CH{=}CH_2$ ist;

$R_{16}$ H, $C_1$—$C_3$-Alkyl ist;

$R_{17}$ $C_1$—$C_3$-Alkyl ist;

$R_{18}$ $C_1$—$C_3$-Alkyl oder $N(CH_3)_2$ ist;

$R_{19}$ $C_1$—$C_3$-Alkyl oder $CH_2CH{=}CH_2$ ist;

$R_{20}$ H, Cl oder $CH_3$ ist;

$R_{21}$ H, Cl, $CH_3$ oder $OCH_3$ ist;

$R_{22}$ H oder $C_1$—$C_4$-Alkyl ist;

$R_{23}$ H oder $CH_3$ ist;

$R_{24}$ H oder $CH_3$ ist;

$R_{25}$ H, $C_1$—$C_4$-Alkyl, $C_3$—$C_4$-Alkenyl, $C_3$—$C_4$-Alkinyl oder $C_3$—$C_6$-Cycloalkyl ist;

$R_{26}$ $C_1$—$C_2$-Alkyl ist;

m 0 oder 1 ist;

n 0 oder 2 ist;

p 0, 1 oder 2 ist;

q 1 oder 2 ist;

$Q_1$ O, S oder $SO_2$ ist;

$Q_2$ O oder S ist;
$Q_3$ O, S, SO oder $SO_2$ ist; und
W O, S oder $SO_2$ ist;
mit der Maßgabe, daß
1) die Gesamtzahl der Kohlenstoffatome von $R_{16}$ und $R_{17}$ weniger oder gleich 4 ist;
2) wenn m 1 ist, dann $R_9$ H ist; und
3) wenn $R_{23}$ $CH_3$ ist, dann $Q_3$ $SO_2$ ist und $R_{22}$ $CH_3$ ist;
und ihre landwirtschaftlich geeigneten Salze.

2. Verbindung nach Anspruch 1, worin R $CH_3$ ist.

3. Verbindung nach Anspruch 2, worin L L-1, L-2, L-3, L-5, L-8, L-10, L-11, L-16 oder L-17 ist.

4. Verbindung nach Anspruch 3, worin L L-1 ist; $R_1$ $OCH_3$, $OC_2H_5$, Cl, $NO_2$, $CF_3$, $CO_2CH_3$, $CO_2C_2H_5$, $SO_2N(CH_3)_2$, $SO_2N(OCH_3)CH_3$, $OSO_2R_{18}$, $C(O)CH_3$, $C(O)C_2H_5$, $S(O)_nR_{19}$, $OCF_2H$, $SCF_2H$, $OCH_2CH{\equiv}CH_2$, $OCH_2C{=}CH$,

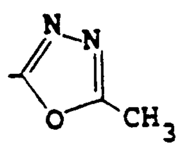 , 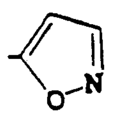 , 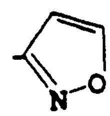 oder 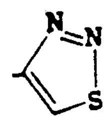 ist;

$R_2$ H, F, Cl, Br, $CH_3$, $C_2H_5$, $S(O)_pC_1$—$C_2$-Alkyl, $S(O)_pCH_2CH{=}CH_2$, $S(O)_pCH_2C{\equiv}CH$, $C_1$—$C_3$-Alkoxy, $OCH_2CH{=}CH_2$, $OCH_2{\equiv}CH$, $C_1$—$C_2$-Halogenalkoxy, $C_1$—$C_2$-Halogenalkyl, $CH_2OCH_3$ oder $CH_2SCH_3$ ist; $R_{18}$ $C_1$—$C_3$-Alkyl ist; $R_{19}$ $CH_3$ oder $C_2H_5$ ist; und n 2 ist.

5. Verbindung nach Anspruch 4, worin L

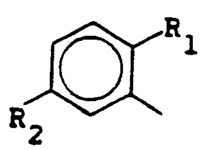 ist;

und $R_2$ H, F, Cl, Br, $CH_3$, $CH_2CH_3$, $SCH_3$, $SC_2H_5$, $OCH_3$, $OC_2H_5$, $CF_3$, $CH_2OCH_3$ oder $CH_2SCH_3$ ist.

6. Verbindung nach Anspruch 3, worin L L-2 ist und $R_3$ Cl, $CH_3$, $OCH_3$, $SCH_3$ oder Br ist.

7. Verbindung nach Anspruch 3, worin L L-3 ist; und $R_4$ Cl, $SO_2CH_3$ oder $SO_2N(CH_3)_2$ ist.

8. Verbindung nach Anspruch 3, worin L L-5 ist; $R_5$ $CO_2CH_3$ oder $CO_2C_2H_5$ ist; und $R_{20}$ H ist.

9. Verbindung nach Anspruch 3, worin L L-8 ist; m 0 ist; $R_2$ H, F, Cl, Br, $CH_3$, $CH_2CH_3$, $SCH_3$, $SC_2H_5$, $OCH_3$, $OC_2H_5$, $CF_3$, $CH_2OCH_3$ oder $CH_2SCH_3$ ist; und $R_9$ H ist.

10. Verbindung nach Anspruch 3, worin L-L-10 ist; $R_2$ H, F, Cl, Br, $CH_3$, $CH_2CH_3$, $SCH_3$, $SC_2H_5$, $OCH_3$, $OC_2H_5$, $CF_3$, $CH_2OCH_3$ oder $CH_2SCH_3$ ist; $R_9$ H ist; und $R_{12}$ H, $C_1$—$C_4$-Alkyl, $C_1$—$C_4$-Alkylcarbonyl, $CH_2F$ oder mit 1 F oder 1 Cl substituiertes $C_2$—$C_3$-Alkyl ist.

11. Verbindung nach Anspruch 3, worin L L-11 ist; $R_2$ H, F, Cl, Br, $CH_3$, $CH_2CH_3$, $SCH_3$, $SC_2H_5$, $OCH_3$, $OC_2H_5$, $CF_3$, $CH_2OCH_3$ oder $CH_2SCH_3$ ist; und $R_9$ H ist.

12. Verbindung nach Anspruch 3, worin L L-16 ist; und $R_2$ H, F, Cl, Br, $CH_3$, $CH_2CH_3$, $SCH_3$, $SC_2H_5$, $OCH_3$, $OC_2H_5$, $CF_3$, $CH_2OCH_3$ oder $CH_2SCH_3$ ist.

13. Verbindung nach Anspruch 3, worin L L-17 ist; und $R_2$ H, F, Cl, Br, $CH_3$, $CH_2CH_3$, $SCH_3$, $SC_2H_5$, $OCH_3$, $OC_2H_5$, $CF_3$, $CH_2OCH_3$ oder $CH_2SCH_3$ ist.

14. Verbindung nach Anspruch 1, welche 2-[[(4-Jod-6-methoxypyrimidin-2-yl)aminocarbonyl]aminosulfonyl]benzoesäure-methylester ist.

15. Verbindung nach Anspruch 1, worin J J-1 ist, $R_{22}$ H, $CH_3$ oder $C_2H_5$ ist und $R_{23}$ H ist.

16. Verbindung nach Anspruch 1, welche N'-[(4-Jod-6-methoxypyrimidin-2-yl)aminocarbonyl]-N,N-dimethyl-1,2-benzoldisulfonamid ist.

17. Verbindung nach Anspruch 1, welche 2-[[(4-Jod-6-methoxypyrimidin-2-yl)aminocarbonyl]aminosulfonyl]-4-methylbenzoesäure-methylester ist.

18. Verbindung nach Anspruch 1, welche N-[(4-Brom-6-methoxypyrimidin-2-yl)aminocarbonyl]-2,3-dihydrobenzo[b]thiophen-7-sulfonamid-1,1-dioxid ist.

19. Verbindung nach Anspruch 1, welche N-[(4-Brom-6-methoxypyrimidin-2-yl)aminocarbonyl]-2,3-dihydro-2-methylbenzo[b]thiophen-7-sulfonamid-1,1-dioxid ist.

20. Zur Bekämpfung des Wachstums unerwünschter Vegetation geeignete Zusammensetzung, welche eine wirksame Menge einer Verbindung nach einem der Ansprüche 1 bis 19 und wenigstens eines der folgenden: oberflächenaktives Mittel, festes oder flüssiges Verdünnungsmittel, umfaßt.

21. Verfahren zur Bekämpfung des Wachstums unerwünschter Vegetation, welches die Anwendung einer wirksamen Menge der Verbindung nach einem der Ansprüche 1 bis 19 auf den zu schützenden Ort umfaßt.

22. Verfahren zur Steuerung des Wachstums von Pflanzen, welches die Anwendung einer wirksamen, jedoch im wesentlichen nicht phytotoxischen Menge eines aus den Verbindungen nach einem der Ansprüche 1 bis 19 ausgewählten Pflanzenwachstumsregulans auf den Ort solcher Pflanzen umfaßt.

23. Verfahren zur Herstellung einer Verbindung nach Anspruch 1 durch

(a) Umsetzen eines geeignet substituierten Sulfonylisocyanats der Formel

$LSO_2NCO$ oder $JSO_2NCO$

(II) (IIa)

mit einem Aminoheterocyclus der Formel

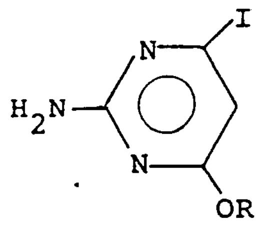

(III)

bzw.

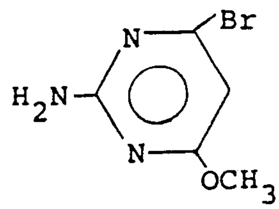

(IIIa)

worin L, J und R wie in Anspruch 1 definiert sind; oder

(b) Umsetzen eines Phenylcarbamats der Formel

$LSO_2NH\overset{\overset{O}{\|}}{C}OC_6H_5$ oder $JSO_2NH\overset{\overset{O}{\|}}{C}OC_6H_5$

(VI) (VIa)

mit dem Aminoheterocyclus der Formel (III) bzw. (IIIa), worin L und J wie in Anspruch 1 definiert sind; oder

(c) Umsetzen eines Sulfonamids der Formel

$LSO_2NH_2$ oder $JSO_2NH_2$

(IV) (IVa)

mit einem heterocyclischen Phenylcarbamat der Formel

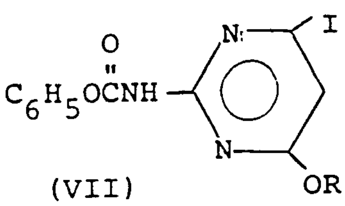

(VII)

bzw.

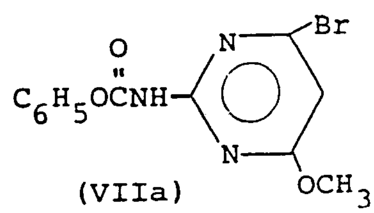

(VIIa)

worin L, J und R wie in Anspruch 1 definiert sind.

24. Jodpyrimidine der Formel

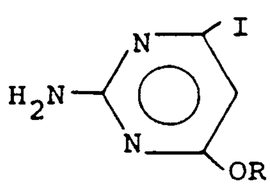

und Phenylcarbamate davon, worin R $CH_3$ oder $C_2H_5$ ist.

**Revendications**

1. Un composé de la formule

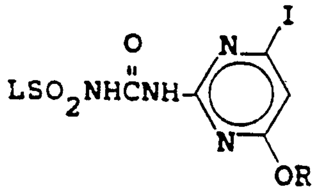

I

ou

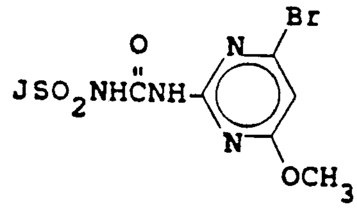

Ia

dans laquelle
R est $CH_3$ ou $CH_2CH_3$;

L is 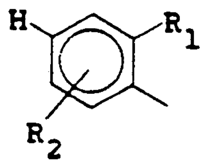 , 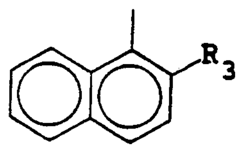 , 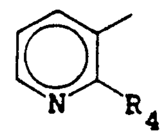 ,

L-1 L-2 L-3

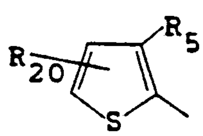 , 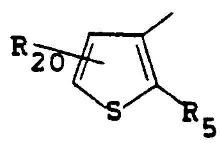 , 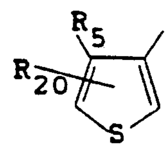 ,

L-4 L-5 L-6

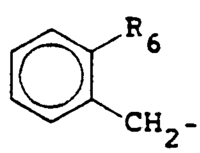 , 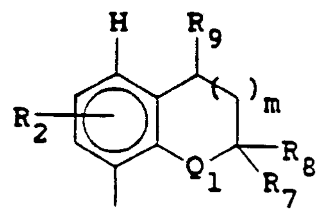 ,

L-7 L-8

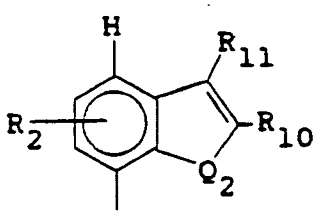 , 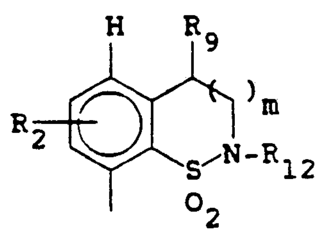 ,

L-9 L-10

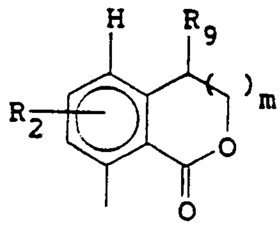 ,

L-11

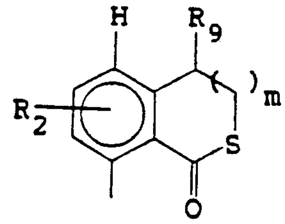 ,

L-12

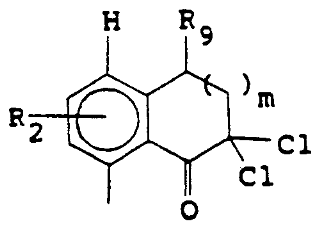 ,

L-13

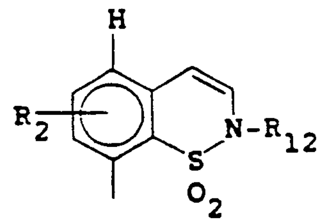 ,

L-14

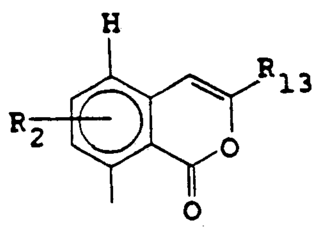 ,

L-15

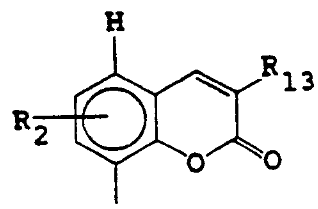

L-16

ou 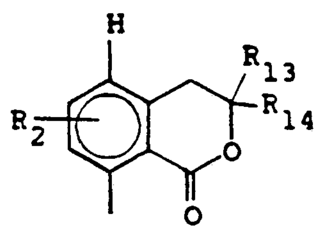 ;

L-17

J est 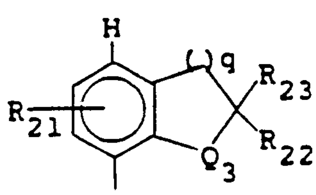 ou 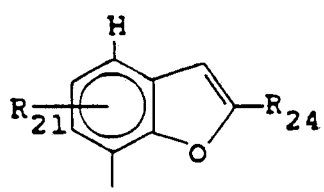 ;

J-1

J-2

$R_1$ est un groupe alkyle en $C_1$—$C_4$, alcoxy en $C_1$—$C_4$, $OCH_2CH_2OCH_3$, F, Cl, Br, $NO_2$, $CF_3$, $CO_2R_{15}$, $SO_2NR_{16}R_{17}$, $C(O)R_{25}$, $CR_{25}(OR_{26})$, $SO_2N(OCH_3)CH_3$, $OSO_2R_{18}$, $S(O)_nR_{19}$, $WCF_3$, $WCHF_2$, alcényloxy en $C_3$—$C_4$, alcynyloxy en $C_3$—$C_4$ alkyle en $C_1$—$C_2$ substitué par $OCH_3$ ou $OCH_2CH_3$, $C_6H_5$,

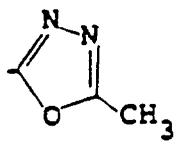 , 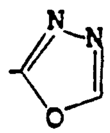 , 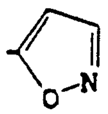 , 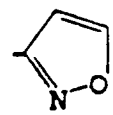 , 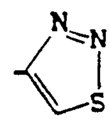 ,

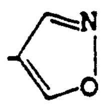 , 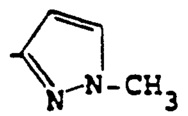 , 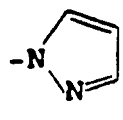 , 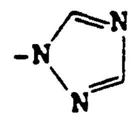 , 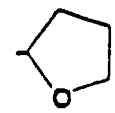 ,

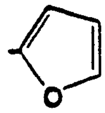 , 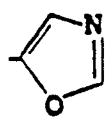 , 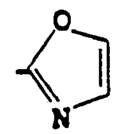 , 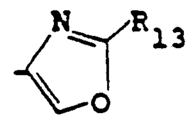 , 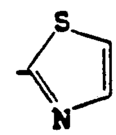 ,

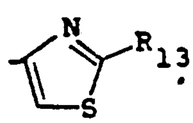 , 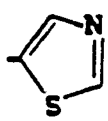 , 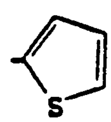 , 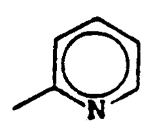 ou 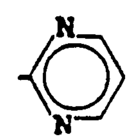 ;

$R_2$ est H, F, Cl, Br, un groupe alkyle en $C_1$—$C_4$, alcoxy en $C_1$—$C_4$, alcényloxy en $C_3$—$C_4$, alcynyloxy en $C_3$—$C_4$, halogénoalcoxy en $C_1$—$C_4$, halogénoalkyle en $C_1$—$C_4$, $S(O)_p$alkyle en $C_1$—$C_4$, $S(O)_p$alcényle en $C_3$—$C_4$, $S(O)_p$alcynyle en $C_3$—$C_4$ ou alkyle en $C_1$—$C_2$ substitué par $OCH_3$ ou $SCH_3$;

$R_3$ est H, $CH_3$, $OCH_3$, F, Cl, Br, $SO_2N(CH_3)_2$, $OSO_2CH_3$ ou $S(O)_nCH_3$;

$R_4$ est $CH_3$, $CH_2CH_3$, $OCH_3$, $OCH_2CH_3$, F, Cl, Br, $SO_2NR_{16}R_{17}$, $SO_2N(OCH_3)CH_3$, $S(O)_nR_{19}$, un groupe alcényloxy en $C_3$—$C_4$ ou un groupe alcynyloxy en $C_3$—$C_4$;

$R_5$ est un groupe alkyle en $C_1$—$C_3$, F, Cl, Br, $NO_2$, $CO_2R_{15}$, $SO_2NR_{16}R_{17}$, $SO_2N(OCH_3)CH_3$ ou $S(O)_nR_{19}$;

$R_6$ est Cl, $NO_2$, $CO_2CH_3$, $CO_2CH_2CH_3$, $SO_2N(CH_3)_2$, $OSO_2CH_3$, $SO_2CH_3$, $SO_2CH_2CH_3$, $OCH_3$ ou $OCH_2CH_3$;

$R_7$ est H, $CH_3$ ou $CH_2CH_3$;

$R_8$ est H, $CH_3$ ou $CH_2CH_3$;

$R_9$ est H ou $CH_3$;

$R_{10}$ est un H ou $CH_3$;

$R_{11}$ est H ou $CH_3$;

$R_{12}$ est H, un groupe alkyle en $C_1$—$C_4$, alcényle en $C_3$—$C_4$, alcynyle en $C_3$—$C_4$, benzyle, (alkyl en $C_1$—$C_4$)-carbonyle, (alcoxy en $C_1$—$C_4$)carbonyle, $CH_2CN$, $CH_2C(O)CH_3$, $CH_2CO_2$(alkyle en $C_1$—$C_2$), alkyle en $C_1$—$C_4$ substitué par 0 à 3 F, 0 à 1 Cl, OH, $OCH_3$ ou $OC_2H_5$ ou alcényle en $C_3$—$C_4$ substitué par 1 à 3 F ou 1 à 3 Cl;

$R_{13}$ est H ou $CH_3$;

$R_{14}$ est $CH_3$ ou $C_2H_5$;

$R_{15}$ est un groupe alkyle en $C_1$—$C_4$, $CH_2CH_2OCH_3$, $CH_2CH_2Cl$ ou $CH_2CH{=}CH_2$;

$R_{16}$ est H ou un groupe alkyle en $C_1$—$C_3$;

$R_{17}$ est un groupe alkyle en $C_1$—$C_3$;

$R_{18}$ est un groupe alkyle en $C_1$—$C_3$ ou $N(CH_3)_2$;

$R_{19}$ est un groupe alkyle en $C_1$—$C_3$ ou $CH_2CH{=}CH_2$;

$R_{20}$ est H, Cl ou $CH_3$;

$R_{21}$ est H, Cl, $CH_3$ ou $OCH_3$;

$R_{22}$ est H ou un groupe alkyle en $C_1$—$C_4$;

$R_{23}$ est H ou $CH_3$;

$R_{24}$ est H ou $CH_3$;

$R_{25}$ est H ou un groupe alkyle en $C_1$—$C_4$, alcényle en $C_3$—$C_4$, alcynyle en $C_3$—$C_4$ ou cycloalkyle en $C_3$—$C_6$;

$R_{26}$ est un groupe alkyle en $C_1$—$C_2$;

m est 0 ou 1;

n est 0 ou 2;

p est 0, 1 ou 2;

q est 1 ou 2;
$Q_1$ est O, S ou $SO_2$;
$Q_2$ est O ou S;
$Q_3$ est O, S, SO ou $SO_2$; et
W est O, S ou $SO_2$;

avec les conditions que:
1) le nombre total d'atomes de carbone de $R_{16}$ et $R_{17}$ est inférieur ou égal à quatre;
2) si m est 1, alors $R_9$ est H; et
3) si $R_{23}$ est $CH_3$, alors $Q_3$ est $SO_2$ et $R_{22}$ est $CH_3$;

et ses sels utilisables en agriculture.

2. Un composé de la revendication 1, où R est $CH_3$.

3. Un composé de la revendication 2, où L est L-1, L-2, L-3, L-5, L-8, L-10, L-11, L-16 ou L-17.

4. Un composé de la revendication 3, où L est L-1; $R_1$ est OCl, $OC_2H_5$, Cl, $NO_2$, $CF_3$, $CO_2CH_3$, $CO_2C_2H_5$, $SO_2N(CH_3)_2$, $SO_2N(OCH_3)CH_3$, $OSO_2R_{18}$, $C(O)CH_3$, $C(O)C_2H_5$, $S(O)_nR_{19}$, $OCF_2H$, $SCF_2H$, $OCH_2CH{=}CH_2$, $OCH_2C{\equiv}CH$,

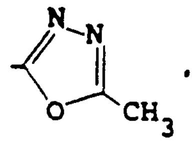 , 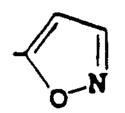 , 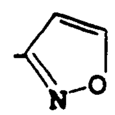 ou 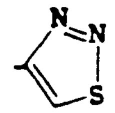 ;

$R_2$ est H, F, Cl, Br, $CH_3$, $C_2H_5$, un groupe $S(O)_p$alkyl en $C_1$—$C_2$, $S(O)_pCH_2CH{=}CH_2$, $S(O)_pCH_2C{\equiv}CH$, alcoxy en $C_1$—$C_3$, $OCH_2CH{=}CH_2$, $OCH_2{\equiv}CH$, halogénoalcoxy en $C_1$—$C_2$, halogénoalkyle en $C_1$—$C_2$, $CH_2OCH_3$ ou $CH_2SCH_3$; $R_{18}$ est un groupe alkyle en $C_1$—$C_3$; $R_{19}$ est $CH_3$ ou $C_2H_5$; et n est 2.

5. Un composé de la revendication 4, où L est

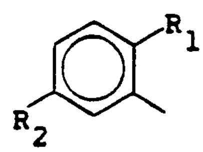 ;

et $R_2$ est H, F, Cl, Br, $CH_3$, $CH_2CH_3$, $SCH_3$, $SC_2H_5$, $OCH_3$, $OC_2H_5$, $CF_3$, $CH_2OCH_3$ ou $CH_2SCH_3$.

6. Un composé de la revendication 3, où L est L-2; et $R_3$ est Cl, $CH_3$, $OCH_3$, $SCH_3$ ou Br.

7. Un composé de la revendication 3, où L est L-3; et $R_4$ est Cl, $SO_2CH_3$ ou $SO_2N(CH_3)_2$.

8. Un composé de la revendication 3, où L est L-5; $R_5$ est $CO_2CH_3$ ou $CO_2C_2H_5$; et $R_{20}$ est H.

9. Un composé de la revendication 3, où L est L-8; m est 0; $R_2$ est H, F, Cl, Br, $CH_3$, $CH_2CH_3$, $SCH_3$, $SC_2H_5$, $OCH_3$, $OC_2H_5$, $CF_3$, $CH_2OCH_3$ ou $CH_2SCH_3$; et $R_9$ est H.

10. Un composé de la revendication 3, où L est L-10; $R_2$ est H, F, Cl, Br, $CH_3$, $CH_2CH_3$, $SCH_3$, $SC_2H_5$, $OCH_3$, $OC_2H_5$, $CF_3$, $CH_2OCH_3$ ou $CH_2SCH_3$; $R_9$ est H; $R_{12}$ est H ou un groupe alkyle en $C_1$—$C_4$, (alkyl en $C_1$—$C_4$)-carbonyle, $CH_2F$ ou alkyle en $C_2$—$C_3$ substitué par un F ou un Cl.

11. Un composé de la revendication 3, où L est L-11; $R_2$ est H, F, Cl, Br, $CH_3$, $CH_2CH_3$, $SCH_3$, $SC_2H_5$, $OCH_3$, $OC_2H_5$, $CF_3$, $CH_2OCH_3$ ou $CH_2SCH_3$; et $R_9$ est H.

12. Un composé de la revendication 3, où L est L-16; et $R_2$ est H, F, Cl, Br, $CH_3$, $CH_2CH_3$, $SCH_3$, $SC_2H_5$, $OCH_3$, $OC_2H_5$, $CF_3$, $CH_2OCH_3$ ou $CH_2SCH_3$.

13. Un composé de la revendication 3, où L est L-17; et $R_2$ est H, F, Cl, Br, $CH_3$, $CH_2CH_3$, $SCH_3$, $SC_2H_5$, $OCH_3$, $OC_2H_5$, $CF_3$, $CH_2OCH_3$ ou $CH_2SCH_3$.

14. Un composé de la revendication 1, qui est l'ester méthylique de l'acide 2-[[(4-iodo-6-méthoxypyrimidine-2-yl)aminocarbonyl]aminosulfonyl]benzoïque.

15. Un composé de la revendication 1, où J est J-1, $R_{22}$ est H, $CH_3$ ou $C_2H_5$ et $R_{23}$ est H.

16. Un composé de la revendication 1, qui est le N'-[(4-iodo-6-méthoxypyrimidine-2-yl)aminocarbonyl]-N,N-diméthyl-1,2-benzènedisulfonamide.

17. Un composé de la revendication 1, qui est l'ester méthylique de l'acide 2-[[(4-iodo-6-méthoxypyrimidine-2-yl)aminocarbonyl]aminosulfonyl]-4-méthylbenzoïque.

18. Un composé de la revendication 1, qui est le 1,1-dioxyde de N-[(4-bromo-6-méthoxypyrimidine-2-yl)aminocarbonyl]-2,3-dihydrobenzo[b]thiophène-7-sulfonamide.

19. Un composé de la revendication 1, qui est le 1,1-oxyde de N-[(4-bromo-6-méthoxypyrimidine-2-yl)-aminocarbonyl]-2,3-dihydro-2-méthylbenzo[b]thiophène-7-sulfonamide.

20. Une composition convenant pour lutter contre la croissance de végétation indésirable, qui comprend une quantité efficace d'un composé de l'une quelconque des revendications 1 à 19 et l'un au moins des ingrédients suivants: agent tensio-actif, diluant solide ou diluant liquide.

21. Un procédé pour lutter contre la croissance de végétation indésirable, qui consiste à appliquer à l'emplacement à protéger une quantité efficace du composé de l'une quelconque des revendications 1 à 19.

22. Un procédé pour réguler la croissance de plantes, qui consiste à appliquer à l'emplacement où se trouvent ces plantes une quantité efficace mais sensiblement non phytotoxique d'un régulateur de la croissance de plantes choisi parmi les composés de l'une quelconques des revendications 1 à 19.

23. Un procédé pour la production d'un composé de la revendication 1, qui consiste à:
(a) faire réagir un sulfonylisocyanate convenablement substitué de formule

$LSO_2NCO$ ou $JSO_2NCO$

(II) (IIa)

avec un aminohétérocycle de formule

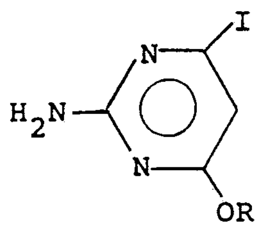

(III)

ou

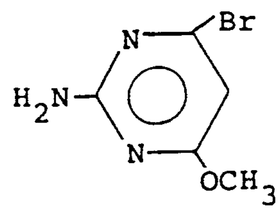

(IIIa)

respectivement, où L, J et R sont comme définis dans la revendication 1; ou
(b) faire réagir un phénylcarbamate de formule

$LSO_2NH\overset{O}{\overset{\|}{C}}OC_6H_5$ ou $JSO_2NH\overset{O}{\overset{\|}{C}}OC_6H_5$

(VI) (VIa)

avec ledit aminohétérocycle de formule (III) ou (IIIa) respectivement, où L et J sont comme définis dans la revendication 1; ou
(c) faire réagir un sulfonamide de formule

$LSO_2NH_2$ ou $JSO_2NH_2$

(IV) (IVa)

avec n phénylcarbamate hétérocyclique de formule

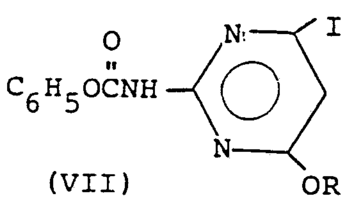

(VII)

ou

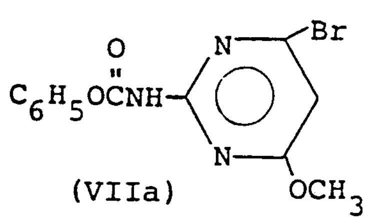

(VIIa)

respectivement, où L, J et R sont comme définis dans la revendication 1.
24. Iodopyrimidines de formule

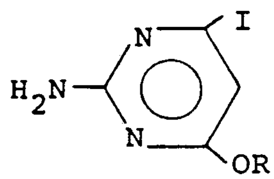

où R est $CH_3$ ou $C_2H_5$, et leurs phénylcarbamates.